# EUROPEAN PATENT SPECIFICATION

(11) **EP 1 736 906 B1**
(45) Date of publication and mention of the grant of the patent: **12.10.2016**
(21) Application number: 04724882.8
(22) Date of filing: 31.03.2004
(51) Int. Cl.: G06F 19/16, G06F 19/00, G06F 19/18

(54) **DRUG-METABOLIZING ENZYME PREDICTION APPARATUS**
MEDIKAMENTENMETABOLISIERUNGS-ENZYMVORHERSAGEVORRICHTUNG
APPAREIL DE PRÉVISION DES ENZYMES DE MÉTABOLISATION DE MÉDICAMENTS

(43) Date of publication of application: 27.12.2006
(73) Proprietor: FUJITSU LIMITED, Kawasaki-shi, Kanagawa 211-8588 (JP); Yamazoe, Yasushi, Yamato-shi, Kanagawa 2420002, (JP)
(72) Inventor: KITAJIMA, Masato, FUJITSU K. S. ENGINEERING LIM., Fukuoka-shi, Kukuoka 8148589 (JP); CILOY, Jose Martin, FUJITSU K. S. ENG. LIMITED, Fukuoka-shi, Fukuoka 8148589 (JP); YAMAZOE, Yasushi, Kanagawa 2420002 (JP)
(74) Representative: Fenlon, Christine Lesley
(86) International application number: PCT/JP2004/004746
(87) International publication number: WO 2005/101278

(56) References cited:
- EP-A2- 0 818 744
- ASANAGA M. ET AL.: 'P450 to Yakubutsu Taisha Database to Taishabutsu Yosoku.' FUJITSU vol. 53, no. 5, September 2002, pages 402 - 406, XP002996284
- KITAJIMA M. ET AL.: 'Yakuri Kassei Oyobi Admet o Doji Hyoka suru Integrated Kosoku/Kaso in Silico.Screening (II) : NTP Hatsugansei Data.' JOHO KAGAKU TORONKAI. KOZO KASSEI SOKAN SYMPOSIUM KOEN YOSHISHU. vol. 25, no. 30, 25 October 2002, pages 216 - 219, XP002996285
- YUDA K. ET AL.: 'Integrated In Silico Screening (V) :Hito P450 Yosoku Model no Kochiku to Kosatsu (Class Model)' KOZO KASSEI SOKAN SYMPOSIUM KOEN YOSHISHU vol. 31, 05 November 2003, pages 89 - 92, XP002996286
- ONO Y. ET AL.: 'Bio technology ni yoru Yakabutsu Taisha Kosokei no Kochiku to Bunshi Level deno Sogo Sayo Yosoku no tame no Riron Kochiku ni Kansuru Kenkyu.' KANMIN KYODO PROJECT KENKYU HOKOKU, HEISEI 8 NENDO FIRST FIELD, LIFE SCIENCE NO KISO TO SHITE NO BIO TECHNOLOGY NO OYO TO HYOKA GIJUTSU NO KAIHATSU 1997, pages 228 - 237, XP002996287

## Description

### TECHNICAL FIELD

The present invention generally relates to a drug-metabolizing enzyme prediction apparatus, a drug-metabolizing enzyme prediction method, and a recording medium, and more particularly, to a drug-metabolizing enzyme prediction apparatus, a drug-metabolizing enzyme prediction method, and a recording medium, capable of accurately predicting a molecular species of a drug-metabolizing enzyme and a reactive site of a compound with a drug-metabolizing enzyme.

### BACKGROUND ART

In recent years, about 50% or more of drugs developed are dropped out caused by a problem on drug metabolism (for example, drug's power and side effect) in the stage of drug development.

Therefore, it is greatly needed to evaluate the problem on drug metabolism related to compounds and to narrow them down to any compound that clears the problem in the early stage of drug development, in terms of development of drugs with fewer side effects and of reduction in cost required for development of drugs.

Conventionally, to specify, for example, a molecular species and a reactive site of a drug-metabolizing enzyme from a compound, a drug-metabolism screening test and metabolite identification using a mass spectrum are conducted.

A technology for predicting a molecular species and a reactive site of a drug-metabolizing enzyme on a computer is described in, for example, Nonpatent literature 1, Patent document 1, Patent document 2, Patent document 3, and Patent document 4.

Nonpatent literature 1 describes a technology for inputting a compound structure and predicting an unknown metabolite on the analogy of known metabolic reactions collected from documents. More specifically, it describes a technology of implementing an experimental technique of extracting a structure of a reactive site between a substrate and a metabolite from known metabolic reactions, and predicting a metabolite based on known metabolism information if the structure of the reactive site extracted is found in a compound to be predicted. This technology allows accurate prediction of a wide range of compounds based on a population (compounds) of training data used for creating a prediction model.

Furthermore, Patent document 1 describes a technology for predicting the relative rate of every metabolic pathway for drugs by regarding a pathway with the fastest reaction rate as a reaction that is the most possible reaction to occur, based on stoichiometric data.

Patent document 2 describes a technology for predicting the susceptibility of every metabolic site of drugs by regarding a site with the highest susceptibility as a first candidate for a metabolic site, based on energy theoretical data.

Patent document 3 describes a technology for predicting the stability of every metabolic site of drugs by regarding a site with the highest stability as a first candidate for a metabolic site, based on energy theoretical data.

Patent document 4 describes a technology for generating a ranked virtual library of candidate compounds for synthesis and experimental evaluation for drug efficacy.
Patent document 1: Specification of US Patent Application Laid Open No. 2003/0054430
Patent document 2: Specification of US Patent Application Laid Open No. 2002/0040276
Patent document 3: Specification of US Patent Application Laid Open No. 2001/0044699
Patent document 4: Specification of EP Patent No. 0,818,744.

Nonpatent literature 1: Homepage of Drug metabolism research support system "BioFrontier/P450" developed by Fujitsu Ltd.: "http://venus.netlaboratory.com/material/messe/biofrontierp 450/"

However, all identifications of metabolites using the drug-metabolism screening test and the mass spectrum are performed based on wet experiments, and this causes a problem to arise such that cost and time are required enormously.

In the technology described in Nonpatent literature 1, because a prediction rate is dependent on the quality of training data used, there is also a problem that it is difficult to predict compounds which are extremely different from the population (compounds) of the training data used for creation of the prediction model. In other words, there is a problem that the range of predictable compounds is limited by the population, and prediction cannot always be applied to all types of compounds. That is, all types of compounds cannot always be accurately predicted.

In the technologies described in Patent document 1, Patent document 2, and Patent document 3, because a cubic form of the compound is not calculated, there is a problem that any compound, of which high reactivity is predicted based on energy calculation but which cannot be bound to a drug-metabolizing enzyme in terms of its form, may also be included in the compounds accordingly.

The present invention has been achieved to solve the conventional problems, and it is an object of the present invention to provide a drug-metabolizing enzyme prediction apparatus, a drug-metabolizing enzyme prediction method, and a recording medium capable of accurately predicting a molecular species of a drug-metabolizing enzyme and a reactive site of a compound with a drug-metabolizing enzyme for all types of compounds without using training data for prediction and in consideration of each cubic form of the compounds.

### DISCLOSURE OF INVENTION

The present invention proposes to predict a molecular species of a drug-metabolizing enzyme and a reactive site of a compound with the drug-metabolizing enzyme.

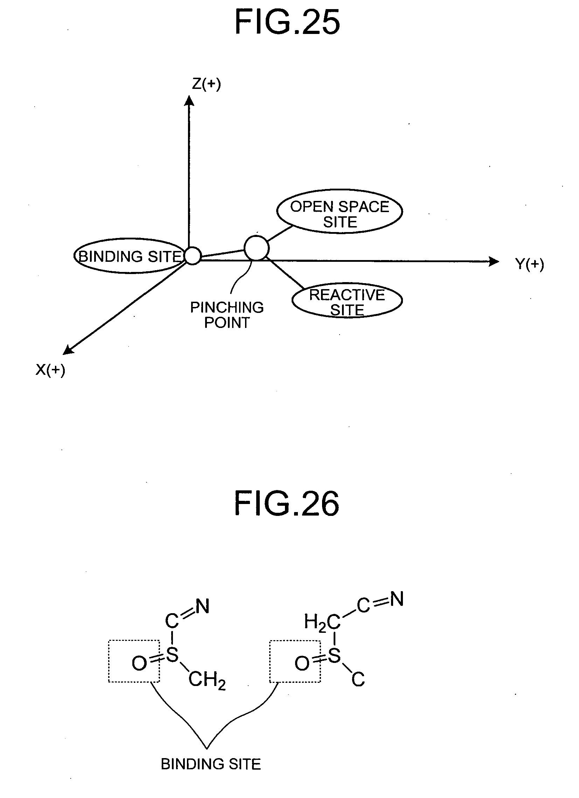

The inventors of the present invention have found, as a result of keen examination, that there are four sites where drug molecules are fixed, in a drug-metabolizing enzyme. And the inventors named these four sites "binding site (or inducing site)", "reactive site", "pinching point", and "open space site". Fig. 25 is a schematic of the binding site, the reactive site, the pinching point, and the open space site in a compound.

In Fig. 25, at first, the binding site is a site where a compound and a drug-metabolizing enzyme are bound to each other. The reactive site is a site where a metabolic reaction occurs in the compound and in the drug-metabolizing enzyme. The pinching point is an atom binding at least between the reactive site and the binding site. The open space site is a site (a group of atoms) other than the binding site and the reactive site which are bound to the pinching point.

In other words, the present invention is configured to predict a molecular species and a reactive site of a drug-metabolizing enzyme by respectively estimating the four sites.

To solve the above problems and to achieve the object, a drug-metabolizing enzyme prediction apparatus according to one aspect of the present invention is defined in claim 1.

Also described herein is an apparatus which includes a compound-structure-information acquiring unit that acquires compound structure information including at least one of atomic coordinate information that is information on coordinates of each of atoms forming a compound, bond information that is information on a bond between the atoms, and ring structure information that is information on a ring structure formed with a plurality of the atoms; a binding-site/molecular-species-information identifying unit that identifies binding site information that is information on an atom forming a binding site in the compound corresponding to the compound structure information and molecular species information that is information on a molecular species, from the compound structure information acquired by the compound-structure-information acquiring unit, based on a binding-site identifying condition for identifying the binding site that is a site where the compound and a drug-metabolizing enzyme are bound to each other and the molecular species of the drug-metabolizing enzyme to be bound; a pinching-point/reactive-site-information acquiring unit that specifies a pinching point that is the atom binding at least between the binding site and a reactive site that is a site where a metabolic reaction occurs in the compound and the drug-metabolizing enzyme, from the binding site information identified by the binding-site/molecular-species-information identifying unit and the compound structure information, and that acquires pinching point information that is information on the atom of the specified pinching point and the reactive site information that is information on the atom forming the reactive site bound to the pinching point corresponding to the pinching point information; a reactive-site-information identifying unit that identifies the reactive site information acquired by the pinching-point/reactive-site-information acquiring unit, from the molecular species information and the compound structure information, based on a reactive-site identifying condition for identifying the reactive site; and a pinching-point-information identifying unit that identifies the pinching point acquired by the pinching-point/reactive-site-information acquiring unit, from the molecular species information and the compound structure information, based on a pinching-point identifying condition for identifying the pinching point.

In the drug-metabolizing enzyme prediction apparatus described herein, the binding-site/molecular-species-information identifying unit includes a binding-site selecting unit that selects the binding site based on a predetermined binding-site selection criterion; a binding-site-information acquiring unit that acquires the binding site information for the binding site selected by the binding-site selecting unit, from the compound structure information; and an acquired-binding-site/molecular-species-information identifying unit that identifies the binding site information acquired by the binding-site-information acquiring unit, from the compound structure information, based on the binding-site identifying condition, and identifies the molecular species corresponding to the identified binding site information as the molecular species information.

In the drug-metabolizing enzyme prediction apparatus according to the present invention, the binding-site identifying condition is a condition in which a binding-site coordinate range that is a range of the atomic coordinate information for the atom forming the binding site is defined for each predetermined molecular species. The acquired-binding-site/molecular-species-information identifying unit includes a coordinate-based acquired-binding-site/molecular-species-information identifying unit that identifies the binding site information when the atomic coordinate information for the atom that forms the binding site corresponding to the binding site information acquired by the binding-site-information acquiring unit satisfies the binding-site coordinate range defined by the binding-site identifying condition, and identifies the molecular species corresponding to the satisfied binding-site coordinate range as the molecular species information.

In the drug-metabolizing enzyme prediction apparatus described herein, the pinching-point/reactive-site-information acquiring unit includes an atom specifying unit that specifies a directly bonded atom that is the atom bonded to the atom forming the binding site corresponding to the binding site information identified by the binding-site/molecular-species-information identifying unit and also specifies the atom bonded to the directly bonded atom, based on the bond information included in the compound structure information; a ring-structure determining unit that determines whether the atom specified by the atom specifying unit is the atom forming the ring structure, based on the ring structure information included in the compound structure information; a pinching-point/reactive-site specifying unit that specifies the atom specified and another atom forming the ring structure together with the atom, as the atoms forming the reactive site, when it is determined by the ring-structure determining unit that the atom specified by the atom specifying unit is the atom forming the ring structure, and that specifies the directly bonded atom specified by the atom specifying unit, as the pinching point; and a specified-pinching-point/reactive-site-information acquiring unit that acquires the pinching point information for the pinching point specified by the pinching-point/reactive-site specifying unit and the reactive site information for the reactive site specified, from the binding site information and the compound structure information.

In the drug-metabolizing enzyme prediction apparatus according to the present invention, the reactive-site identifying condition is such that a reactive-site coordinate range that is a range of the atomic coordinate information for the atoms forming the reactive site, a reactive-site angle range that is a range of an angle value between each of the atoms forming the reactive site and the pinching point, and a reactive-site distance range that is a range of a distance value between each of the atoms forming the reactive site and the pinching point are defined for each of the molecular species previously specified.

The reactive-site-information identifying unit includes a coordinate-based reactive-site-information determining unit that determines whether the atomic coordinate information for the atoms, which form the reactive site corresponding to the reactive site information acquired by the pinching-point/reactive-site-information acquiring unit, satisfies the reactive-site coordinate range defined by the reactive-site identifying condition, in the molecular species corresponding to the molecular species information identified; an angle-based reactive-site-information determining unit that calculates the angle value between the atom forming the reactive site corresponding to the reactive site information and the pinching point corresponding to the pinching point information, and determines whether the angle value calculated satisfies the reactive-site angle range defined by the reactive-site identifying condition, in the molecular species corresponding to the molecular species information identified; a distance-based reactive-site-information determining unit that calculates the distance value between the atom forming the reactive site corresponding to the reactive site information and the pinching point corresponding to the pinching point information, and determines whether the distance value calculated satisfies the reactive-site distance range defined by the reactive-site identifying condition, in the molecular species corresponding to the molecular species information identified; and a determination-result-based reactive-site-information identifying unit that identifies the reactive site information determined, as being satisfied, by the coordinate-based reactive-site-information determining unit, the angle-based reactive-site-information determining unit, and the distance-based reactive-site-information determining unit, as the reactive site information that satisfies the reactive-site identifying condition.

In the drug-metabolizing enzyme prediction apparatus according to the present invention, the pinching-point identifying condition is such that a pinching-point coordinate range that is a range of the atomic coordinate information for the atom as the pinching point is defined for each of the molecular species previously specified.

The pinching-point-information identifying unit includes a coordinate-based pinching-point-information identifying unit that identifies the pinching point information when the atomic coordinate information for the atom, which is the pinching point corresponding to the pinching point information acquired by the pinching-point/reactive-site-information acquiring unit, satisfies the pinching-point coordinate range defined by the pinching-point identifying condition, in the molecular species corresponding to the molecular species information identified.

The drug-metabolizing enzyme prediction apparatus described herein further includes an open-space-site-information acquiring unit that acquires open-space site information that is information on the atom forming an open space site, from the binding site information, the reactive site information, the pinching point information, and the compound structure information, if there exists the open space site that is a site other than the reactive site corresponding to the reactive site information identified by the reactive-site-information identifying unit and the binding site corresponding to the binding site information identified by the binding-site/molecular-species-information identifying unit and which is bound to the pinching point corresponding to the pinching point information identified by the pinching-point-information identifying unit; and an open-space-site-information identifying unit that identifies the open-space site information acquired by the open-space-site-information acquiring unit, from the molecular species information and the compound structure information, based on an open-space-site identifying condition for identifying the open space site.

In the drug-metabolizing enzyme prediction apparatus according to the present invention, the open-space-site identifying condition is such that an open-space-site coordinate range, which is a range of the atomic coordinate information for the atom forming the open space site, is defined for each of the molecular species previously specified.

The open-space-site-information identifying unit described herein includes a coordinate-based open-space-site-information identifying unit that identifies the open-space site information when the atomic coordinate information for the atom, which forms the open space site corresponding to the open-space site information acquired by the open-space-site-information acquiring unit, satisfies the open-space-site coordinate range defined by the open-space-site identifying condition, in the molecular species corresponding to the molecular species information identified.

The drug-metabolizing enzyme prediction apparatus described herein further includes an inhibition determining unit that determines whether the atom forming the reactive site corresponding to the reactive site information or a partial-structure atom group which is a group of part of the atoms in a molecular structure at the reactive site inhibits the metabolic reaction, when the reactive site information is identified by the reactive-site-information identifying unit, based on an inhibition determining condition for determining whether the metabolic reaction is inhibited in the compound of which the reactive site information is identified.

A drug-metabolizing enzyme prediction method according to another aspect of the present invention is defined in claim 10.

Also described herein is a drug-metabolizing enzyme prediction program which causes a computer to execute a compound-structure-information acquiring procedure of acquiring compound structure information including at least one of atomic coordinate information that is information on coordinates of each of atoms forming a compound, bond information that is information on a bond between the atoms, and ring structure information that is information on a ring structure formed with a plurality of the atoms; a binding-site/molecular-species-information identifying procedure of identifying binding site information that is information on an atom forming a binding site in the compound corresponding to the compound structure information and molecular species information that is information on a molecular species, from the compound structure information acquired at the compound-structure-information acquiring procedure, based on a binding-site identifying condition for identifying the binding site that is a site where the compound and a drug-metabolizing enzyme are bound to each other and the molecular species of the drug-metabolizing enzyme to be bound; a pinching-point/reactive-site-information acquiring procedure of specifying a pinching point that is the atom binding at least between the binding site and a reactive site that is a site where a metabolic reaction occurs in the compound and the drug-metabolizing enzyme, from the binding site information identified at the binding-site/molecular-species-information identifying procedure and the compound structure information, and acquiring pinching point information that is information on the atom of the specified pinching point and the reactive site information that is information on the atom forming the reactive site bound to the pinching point corresponding to the pinching point information; a reactive-site-information identifying procedure of identifying the reactive site information acquired at the pinching-point/reactive-site-information acquiring procedure, from the molecular species information and the compound structure information, based on a reactive-site identifying condition for identifying the reactive site; and a pinching-point-information identifying procedure of identifying the pinching point acquired at the pinching-point/reactive-site-information acquiring procedure, from the molecular species information and the compound structure information, based on a pinching-point identifying condition for identifying the pinching point.

In the drug-metabolizing enzyme prediction program described, the binding-site/molecular-species-information identifying procedure includes a binding-site selecting procedure of selecting the binding site based on a predetermined binding-site selection criterion; a binding-site-information acquiring procedure of acquiring the binding site information for the binding site selected at the binding-site selecting procedure, from the compound structure information; and an acquired-binding-site/molecular-species-information identifying procedure of identifying the binding site information acquired at the binding-site-information acquiring procedure, from the compound structure information, based on the binding-site identifying condition, and identifies the molecular species corresponding to the identified binding site information as the molecular species information.

In the drug-metabolizing enzyme prediction method according to the present invention, the binding-site identifying condition is a condition in which a binding-site coordinate range that is a range of the atomic coordinate information for the atom forming the binding site is defined for each predetermined molecular species.

The acquired-binding-site/molecular-species-information identifying procedure includes a coordinate-based acquired-binding-site/molecular-species-information identifying procedure of identifying the binding site information when the atomic coordinate information for the atom that forms the binding site corresponding to the binding site information acquired at the binding-site-information acquiring procedure satisfies the binding-site coordinate range defined by the binding-site identifying condition, and identifying the molecular species corresponding to the satisfied binding-site coordinate range as the molecular species information.

In the drug-metabolizing enzyme prediction program described, the pinching-point/reactive-site-information acquiring procedure includes an atom specifying procedure of specifying a directly bonded atom that is the atom bonded to the atom forming the binding site corresponding to the binding site information identified at the binding-site/molecular-species-information identifying procedure and also specifying the atom bonded to the directly bonded atom, based on the bond information included in the compound structure information; a ring-structure determining procedure of determining whether the atom specified at the atom specifying procedure is the atom forming the ring structure, based on the ring structure information included in the compound structure information; a pinching-point/reactive-site specifying procedure of specifying the atom specified and another atom forming the ring structure together with the atom, as the atoms forming the reactive site, when it is determined at the ring-structure determining procedure that the atom specified at the atom specifying procedure is the atom forming the ring structure, and specifying the directly bonded atom specified at the atom specifying procedure, as the pinching point; and a specified pinching-point/reactive-site-information acquiring procedure of acquiring the pinching point information for the pinching point specified at the pinching-point/reactive-site specifying procedure and the reactive site information for the reactive site specified, from the binding site information and the compound structure information.

In the drug-metabolizing enzyme prediction method according to the present invention, the reactive-site identifying condition is such that a reactive-site coordinate range that is a range of the atomic coordinate information for the atoms forming the reactive site, a reactive-site angle range that is a range of an angle value between each of the atoms forming the reactive site and the pinching point, and a reactive-site distance range that is a range of a distance value between each of the atoms forming the reactive site and the pinching point are defined for each of the molecular species previously specified.

The reactive-site-information identifying procedure includes a coordinate-based reactive-site-information determining procedure of determining whether the atomic coordinate information for the atoms, which form the reactive site corresponding to the reactive site information acquired at the pinching-point/reactive-site-information acquiring procedure, satisfies the reactive-site coordinate range defined by the reactive-site identifying condition, in the molecular species corresponding to the molecular species information identified; an angle-based reactive-site-information determining procedure of calculating the angle value between the atom forming the reactive site corresponding to the reactive site information and the pinching point corresponding to the pinching point information, and determining whether the angle value calculated satisfies the reactive-site angle range defined by the reactive-site identifying condition, in the molecular species corresponding to the molecular species information identified; a distance-based reactive-site-information determining procedure of calculating the distance value between the atom forming the reactive site corresponding to the reactive site information and the pinching point corresponding to the pinching point information, and determining whether the distance value calculated satisfies the reactive-site distance range defined by the reactive-site identifying condition, in the molecular species corresponding to the molecular species information identified; and a determination-result-based reactive-site-information identifying procedure of identifying the reactive site information determined, as being satisfied, at the coordinate-based reactive-site-information determining procedure, the angle-based reactive-site-information determining procedure, and the distance-based reactive-site-information determining procedure, as the reactive site information that satisfies the reactive-site identifying condition.

In the drug-metabolizing enzyme prediction method according to the present invention, the pinching-point identifying condition is such that a pinching-point coordinate range that is a range of the atomic coordinate information for the atom as the pinching point is defined for each of the molecular species previously specified.

The pinching-point-information identifying procedure includes a coordinate-based pinching-point-information identifying procedure of identifying the pinching point information when the atomic coordinate information for the atom, which is the pinching point corresponding to the pinching point information acquired at the pinching-point/reactive-site-information acquiring procedure, satisfies the pinching-point coordinate range defined by the pinching-point identifying condition, in the molecular species corresponding to the molecular species information identified.

The drug-metabolizing enzyme prediction program described further causes the computer to execute an open-space-site-information acquiring procedure of acquiring open-space site information that is information on the atom forming an open space site, from the binding site information, the reactive site information, the pinching point information, and the compound structure information, if there exists the open space site that is a site other than the reactive site corresponding to the reactive site information identified at the reactive-site-information identifying procedure and the binding site corresponding to the binding site information identified at the binding-site/molecular-species-information identifying procedure and which is bound to the pinching point corresponding to the pinching point information identified at the pinching-point-information identifying procedure; and an open-space-site-information identifying procedure of identifying the open-space site information acquired at the open-space-site-information acquiring procedure, from the molecular species information and the compound structure information, based on an open-space-site identifying condition for identifying the open space site.

In the drug-metabolizing enzyme prediction method according to the present invention, the open-space-site identifying condition is such that an open-space-site coordinate range, which is a range of the atomic coordinate information for the atom forming the open space site, is defined for each of the molecular species previously specified.

The open-space-site-information identifying procedure includes a coordinate-based open-space-site-information identifying procedure of identifying the open-space site information when the atomic coordinate information for the atom, which forms the open space site corresponding to the open-space site information acquired at the open-space-site-information acquiring procedure, satisfies the open-space-site coordinate range defined by the open-space-site identifying condition, in the molecular species corresponding to the molecular species information identified.

The drug-metabolizing enzyme prediction program described further causes the computer to execute an inhibition determining procedure of determining whether the atom forming the reactive site corresponding to the reactive site information or a partial-structure atom group which is a group of part of the atoms in a molecular structure at the reactive site inhibits the metabolic reaction, when the reactive site information is identified at the reactive-site-information identifying procedure, based on an inhibition determining condition for determining whether the metabolic reaction is inhibited in the compound of which the reactive site information is identified.

A computer-readable recording medium according to still another aspect of the present invention as defined in claim 19 stores therein the drug-metabolizing enzyme prediction method according to the present invention.

The present invention is configured to acquire the compound structure information including atomic coordinate information of each of a plurality of atoms forming a compound, bond information between the atoms, the ring structure information on a ring structure formed with the atoms; identify the binding site information on an atom in the compound corresponding to the compound structure information and the molecular species information, from the compound structure information acquired, based on the binding-site identifying condition for identifying the molecular species of the drug-metabolizing enzyme belonging to the CYP families 1-4 of cytochromes P450 to be bound and for identifying a site in the compound where the compound and the drug-metabolizing enzyme are bound to each other; specify the pinching point which is the atom binding the binding site to a reactive site where a metabolic reaction of the compound and the drug-metabolizing enzyme occurs, from the binding site information identified and the compound structure information; acquire the pinching point information on the atom of the pinching point specified and the reactive site information on the atom forming the reactive site bound to the pinching point; identify the reactive site information acquired, from the molecular species information and the compound structure information, based on the reactive-site identifying condition for identifying the reactive site; and identify the pinching point information acquired, from the molecular species information and the compound structure information, based on the pinching-point identifying condition for identifying the pinching point. Therefore, it is possible to accurately predict a molecular species of a drug-metabolizing enzyme and a reactive site of a compound with a drug-metabolizing enzyme, for all types of compounds without using training data for prediction and in consideration of each cubic form of the compounds.

### BRIEF DESCRIPTION OF DRAWINGS

Fig. 1 is a principle diagram of a basic principle of the present invention; Fig. 2 is a block diagram of one example of a system to which the present invention is applied; Fig. 3 is a diagram of one example of information stored in a compound-structure information file 106a; Fig. 4 is a diagram of one example of information stored in a binding-site identifying condition file 106b; Fig. 5 is a diagram of one example of information stored in a binding-site/molecular-species information file 106c; Fig. 6 is a diagram of one example of information stored in a pinching-point information file 106d; Fig. 7 is a diagram of one example of information stored in a reactive-site information file 106e; Fig. 8 is a diagram of one example of information stored in a reactive-site identifying condition file 106f; Fig. 9 is a diagram of one example of information stored in a pinching-point identifying condition file 106g; Fig. 10 is a diagram of one example of information stored in an open-space-site information file 106h; Fig. 11 is a diagram of one example of information stored in an open-space-site identifying condition file 106i; Fig. 12 is a diagram of one example of information stored in a prediction result file 106j; Fig. 13 is a block diagram of one example of a binding-site/molecular-species information identifying unit 102b in the system to which the present invention is applied; Fig. 14 is a block diagram of one example of a pinching-point/reactive-site-information acquiring unit 102c in the system to which the present invention is applied; Fig. 15 is a block diagram of one example of a reactive-site-information identifying unit 102d in the system to which the present invention is applied; Fig. 16 is a block diagram of one example of a pinching-point-information identifying unit 102e in the system to which the present invention is applied; Fig. 17 is a block diagram of one example of an open-space-site-information identifying unit 102g in the system to which the present invention is applied; Fig. 18 is a flowchart of one example of the main process in the system; Fig. 19 is a flowchart of one example of a binding-site/molecular-species-information identifying process in the system according to the embodiment; Fig. 20 is a flowchart of one example of a pinching-point/reactive-site-information acquiring process in the system according to the embodiment; Fig. 21 is a flowchart of one example of a reactive-site-information identifying process in the system according to the embodiment; Fig. 22 is a flowchart of one example of a reactive-site coordinate range determining process in the system according to the embodiment; Fig. 23 is a flowchart of one example of a pinching-point-information identifying process in the system according to the embodiment; Fig. 24 is a flowchart of one example of an open-space-site-information identifying process in the system according to the embodiment; Fig. 25 is a schematic of the binding site, the reactive site, the pinching point, and the open space site in a compound; Fig. 26 is a schematic of one example of a binding-site selection criterion; Fig. 27 is a schematic of one example of a binding-site identifying condition; Fig. 28 is a schematic of one example of a reactive-site identifying condition;

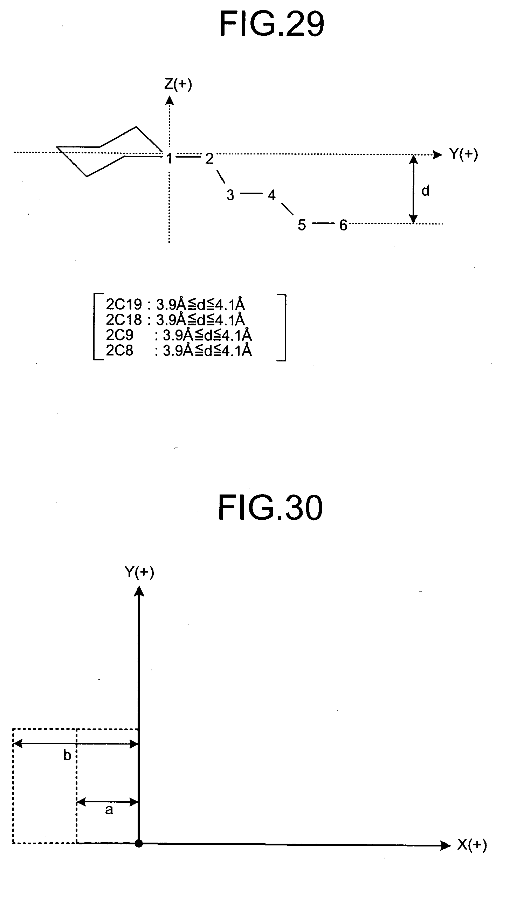

Fig. 29 is a schematic of one example of the reactive-site identifying condition; Fig. 30 is a schematic of one example of an open-space-site identifying condition; and

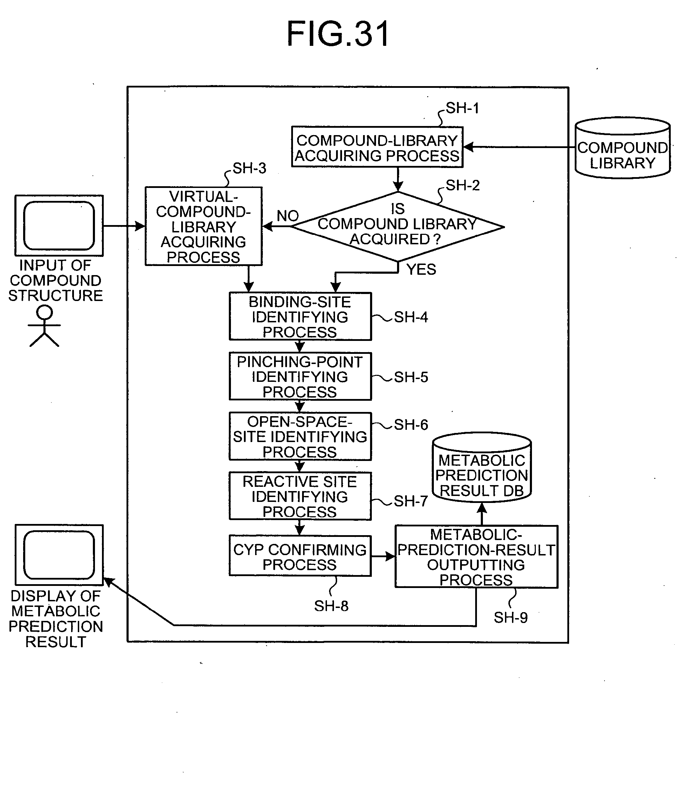

Fig. 31 is a diagram for explaining one example of the main process in a drug-metabolizing enzyme prediction apparatus 100 according to an example of the present invention.

### BEST MODE(S) FOR CARRYING OUT THE INVENTION

Exemplary embodiments of a drug-metabolizing enzyme prediction apparatus, a drug-metabolizing enzyme prediction method, and a recording medium according to the present invention are explained in detail below with reference to the accompanying drawings. It should be noted that the present invention is not limited by these embodiments.

### [Outline of the present invention]

The outline of the present invention is explained below first, and the configuration and processes of the present invention are explained in detail next.

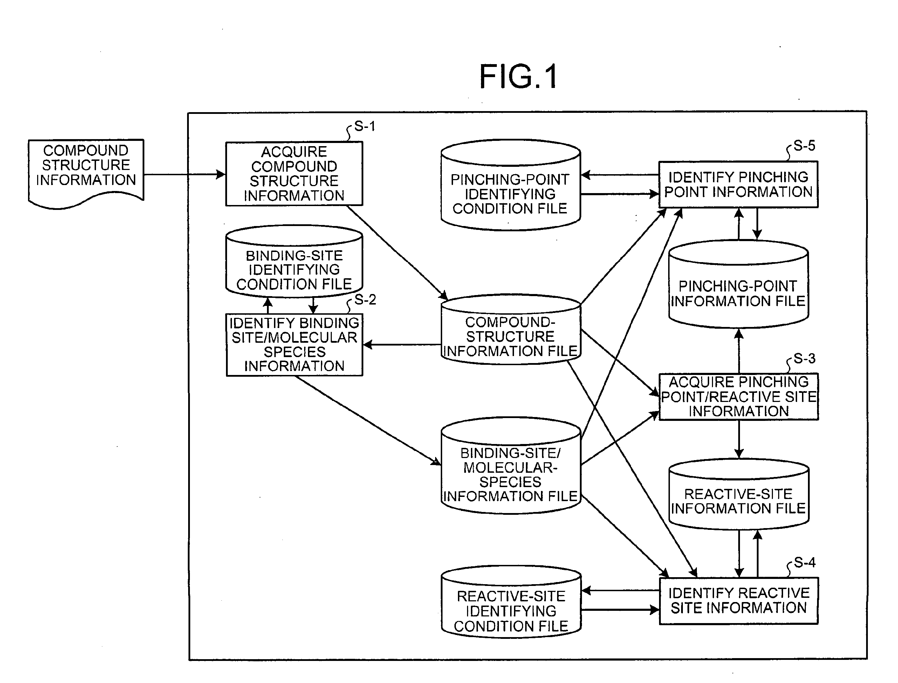

Fig. 1 is a principle diagram of a basic principle of the present invention.

The present invention schematically includes the following basic features. That is, at first, the present invention acquires compound structure information including atomic coordinate information being information on coordinates of each of a plurality of atoms forming a compound, bond information between the atoms, and ring structure information on a ring structure formed with the atoms (step S-1).

Then, the present invention identifies binding site information on an atom in the compound corresponding to the compound structure information and also molecular species information, from the compound structure information acquired at step S-1, based on a binding-site identifying condition for identifying the molecular species of a drug-metabolizing enzyme belonging to the CYP families 1-4 of cytochromes P450 to be bound and for identifying a site in the compound, where the compound and the drug-metabolizing enzyme are bound to each other (step S-2).

From the binding site information identified at step S-2 and the compound structure information acquired at step S-1, the present invention identifies a pinching point which is an atom binding the binding site to a reactive site in which a metabolic reaction of the compound and the drug-metabolizing enzyme occurs, and acquires pinching point information on the atom of the pinching point identified and reactive site information being information on the atom forming the reactive site bound to the pinching point (step S-3).

Then, the present invention identifies the reactive site information acquired at step S-3, from the molecular species information and the compound structure information, based on a reactive-site identifying condition for identifying a reactive site (step S-4).

Thereafter, the present invention identifies the pinching point information acquired at step S-3, from the molecular species information and the compound structure information, based on the pinching-point identifying condition for identifying a pinching point (step S-5).

Here, if there exists an open space site being a site which is other than the reactive site corresponding to the reactive site information identified at step S-4 and the binding site corresponding to the binding site information identified at step S-2 and which is bound to the pinching point corresponding to the pinching point information identified at step S-5, the present invention may acquire open-space-site information being information on an atom forming an open space site from the binding site information, the reactive site information, the pinching point information, and the compound structure information, and identify the open-space-site information acquired, from the molecular species information and the compound structure information, based on the open-space-site identifying condition for identifying an open space site.

When the reactive site information is identified at step S-4, the present invention may determine an atom forming a reactive site corresponding to the reactive site information or a partial-structure atom group which is a group of part of atoms in a molecular structure of the reactive site, based on an inhibition determining condition (e.g., a list of predetermined atoms as inhibitors) for determining whether a metabolic reaction is inhibited in the compound of which the reactive site information is identified.

Furthermore, the present invention may output, as a prediction result for a compound, at least one of the binding site information, the molecular species information, the reactive site information, the pinching point information, the open-space site information, and the inhibition determination result in the compound, which are identified (predicted).

As explained above, the present invention can automatically identify the binding site, the pinching point, the reactive site, and the open space site in the compound from the compound structure information. Therefore, it is possible to accurately predict a molecular species of a drug-metabolizing enzyme and a reactive site of a compound with the drug-metabolizing enzyme for all kinds of compounds without using training data for prediction and in consideration of each cubic form of the compounds.

The present invention can further determine (check) whether any atom inhibiting a metabolic reaction is contained in the reactive site identified, for the compound of which the reactive site with the drug-metabolizing enzyme is identified. Therefore, a useful compound group can be narrowed highly accurately, for example, in the early stage of drug development.

### [System configuration]

The configuration of the system is explained in detail below.

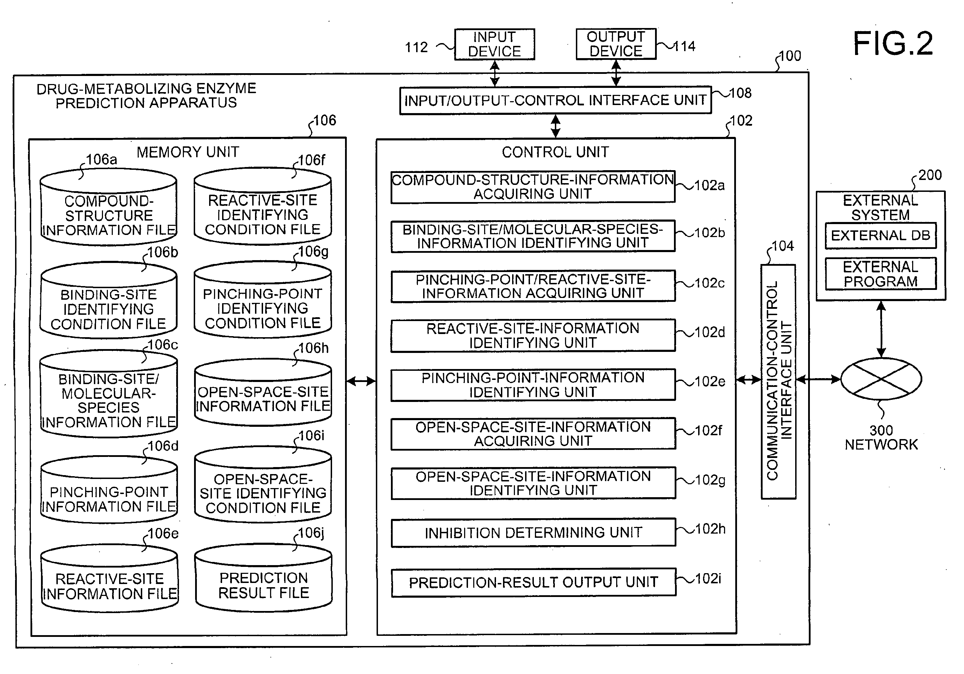

Fig. 2 is a block diagram of one example of the system to which the present invention is applied, and only a portion related to the present invention in the configuration is conceptually shown therein.

In Fig. 2, a network 300 such as the Internet has functions of mutually connecting between the drug-metabolizing enzyme prediction apparatus 100 and an external system 200.

In Fig. 2, the external system 200 is mutually connected with the drug-metabolizing enzyme prediction apparatus 100 through the network 300, and has a function of providing a compound library, an external database on the compound structure information, and a website for executing various external programs, to users.

Here, the external system 200 may be configured as a WEB server or an ASP server, and its hardware may be configured by an information processor such as commercially available work station and personal computer, and its attachment device. The functions of the external system 200 are implemented by a CPU, a disk drive, a memory device, an input device, an output device, a communication control device in the hardware of the external system 200, and by programs for controlling these devices.

In Fig. 2, the drug-metabolizing enzyme prediction apparatus 100 schematically includes a control unit 102 such as CPU that integrally controls the whole of the drug-metabolizing enzyme prediction apparatus 100; a communication-control interface unit 104 connected to a communication device (not shown) such as a router connected to a communication line and the like; a memory unit 106 that stores various types of databases and files; and an input/output-control interface unit 108 connected to an input device 112 and an output device 114. These units are communicably connected to one another through arbitrary communication paths. Furthermore, the drug-metabolizing enzyme prediction apparatus 100 is communicably connected to the network 300 through a communication device such as a router and a wired or a wireless communication line such as a dedicated line.

The various types of databases, tables, and files (the compound-structure information file 106a to the prediction result file 106j) stored in the memory unit 106 of Fig. 2 are storage units such as fixed disk devices, which store various programs, tables, files, and databases used for various processes, and files for web pages.

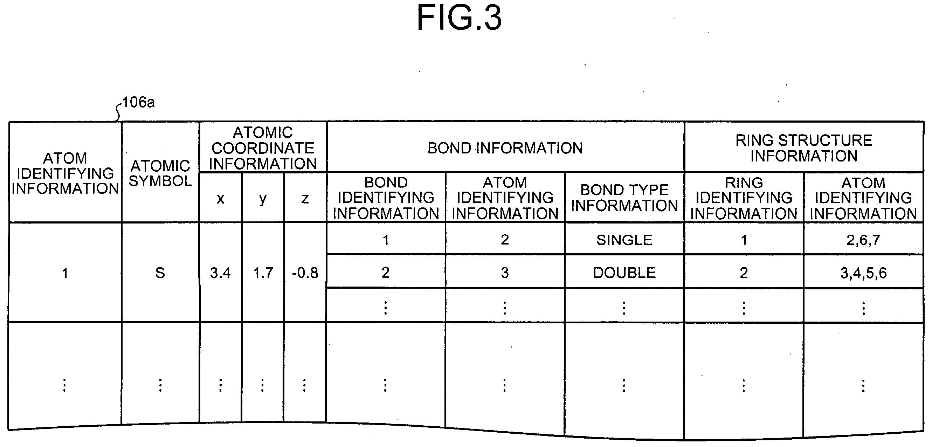

Among these components in the memory unit 106, the compound-structure information file 106a is a compound-structure-information storage unit that stores the compound structure information including at least one of the atomic coordinate information being information on coordinates of each of atoms forming a compound, the bond information being information on a bond between the atoms, and the ring structure information being information on a ring structure formed with a plurality of the atoms. Information stored in the compound-structure information file 106a is explained below with reference to Fig. 3.

Fig. 3 is a diagram of one example of information stored in the compound-structure information file 106a. As shown in Fig. 3, the information stored in the compound-structure information file 106a includes atom identifying information for uniquely identifying an atom forming a compound; an atomic symbol of the atom corresponding to the atom identifying information; atomic coordinate information (x coordinate value, y coordinate value, and z coordinate value) for the atom corresponding to the atom identifying information; bond information that includes bond identifying information for uniquely identifying the bond information, atom identifying information for an atom bonded to the atom corresponding to the atom identifying information in the bond identifying information, and bond type information on bond types; and ring structure information that includes ring identifying information for uniquely identifying the ring structure information and also includes atom identifying information for atoms forming the ring structure, which are mutually associated with one another.

The compound-structure information file 106a may be external compound-structure information databases, which are accessed through the Internet, or may be an in-house database created by copying these databases, storing original compound structure information, and further adding unique annotation information and so forth.

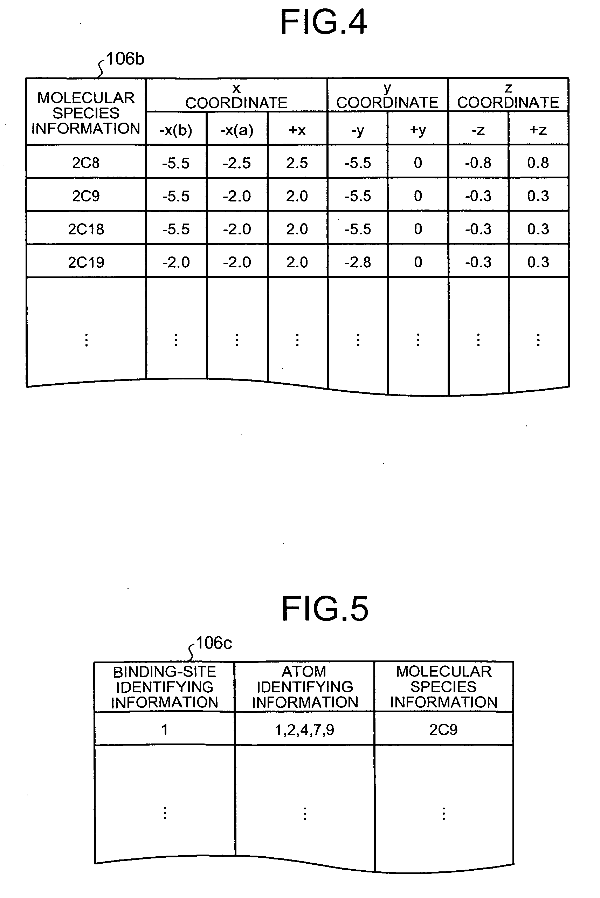

The binding-site identifying condition file 106b is a binding-site identifying condition storage unit that stores the binding-site identifying condition (e.g., condition under which a binding-site coordinate range, which is a range of atomic coordinate information for an atom forming a binding site, is defined for each of predetermined molecular species) for identifying a binding site being a site where a compound and a drug-metabolizing enzyme are bound to each other and also identifying a molecular species of the drug-metabolizing enzyme to be bound. Here, information stored in the binding-site identifying condition file 106b is explained below with reference to Fig. 4.

Fig. 4 is a diagram of one example of information stored in the binding-site identifying condition file 106b. As shown in Fig. 4, the information stored in the binding-site identifying condition file 106b includes molecular species information and ranges (range of ±x direction, range of ±y direction, and range of ±z direction) of atomic coordinate information for an atom forming a binding site, which are mutually associated with one another (in a matrix form).

The binding-site/molecular-species information file 106c is a binding-site/molecular-species information storage unit that stores the binding site information and the molecular species information identified by a binding-site/molecular-species-information identifying unit 102b explained later. Information stored in the binding-site/molecular-species information file 106c is explained below with reference to Fig. 5.

Fig. 5 is a diagram of one example of information stored in the binding-site/molecular-species information file 106c. As shown in Fig. 5, the information stored in the binding-site/molecular-species information file 106c includes binding-site identifying information for uniquely identifying a binding site, atom identifying information for an atom forming the binding site, and molecular species information for a drug-metabolizing enzyme bound to the binding site, which are mutually associated with one another.

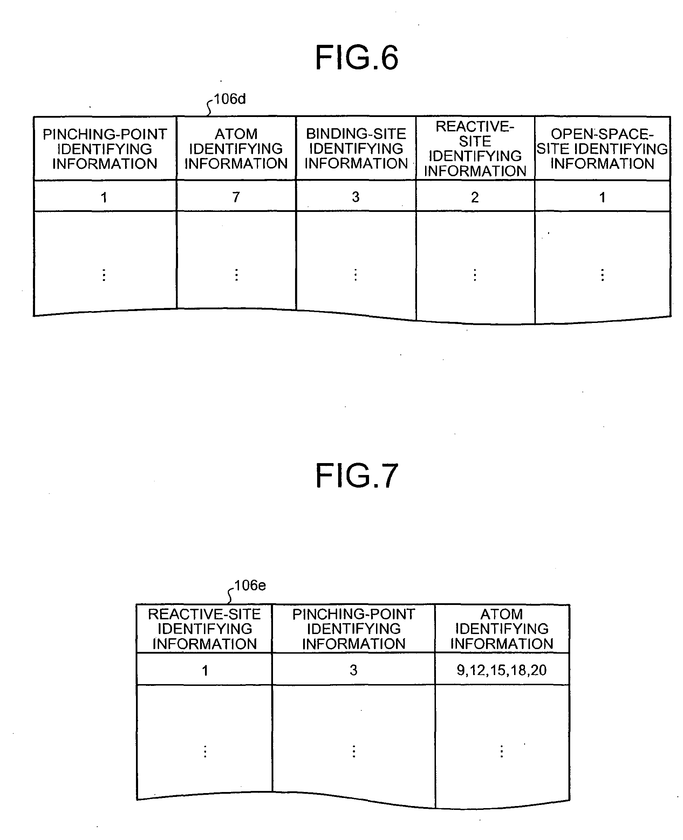

The pinching-point information file 106d is a pinching-point information storage unit that stores pinching point information which is identified by the pinching-point-information identifying unit 102e and which is information on a pinching point being an atom for binding at least a reactive site to a binding site, the reactive site being a site where a metabolic reaction occurs in a compound and a drug-metabolizing enzyme. Information stored in the pinching-point information file 106d is explained below with reference to Fig. 6.

Fig. 6 is a diagram of one example of information stored in the pinching-point information file 106d. As shown in Fig. 6, the information stored in the pinching-point information file 106d includes pinching-point identifying information for uniquely identifying a pinching point, atom identifying information for an atom as the pinching point, binding-site identifying information for a binding site bound to the pinching point, reactive-site identifying information for a reactive site bound to the pinching point, and open-space-site identifying information for an open space site bound to the pinching point, which are mutually associated with one another.

The reactive-site information file 106e is a reactive site information storage unit that stores reactive site information identified by the reactive-site-information identifying unit 102d explained later. Information stored in the reactive-site information file 106e is explained below with reference to Fig. 7.

Fig. 7 is a diagram of one example of information stored in the reactive-site information file 106e. As shown in Fig. 7, the information stored in the reactive-site information file 106e includes reactive-site identifying information for uniquely identifying a reactive site, pinching-point identifying information for a pinching point bound to the reactive site, and atom identifying information for atoms forming the reactive site, which are mutually associated with one another.

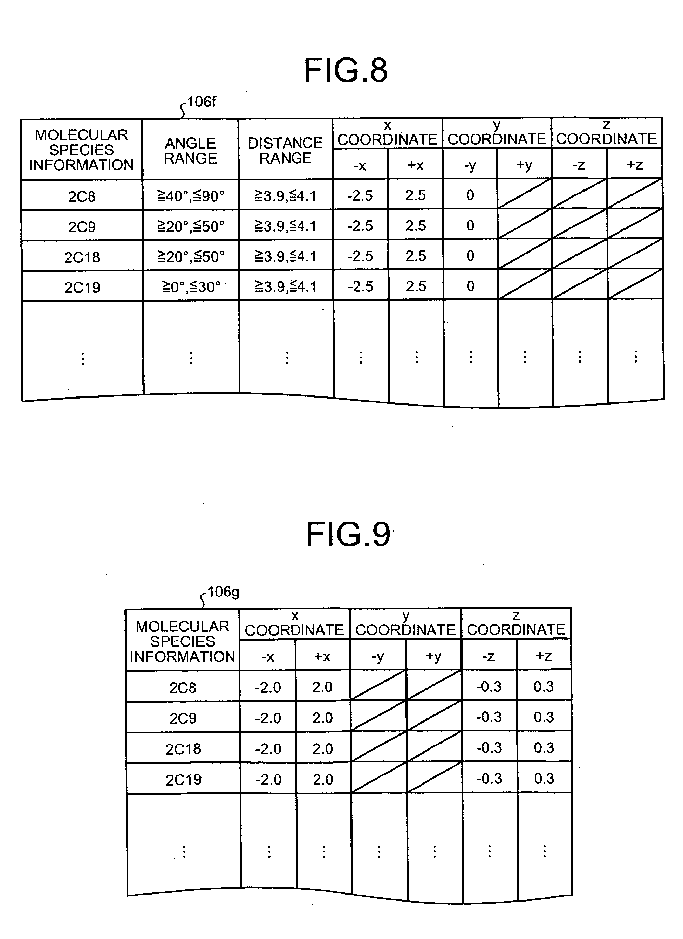

The reactive-site identifying condition file 106f is a reactive-site identifying condition storage unit that stores the reactive-site identifying condition for identifying a reactive site being a site where a metabolic reaction occurs in a compound and a drug-metabolizing enzyme (e.g., condition under which the ranges as follows are defined for each of the predetermine molecular species: a reactive-site coordinate range being a range of atomic coordinate information for atoms forming the reactive site, a reactive-site angle range being a range of an angle value between each of the atoms forming the reactive site and the pinching point, and a reactive-site distance range being a range of a distance value between each of the atoms forming the reactive site and the pinching point). Information stored in the reactive-site identifying condition file 106f is explained below with reference to Fig. 8.

Fig. 8 is a diagram of one example of information stored in the reactive-site identifying condition file 106f. As shown in Fig. 8, the information stored in the reactive-site identifying condition file 106f includes the molecular species information, ranges of the atomic coordinate information for an atom forming a reactive site (range of ±x direction, range of ±y direction, and range of ±z direction), a range of an angle value between the atom forming the reactive site and the pinching point, and a range of a distance value between the atom forming the reactive site and the pinching point, which are mutually associated with one another (in a matrix form).

The pinching-point identifying condition file 106g is a pinching-point identifying condition storage unit that stores the pinching-point identifying condition for identifying a pinching point which is an atom binding at least between a reactive site and a binding site.
Information stored in the pinching-point identifying condition file 106g is explained below with reference to Fig. 9.

Fig. 9 is a diagram of one example of information stored in the pinching-point identifying condition file 106g. As shown in Fig. 9, the information stored in the pinching-point identifying condition file 106g includes the molecular species information, and the ranges of the atomic coordinate information for an atom as a pinching point (range of ±x direction, range of ±y direction, and range of ±z direction), which are mutually associated with one another (in a matrix form).

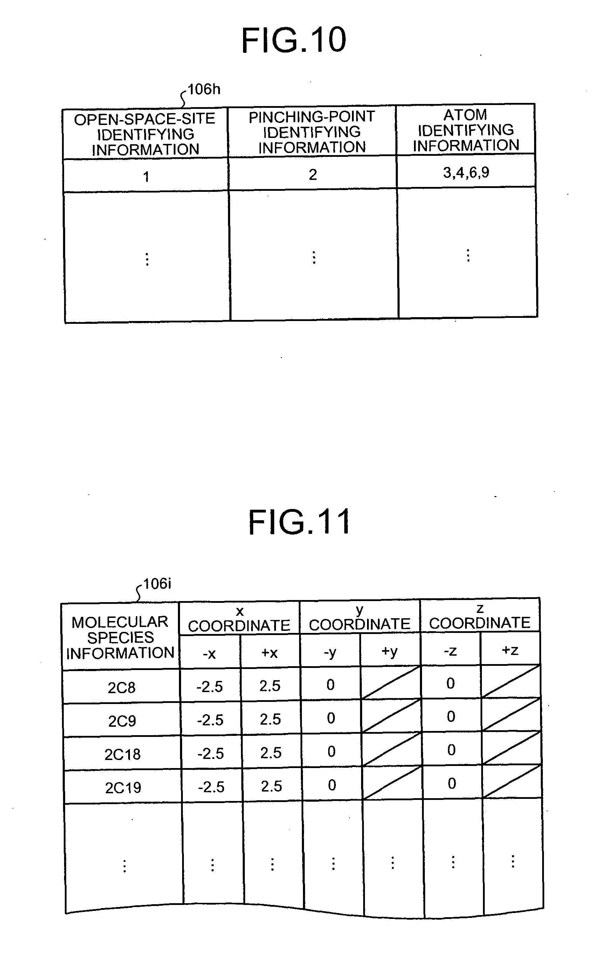

The open-space-site information file 106h is an open-space-site-information storage unit that stores open-space site information identified by the open-space-site-information identifying unit 102g explained later. Information stored in the open-space-site information file 106h is explained below with reference to Fig. 10.

Fig. 10 is a diagram of one example of information stored in the open-space-site information file 106h. As shown in Fig. 10, the information stored in the open-space-site information file 106h includes open-space-site identifying information for uniquely identifying an open space site, pinching-point identifying information for a pinching point bound to the open space site, and the atom identifying information for an atom forming the open space site, which are mutually associated with one another.

The open-space-site identifying condition file 106i is an open-space-site identifying condition storage unit that stores the open-space-site identifying condition for identifying an open space site being a site which is other than the reactive site and the binding site and is bound to the pinching point. Information stored in the open-space-site identifying condition file 106i is explained below with reference to Fig. 11.

Fig. 11 is a diagram of one example of information stored in the open-space-site identifying condition file 106i. As shown in Fig. 11, the information stored in the open-space-site identifying condition file 106i includes the molecular species information, and the ranges of the atomic coordinate information for an atom forming the open space site (range of ±x direction, range of ±y direction, and range of ±z direction), which are mutually associated with one another (in a matrix form).

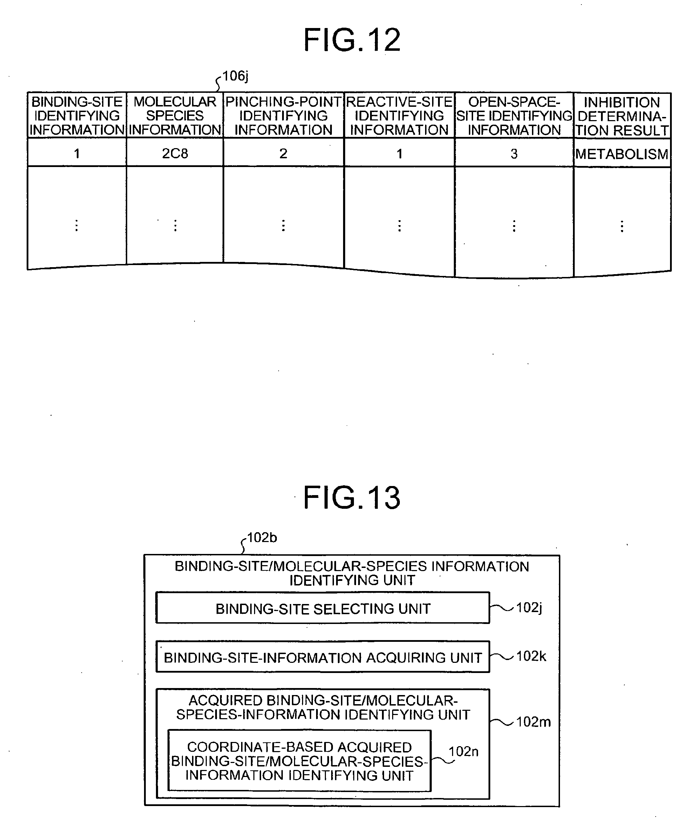

The prediction result file 106j is a prediction-result storage unit that stores a prediction result including at least one of the binding site information, the molecular species information, the reactive site information, the pinching point information, the open-space site information, and the inhibition determination result, for a compound, which are identified. Information stored in the prediction result file 106j is explained below with reference to Fig. 12.

Fig. 12 is a diagram of one example of information stored in the prediction result file 106j. As shown in Fig. 12, the information stored in the prediction result file 106j includes the binding-site identifying information for the binding site identified, the molecular species information identified, the pinching-point identifying information for the pinching point identified, the reactive-site identifying information for the reactive site identified, the open-space-site identifying information for the open space site identified, and the inhibition determination result that is the result of determination on the inhibition of the reactive site in an inhibition determining unit 102h explained later, which are mutually associated with one another.

As other information, the memory unit 106 of the drug-metabolizing enzyme prediction apparatus 100 stores, for example, programs for automatically generating a plurality of conformations.

In Fig. 2, the communication-control interface unit 104 controls communications between the drug-metabolizing enzyme prediction apparatus 100 and the network 300 (or the communication device such as a router). That is, the communication-control interface unit 104 has a function of communicating data with other terminals through the communication line.

In Fig. 2, the input/output-control interface unit 108 controls the input device 112 and the output device 114. As the output device 114, a speaker can be used other than a monitor (including a home television) (hereinafter, the output device 114 may sometimes be described "monitor"). As the input device 112, a keyboard, a mouse, and a microphone can be used. The monitor also implements a function as a pointing device in cooperation with the mouse.

Furthermore, in Fig. 2, the control unit 102 includes an internal memory for storing control programs such as OS (Operating System), programs defining various types of procedures, and required data, and performs information processing to execute various processes by these programs. The control unit 102 functionally includes a compound-structure-information acquiring unit 102a, the binding-site/molecular-species-information identifying unit 102b, the pinching-point/reactive-site-information acquiring unit 102c, the reactive-site-information identifying unit 102d, the pinching-point-information identifying unit 102e, an open-space-site-information acquiring unit 102f, the open-space-site-information identifying unit 102g, the inhibition determining unit 102h, and a prediction-result output unit 102i.

Among these, the compound-structure-information acquiring unit 102a acquires the compound structure information including at least one of the atomic coordinate information being information on coordinates of each of atoms forming a compound, the bond information being information on a bond between the atoms, and the ring structure information being information on a ring structure formed with a plurality of the atoms.

The binding-site/molecular-species-information identifying unit 102b identifies the binding site information being information on an atom forming a binding site in a compound corresponding to the compound structure information, and the molecular species information being information on molecular species, based on the binding-site identifying condition. Here, as shown in Fig. 13, the binding-site/molecular-species-information identifying unit 102b further includes a binding-site selecting unit 102j, a binding-site-information acquiring unit 102k, and an acquired-binding-site/molecular-species-information identifying unit 102m.

Fig. 13 is a block diagram of one example of the binding-site/molecular-species-information identifying unit 102b in the system to which the present invention is applied, and only a portion related to the present invention in the configuration is conceptually shown therein. In Fig. 13, the binding-site selecting unit 102j selects a binding site based on a predetermined binding-site selection criterion. The binding-site-information acquiring unit 102k acquires binding site information from the compound structure information. The acquired-binding-site/molecular-species-information identifying unit 102m identifies the binding site information acquired in the binding-site-information acquiring unit 102k from the compound structure information based on the binding-site identifying condition, and identifies the molecular species corresponding to the binding site information identified, as molecular species information. Here, as shown in Fig. 13, the acquired-binding-site/molecular-species-information identifying unit 102m further includes a coordinate-based acquired-binding-site/molecular-species-information identifying unit 102n. The coordinate-based acquired-binding-site/molecular-species-information identifying unit 102n identifies binding site information when the atomic coordinate information for an atom forming a binding site corresponding to the binding site information acquired in the binding-site-information acquiring unit 102k satisfies a binding-site coordinate range defined by the binding-site identifying condition, and identifies the molecular species corresponding to the binding-site coordinate range satisfied, as molecular species information.

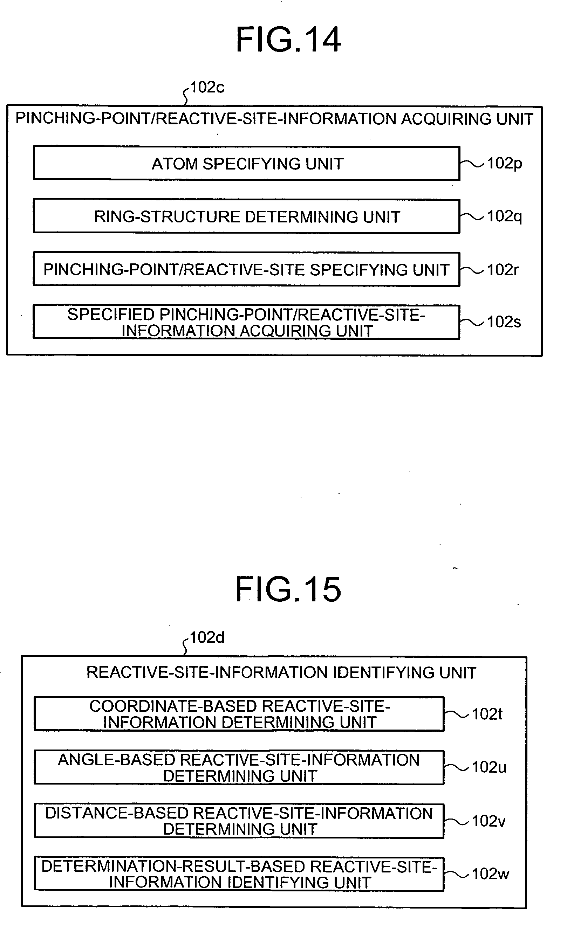

Referring back to Fig. 2, the pinching-point/reactive-site-information acquiring unit 102c specifies a pinching point, and acquires pinching point information for the pinching point specified and reactive site information for a reactive site bound to the pinching point specified, from the binding site information identified and the compound structure information. Here, as shown in Fig. 14, the pinching-point/reactive-site-information acquiring unit 102c further includes an atom specifying unit 102p, a ring-structure determining unit 102q, a pinching-point/reactive-site specifying unit 102r, and a specified-pinching-point/reactive-site-information acquiring unit 102s.

Fig. 14 is a block diagram of one example of the pinching-point/reactive-site-information acquiring unit 102c in the system to which the present invention is applied, and only a portion related to the present invention in the configuration is conceptually shown therein. In Fig. 14, the atom specifying unit 102p specifies a directly bonded atom being an atom bonded to the atom forming a binding site corresponding to the binding site information identified, and specifies an atom bonded to the directly bonded atom, based on the bond information included in the compound structure information. The ring-structure determining unit 102q determines whether the atom specified is an atom forming the ring structure, based on the ring structure information included in the compound structure information. The pinching-point/reactive-site specifying unit 102r specifies the atom specified and another atom forming the ring structure together with the atom as atoms forming the reactive site when it is determined that the atom specified forms the ring structure, and specifies the directly bonded atom specified, as a pinching point. The specified-pinching-point/reactive-site-information acquiring unit 102s acquires the pinching point information for the pinching point specified and the reactive site information for the reactive site specified, from the binding site information and the compound structure information.

Referring back again to Fig. 2, the reactive-site-information identifying unit 102d identifies reactive site information from the molecular species information and the compound structure information, based on the reactive-site identifying condition for identifying a reactive site. As shown in Fig. 15, the reactive-site-information identifying unit 102d further includes a coordinate-based reactive-site-information determining unit 102t, an angle-based reactive-site-information determining unit 102u, a distance-based reactive-site-information determining unit 102v, and a determination-result-based reactive-site-information identifying unit 102w.

Fig. 15 is a block diagram of one example of the reactive-site-information identifying unit 102d in the system to which the present invention is applied, and only a portion related to the present invention in the configuration is conceptually shown therein. In Fig. 15, the coordinate-based reactive-site-information determining unit 102t determines whether atomic coordinate information for an atom forming the reactive site corresponding to the reactive site information satisfies the reactive-site coordinate range defined by the reactive-site identifying condition, in the molecular species corresponding to the molecular species information identified. The angle-based reactive-site-information determining unit 102u calculates an angle value between each of the atoms forming the reactive site corresponding to the reactive site information and the pinching point corresponding to the pinching point information, and determines whether the angle value calculated satisfies the reactive-site angle range defined by the reactive-site identifying condition, in the molecular species corresponding to the molecular species information identified. The distance-based reactive-site-information determining unit 102v calculates a distance value between the atom forming the reactive site corresponding to the reactive site information and the pinching point corresponding to the pinching point information, and determines whether the distance value calculated satisfies the reactive-site distance range defined by the reactive-site identifying condition, in the molecular species corresponding to the molecular species information identified. The determination-result-based reactive-site-information identifying unit 102w identifies the reactive site information determined as being satisfied in the coordinate-based reactive-site-information determining unit 102t, the angle-based reactive-site-information determining unit 102u, and the distance-based reactive-site-information determining unit 102v, as reactive site information that satisfies the reactive-site identifying condition.

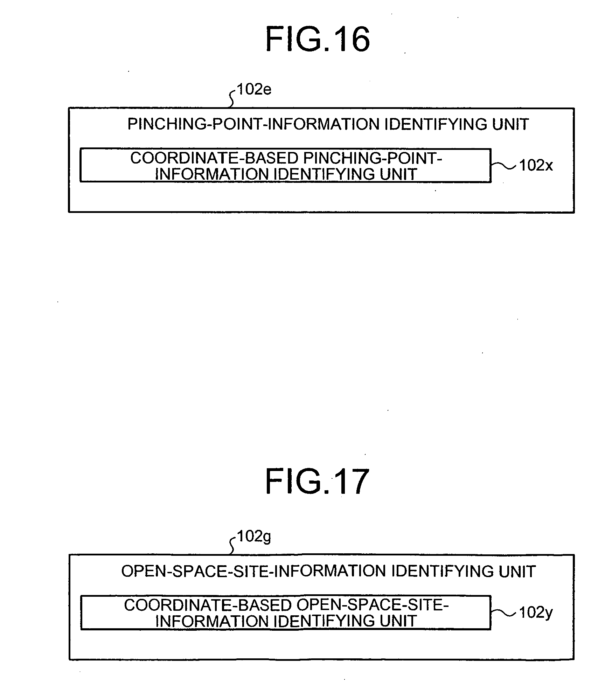

Referring back again to Fig. 2, the pinching-point-information identifying unit 102e identifies pinching point information from the molecular species information and the compound structure information, based on the pinching-point identifying condition. Here, as shown in Fig. 16, the pinching-point-information identifying unit 102e further includes a coordinate-based pinching-point-information identifying unit 102x.

Fig. 16 is a block diagram of one example of the pinching-point-information identifying unit 102e in the system to which the present invention is applied, and only a portion related to the present invention in the configuration is conceptually shown therein. In Fig. 16, the coordinate-based pinching-point-information identifying unit 102x identifies the pinching point information when the atomic coordinate information for the atom corresponding to the pinching point information satisfies the pinching-point coordinate range defined by the pinching-point identifying condition, in the molecular species corresponding to the molecular species information identified.

Referring back again to Fig. 2, if there exists an open space site which is a site other than the reactive site identified and the binding site identified and is bound to the pinching point identified, the open-space-site-information acquiring unit 102f acquires open-space site information being information on an atom forming the open space site, from the binding site information, the reactive site information, the pinching point information, and the compound structure information.

The open-space-site-information identifying unit 102g identifies open-space-site information from the molecular species information and the compound structure information, based on the open-space-site identifying condition. Here, as shown in Fig. 17, the open-space-site-information identifying unit 102g further includes a coordinate-based open-space-site-information identifying unit 102y.

Fig. 17 is a block diagram of one example of the open-space-site-information identifying unit 102g in the system to which the present invention is applied, and only a portion related to the present invention in the configuration is conceptually shown therein. In Fig. 17, the coordinate-based open-space-site-information identifying unit 102y identifies open-space-site information when the atomic coordinate information for an atom forming an open space site corresponding to the open-space-site information satisfies an open-space-site coordinate range defined by the open-space-site identifying condition, in the molecular species corresponding to the molecular species information identified.

Referring back again to Fig. 2, when the reactive site information is identified by the reactive-site-information identifying unit 102d explained later, the inhibition determining unit 102h determines whether an atom forming the reactive site corresponding to the reactive site information or a partial-structure atom group which is a group of part of the atoms in a molecular structure at the reactive site inhibits a metabolic reaction in the compound, based on the inhibition determining condition (e.g., a list of predetermined atoms as inhibitors) for determining whether the metabolic reaction is inhibited in the compound of which the reactive site information is identified.

The prediction-result output unit 102i outputs at least one of the binding site information, the molecular species information, the reactive site information, the pinching point information, the open-space site information, and the inhibition determination result identified in a compound, as a result of prediction for the compound.

Details of the processes in these units are explained later.

### [Process in system]

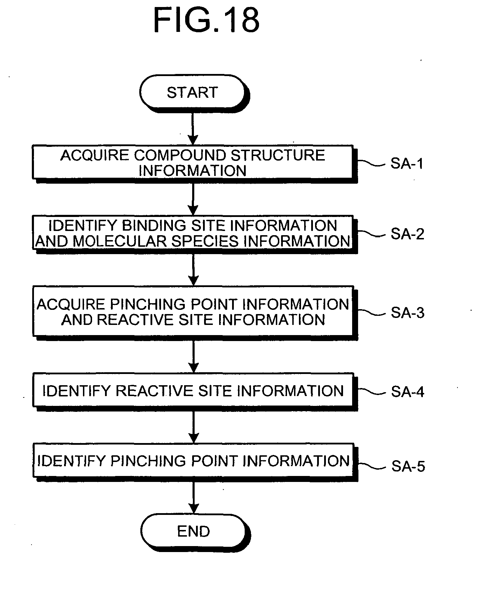

One of the process in the system according to the embodiment configured in the above manner is explained in detail below with reference to Fig. 18 to Fig. 24.

Details of the main process are explained first with reference to Fig. 18. Fig. 18 is a flowchart of one example of the main process in the system according to the embodiment.

At first, in the drug-metabolizing enzyme prediction apparatus 100, the compound-structure-information acquiring unit 102a acquires the compound structure information including at least one of the atomic coordinate information being information on coordinates of each of atoms forming a compound, the bond information being information on a bond between the atoms, and the ring structure information being information on a ring structure formed with a plurality of the atoms, and stores the compound structure information in a predetermined storage area of the compound-structure information file 106a (step SA-1).

At step SA-1, the compound structure information to be acquired may be, for example, compound structure information stored in the external system 200 or the like, compound structure information stored in the compound library, and virtual compound structure information input by a user through the input device 112.

Then, in the drug-metabolizing enzyme prediction apparatus 100, the binding-site/molecular-species-information identifying unit 102b identifies binding site information and molecular species information from the compound structure information acquired at step SA-1, based on the binding-site identifying condition stored in the binding-site identifying condition file 106b, and stores them in a predetermined storage area of the binding-site/molecular-species information file 106c (step SA-2).

At step SA-2, the binding-site/molecular-species-information identifying unit 102b may cause the user to select a binding site based on the predetermined binding-site selection criterion, to acquire binding site information corresponding to the binding site selected from the compound structure information, identify the binding site information acquired, from the compound structure information, based on the binding-site identifying condition, and identify a molecular species corresponding to the binding site information identified, as molecular species information (binding-site/molecular-species-information identifying process). It may also cause the user to select at least one of an original point, and the x-axis, the y-axis, and the z-axis with respect to the original point, from an atom forming a binding site.

After the user selects the binding site, the drug-metabolizing enzyme prediction apparatus 100 may generate a plurality of conformations for the compound structure information, using, for example, automatic conformation generating software (see, for example,
"http://www.rsi.co.jp/kagaku/cs/info/pdf.files/moe.pdf", "http://homepage2.nifty.com/ccsnews2/2002/3q/2002 3Qrsimoe2 00203.htm", and
"http://homepage2.nifty.com/ccsnews2/2003/2q/2003_2Q/ccs03s rsi.htm"), and perform processes at step SA-3 and thereafter as follows for each conformation generated.

The binding-site selection criterion may be specifically defined such that a site is selected as a binding site, the site being a ring structure in a compound or a pseudo-ring structure externally similar to the ring structure (or pseudo-ring structure containing an acid group (e.g., carboxyl and sulfone)). More specifically, as shown in Fig. 26, it may be defined to select a site as a binding site if there is the site containing a bond "S=O" in its structure (e.g., Omeprazole or H259/31) specific to CYP (cytochrome P450) 2C19, and to select a site, as a binding site, where one pseudo-ring structure is formed with three ring structures if Losartan, in its structure specific to CYP2C9 (e.g., Losartan).

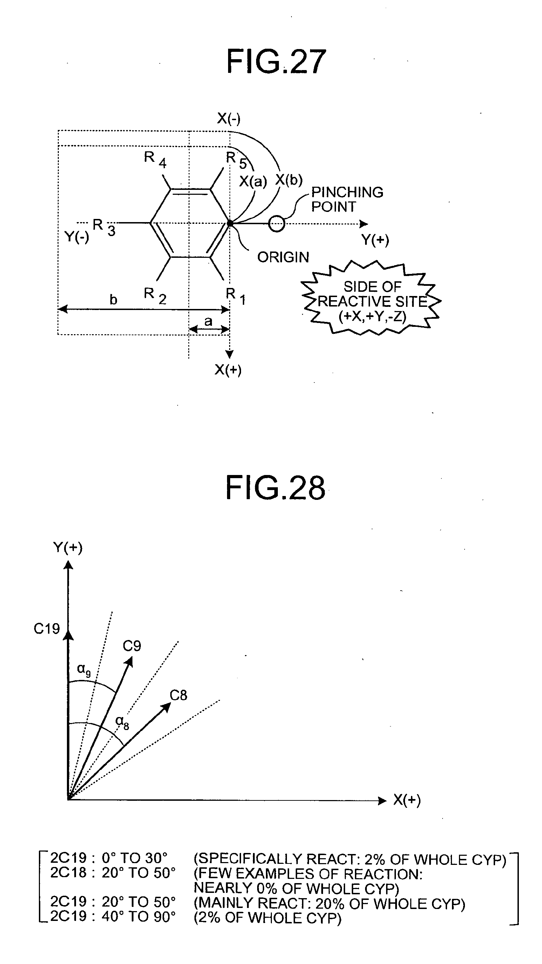

The binding-site identifying condition may be such that each range of a value which each atom forming a binding site can take is conceptually defined for each width (±x direction), depth (±y direction), and thickness (±z direction). More specifically, for example, the width (±x direction) may conceptually be a range decided based on the condition of functional group as follows. The depth (±y direction) may conceptually be a range decided for each molecular species of CYP, and may be a range where a benzene ring is the maximum if, for example, CYP2C19. The thickness (±z direction) may conceptually be a range where the thickness of a mesyl group is the maximum. As shown in Fig. 27, the condition of functional group is such that R1 and R2 form a planar structure and a carboxyl group is the maximum (e.g., if the bond is "=O" and "=S"), R3 and R4 form a planar structure and a benzene ring is the maximum, and R5 becomes the same as that of R1 and R2 (however, CH₃ is also allowed if CYP2C8).

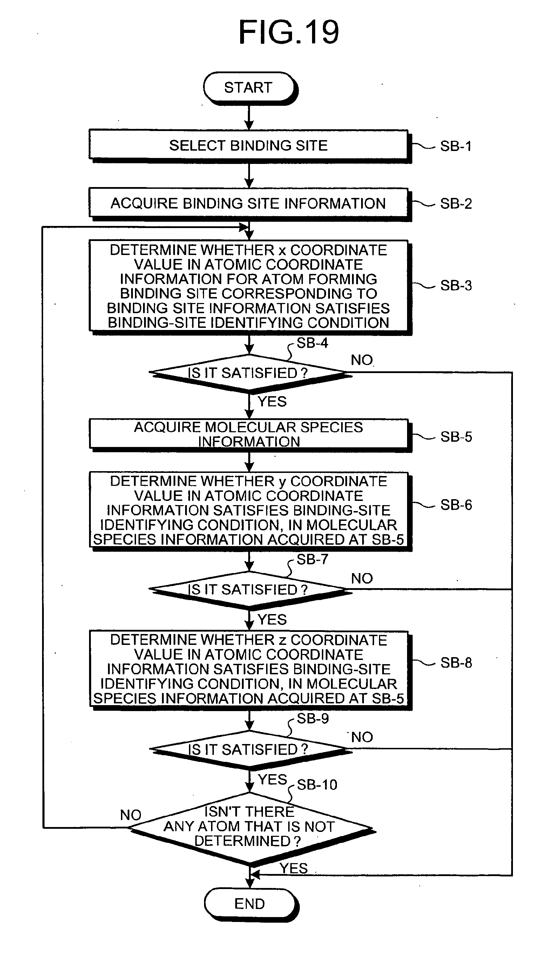

Details of the binding-site/molecular-species-information identifying process are explained below with reference to Fig. 19. Fig. 19 is a flowchart of one example of the binding-site/molecular-species-information identifying process in the system according to the embodiment.

At first, in the binding-site/molecular-species-information identifying unit 102b, the binding-site selecting unit 102j causes the user to select a binding site based on the predetermined binding-site selection criterion (step SB-1).

Then, in the binding-site/molecular-species-information identifying unit 102b, the binding-site-information acquiring unit 102k acquires binding site information for the binding site selected at step SB-1, and stores the binding site information in a predetermined storage area of the binding-site/molecular-species information file 106c (step SB-2).

In the binding-site/molecular-species-information identifying unit 102b, the acquired-binding-site/molecular-species-information identifying unit 102m identifies the binding site information acquired at step SB-2, from the compound structure information, based on the binding-site identifying condition stored in the binding-site identifying condition file 106b, and identifies molecular species corresponding to the binding site information identified, as molecular species information.

More specifically, in the acquired-binding-site/molecular-species-information identifying unit 102m, the coordinate-based acquired-binding-site/molecular-species-information identifying unit 102n determines whether the x coordinate value, in the atomic coordinate information for the atom forming the binding site corresponding to the binding site information acquired at step SB-2, satisfies the binding-site coordinate range (±x direction) defined by the binding-site identifying condition stored in the binding-site identifying condition file 106b (step SB-3).

Then, in the acquired-binding-site/molecular-species-information identifying unit 102m, if "it is satisfied" is determined at step SB-3 (step SB-4: Yes), the coordinate-based acquired-binding-site/molecular-species-information identifying unit 102n acquires molecular species information corresponding to the binding-site coordinate range (±x direction) satisfied, and stores the molecular species information in a predetermined storage area of the binding-site/molecular-species information file 106c (step SB-5).

In the acquired-binding-site/molecular-species-information identifying unit 102m, the coordinate-based acquired-binding-site/molecular-species-information identifying unit 102n determines whether the y coordinate value in the atomic coordinate information satisfies the binding-site coordinate range (±y direction) defined by the binding-site identifying condition stored in the binding-site identifying condition file 106b, in the molecular species corresponding to the molecular species information acquired at step SB-5 (step SB-6).

Then, in the acquired-binding-site/molecular-species-information identifying unit 102m, if "it is satisfied" is determined at step SB-6 (step SB-7: Yes), the coordinate-based acquired-binding-site/molecular-species-information identifying unit 102n determines whether the z coordinate value in the atomic coordinate information satisfies the binding-site coordinate range (±z direction) defined by the binding-site identifying condition stored in the binding-site identifying condition file 106b, in the molecular species corresponding to the molecular species information acquired at step SB-5 (step SB-8).

Then, in the acquired-binding-site/molecular-species-information identifying unit 102m, if "it is satisfied" is determined at step SB-8 (step SB-9: Yes), the coordinate-based acquired-binding-site/molecular-species-information identifying unit 102n returns again to the process at step SB-3 if there is any atom that is not determined (step SB-10: Yes), and identifies the binding site information acquired at step SB-2 if there are no atoms that are not determined (step SB-10: No), in other words, if all the atoms are determined, and identifies the molecular species information acquired at step SB-5.

When "it is not satisfied" is determined at step SB-3, step SB-6, and step SB-8 (step SB-4: No, step SB-7: No, and step SB-9: No), the process is terminated even if there still remains an atom which is not determined.

Now, the binding-site/molecular-species-information identifying process is completed.

Referring back to Fig. 18, in the drug-metabolizing enzyme prediction apparatus 100, the pinching-point/reactive-site-information acquiring unit 102c specifies a pinching point from the binding site information identified at step SA-2 and from the compound structure information acquired at step SA-1, acquires pinching point information for the pinching point specified and reactive site information for a reactive site bound to the pinching point corresponding to the pinching point information, and stores them in a predetermined storage area of the pinching-point information file 106d and in a predetermined storage area of the reactive-site information file 106e, respectively (step SA-3).

At step SA-3, the pinching-point/reactive-site-information acquiring unit 102c may also be configured to specify a directly bonded atom being an atom bonded to the atom forming the binding site corresponding to the binding site information identified at step SA-2, and also specify an atom bonded to the directly bonded atom, based on the bond information included in the compound structure information; determine whether the atom specified is an atom forming the ring structure, based on the ring structure information included in the compound structure information; specify, if it is determined that the atom specified is an atom forming the ring structure, this atom specified and another atom forming the ring structure together with this atom as atoms forming the reactive site; to specify the directly bonded atom specified, as a pinching point; and acquire the pinching point information for the pinching point specified and the reactive site information for the reactive site specified, from the binding site information and the compound structure information, respectively (pinching-point/reactive-site-information acquiring process).

If it is determined that the atom specified is not an atom forming the ring structure, it is determined whether the directly bonded atom is an atom forming the ring structure. If it is determined that the directly bonded atom is an atom forming the ring structure, an atom, which forms a binding site corresponding to the binding site information identified at step SA-2 and is bonded to the directly bonded atom, is specified as a pinching point, and the directly bonded atom and another atom forming the ring structure together with the directly bonded atom may be specified as atoms forming a reactive site. In other words, the atom forming the binding site may sometimes be a pinching point.

The atom specified as the pinching point may be an atom bonded to the atom forming the ring structure, selected from among atoms each located, for example, one bond away from the atom forming the binding site.

It may also be considered that a compound is suppressed by CYP at the most strict point of an allowable range of the atomic coordinate information (e.g., ±x direction) between an atom forming the binding site and a pinching point bound to the atom.

The pinching-point/reactive-site-information acquiring unit 102c may acquire a plurality pieces of pinching point information. In this case, the processes for identifying pinching point information and identifying reactive site information explained later are performed on the pinching point information and the corresponding reactive site information, respectively.

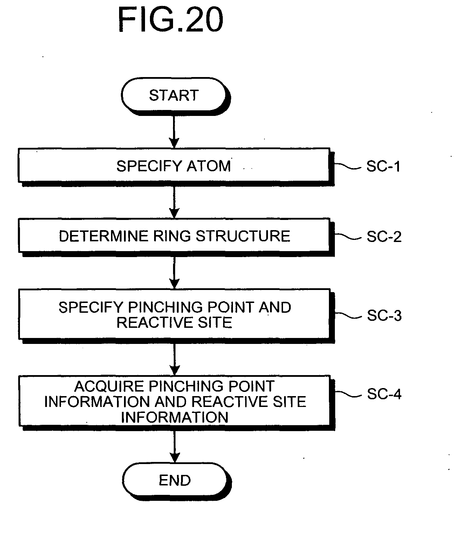

Details of the pinching-point/reactive-site-information acquiring process are explained below with reference to Fig. 20. Fig. 20 is a flowchart of one example of the pinching-point/reactive-site-information acquiring process in the system according to the embodiment.

At first, in the pinching-point/reactive-site-information acquiring unit 102c, the atom specifying unit 102p specifies a directly bonded atom and an atom bonded to the directly bonded atom, based on the bond information included in the compound structure information (step SC-1).

Then, the ring-structure determining unit 102q in the pinching-point/reactive-site-information acquiring unit 102c determines whether the atom specified at step SC-1 is an atom forming the ring structure based on the ring structure information included in the compound structure information (step SC-2).

Then, if it is determined that the atom specified at step SC-2 is an atom forming the ring structure, the pinching-point/reactive-site specifying unit 102r in the pinching-point/reactive-site-information acquiring unit 102c specifies the atom specified and another atom forming the ring structure together with the atom, as atoms forming a reactive site, and specifies the directly bonded atom specified, as a pinching point (step SC-3).

If it is determined at step SC-3 that the atom specified is not an atom forming the ring structure, the ring-structure determining unit 102q in the pinching-point/reactive-site-information acquiring unit 102c determines whether the directly bonded atom is an atom forming the ring structure. If it is determined that the directly bonded atom is an atom forming the ring structure, the pinching-point/reactive-site specifying unit 102r specifies the atom, which forms the binding site corresponding to the binding site information identified and is bonded to the directly bonded atom, as a pinching point.

Then, the specified-pinching-point/reactive-site-information acquiring unit 102s in the pinching-point/reactive-site-information acquiring unit 102c acquires pinching point information for the pinching point specified at step SC-3 and reactive site information for the reactive site specified from the binding site information and the compound structure information, and stores these information in a predetermined storage area of the pinching-point information file 106d and in a predetermined storage area of the reactive-site information file 106e, respectively (step SC-4).

Now, the pinching-point/reactive-site-information acquiring process is completed.

Referring back to Fig. 18, the reactive-site-information identifying unit 102d in the drug-metabolizing enzyme prediction apparatus 100 identifies the reactive site information acquired at step SA-3 from the molecular species information and the compound structure information, based on the reactive-site identifying condition stored in the reactive-site identifying condition file 106f (step SA-4).

The reactive-site identifying condition used at step SA-4 may be a condition such that the reactive-site coordinate range, the reactive-site angle range, and the reactive-site distance range are defined for each of the predetermine molecular species. Alternatively, the reactive-site identifying condition may be processes of determining whether the atomic coordinate information for an atom forming the reactive site corresponding to the reactive site information acquired at step SA-3 satisfies the reactive-site coordinate range in the molecular species corresponding to the molecular species information identified at step SA-2; calculating an angle value between the atom forming the reactive site corresponding to the reactive site information and the pinching point corresponding to the pinching point information; determining whether the angle value calculated satisfies the reactive-site angle range in the molecular species corresponding to the molecular species information identified at step SA-2; calculating a distance value between the atom forming the reactive site corresponding to the reactive site information and the pinching point corresponding to the pinching point information; determining whether the distance value calculated satisfies the reactive-site distance range in the molecular species corresponding to the molecular species information identified at step SA-2; and identifying the reactive site information determined that it satisfies the reactive-site coordinate range, the reactive-site angle range, and the reactive-site distance range, as reactive site information that satisfies the reactive-site identifying condition (reactive-site-information identifying process).

The reactive-site coordinate range may be such that a range of a value, which each atom forming the reactive site can take, is conceptually defined for each of width (±x direction), depth (±y direction), and thickness (±z direction). More specifically, for example, the width (±x direction) may conceptually be a range (extension in +x direction from, for example, the binding site) similar to the range in the binding-site identifying condition. The depth (±y direction) may conceptually be a range defined for each molecular species of CYP, and may be a narrow range if, for example, CYP2C9, and may be a wide range if CYP2C19. Furthermore, the thickness (±z direction) may conceptually be a range such that the thickness of cyclohexane (chair form) is the maximum.

As shown in Fig. 28, the reactive-site angle range may conceptually be a range of an angle value from a coordinate axis between atoms when the atom as the pinching point and the atom forming the reactive site are projected to a previously defined two-dimensional coordinate system (two-dimensional coordinate system in which the pinching point is set as the origin in Fig. 28).

The reactive-site distance range may conceptually be a range of a linear distance between the atom as the pinching point and the atom forming the reactive site. More specifically, as shown in Fig. 29, the reactive-site distance range may be a range of a distance (e.g., distance on the z coordinate) between a pinching point (in Fig. 29, atom with number of "1") and an atom apart from the pinching point by five carbons (in Fig. 29, atom with number of "5").

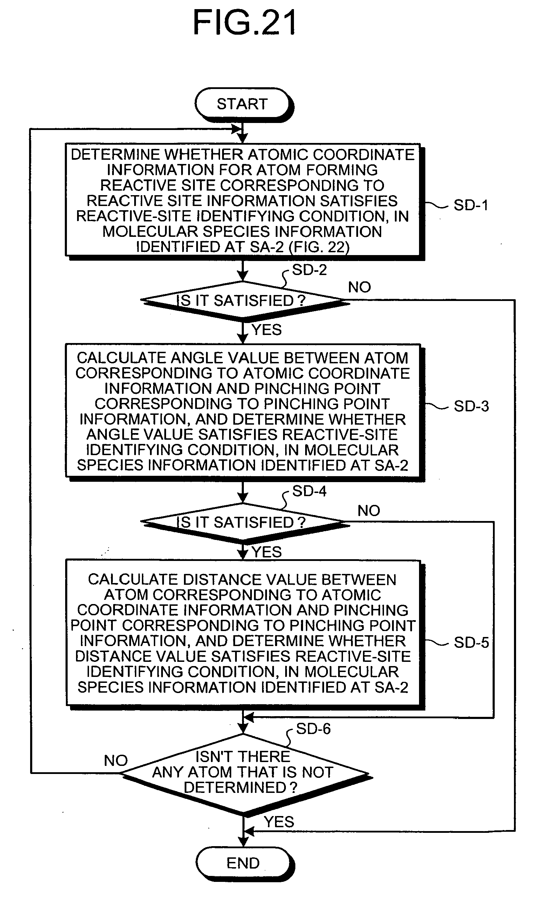

Details of the reactive-site-information identifying process are explained below with reference to Fig. 21. Fig. 21 is a flowchart of one example of the reactive-site-information identifying process in the system according to the embodiment.

At first, in the reactive-site-information identifying unit 102d, the coordinate-based reactive-site-information determining unit 102t determines whether the atomic coordinate information for an atom forming the reactive site corresponding to the reactive site information acquired at step SA-3 satisfies the reactive-site coordinate range defined by the reactive-site identifying condition stored in the reactive-site identifying condition file 106f, in the molecular species corresponding to the molecular species information identified at step SA-2 (step SD-1) (reactive-site coordinate range determining process).

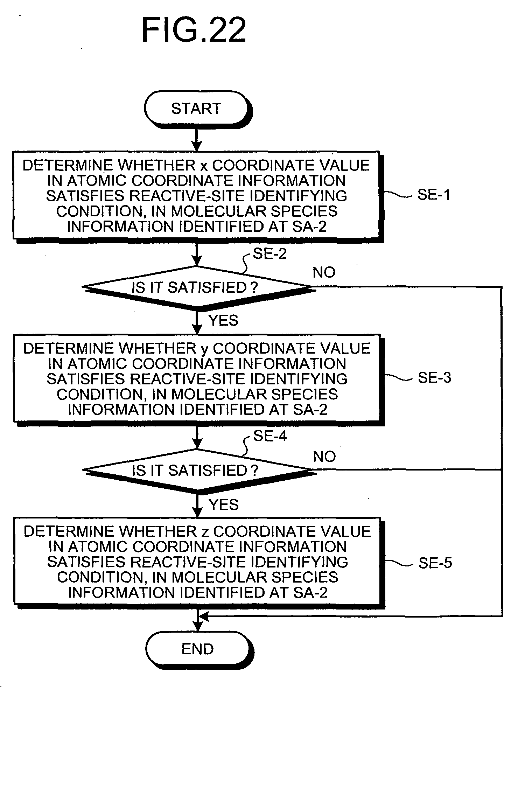

Details of the reactive-site coordinate range determining process performed at step SD-1 are explained below with reference to Fig. 22. Fig. 22 is a flowchart of one example of the reactive-site coordinate range determining process in the system according to the embodiment.

At first, in the reactive-site-information identifying unit 102d, the coordinate-based reactive-site-information determining unit 102t determines whether the x coordinate value in the atomic coordinate information for the atom forming the reactive site corresponding to the reactive site information acquired at step SA-3 satisfies the reactive-site coordinate range (±x direction) defined by the reactive-site identifying condition stored in the reactive-site identifying condition file 106f, in the molecular species corresponding to the molecular species information identified at step SA-2 (step SE-1).

Then, if "it is satisfied" is determined at step SE-1 (step SE-2: Yes), the coordinate-based reactive-site-information determining unit 102t in the reactive-site-information identifying unit 102d determines whether the y coordinate value in the atomic coordinate information satisfies the reactive-site coordinate range (±y direction) defined by the reactive-site identifying condition stored in the reactive-site identifying condition file 106f, in the molecular species corresponding to the molecular species information identified at step SA-2 (step SE-3).

Then, if "it is satisfied" is determined at step SE-3 (step SE-4: Yes), the coordinate-based reactive-site-information determining unit 102t in the reactive-site-information identifying unit 102d determines whether the z coordinate value in the atomic coordinate information satisfies the reactive-site coordinate range (±z direction) defined by the reactive-site identifying condition stored in the reactive-site identifying condition file 106f, in the molecular species corresponding to the molecular species information identified at step SA-2 (step SE-5).

If "it is not satisfied" is determined at step SE-1 and step SE-3 (step SE-2: No and step SE-4: No), the process is terminated.

Now, the reactive-site coordinate range determining process is completed.

Referring back again to Fig. 21, in the reactive-site-information identifying unit 102d, if "it is satisfied" is determined at step SD-1 (if "it is satisfied" is determined at step SE-5) (step SD-2: Yes), the angle-based reactive-site-information determining unit 102u calculates an angle value between the atom forming the reactive site corresponding to the reactive site information and the pinching point corresponding to the pinching point information, and determines whether the angle value calculated satisfies the reactive-site angle range defined by the reactive-site identifying condition stored in the reactive-site identifying condition file 106f, in the molecular species corresponding to the molecular species information identified at step SA-2 (step SD-3).

Then, in the reactive-site-information identifying unit 102d, if "it is satisfied" is determined at step SD-3 (step SD-4: Yes), the distance-based reactive-site-information determining unit 102v calculates a distance value between the atom forming the reactive site corresponding to the reactive site information and the pinching point corresponding to the pinching point information, and determines whether the distance value calculated satisfies the reactive-site distance range defined by the reactive-site identifying condition stored in the reactive-site identifying condition file 106f, in the molecular species corresponding to the molecular species information identified at step SA-2 (step SD-5).

In the reactive-site-information identifying unit 102d, the determination-result-based reactive-site-information identifying unit 102w returns again to the process at step SD-1 if there is any atom that is not determined yet (step SD-6: Yes), and identifies the reactive site information acquired at step SA-3 if there are no atoms that are not determined (step SB-6: No), in other words, if all the atoms are determined. More specifically, when it is determined that the atomic coordinate information for all the atoms forming the reactive site corresponding to the reactive site information acquired at step SA-3 "is satisfied" at step SD-1, and when it is determined that the atomic coordinate information for the atoms forming the reactive site corresponding to the reactive site information acquired at step SA-3 "is satisfied" at step SD-3 and step SD-5, the reactive site information acquired at SA-3 is identified.

If "it is not satisfied" is determined at step SD-1 (step SD-2: No), the process is terminated even if there still remains an atom which is not determined. If "it is not satisfied" is determined at step SD-3 (step SD-4: No), the process proceeds to step SD-6 as shown in Fig. 21.

Now, the reactive-site-information identifying process is completed.

Referring back again to Fig. 18, in the drug-metabolizing enzyme prediction apparatus 100, the pinching-point-information identifying unit 102e identifies the pinching point information acquired at step SA-3 from the molecular species information and the compound structure information, based on the pinching-point identifying condition stored in the pinching-point identifying condition file 106g (step SA-5).

The pinching-point identifying condition used at step SA-5 may be a condition such that the pinching-point coordinate range, which is a range of the atomic coordinate information for an atom as the pinching point, is defined for each of the predetermine molecular species. Alternatively, the pinching-point-information identifying unit 102e may identify the pinching point information when the atomic coordinate information for the atom as the pinching point corresponding to the pinching point information acquired at step SA-3 satisfies the pinching-point coordinate range, in the molecular species corresponding to the molecular species information identified at step SA-2 (pinching-point-information identifying process).

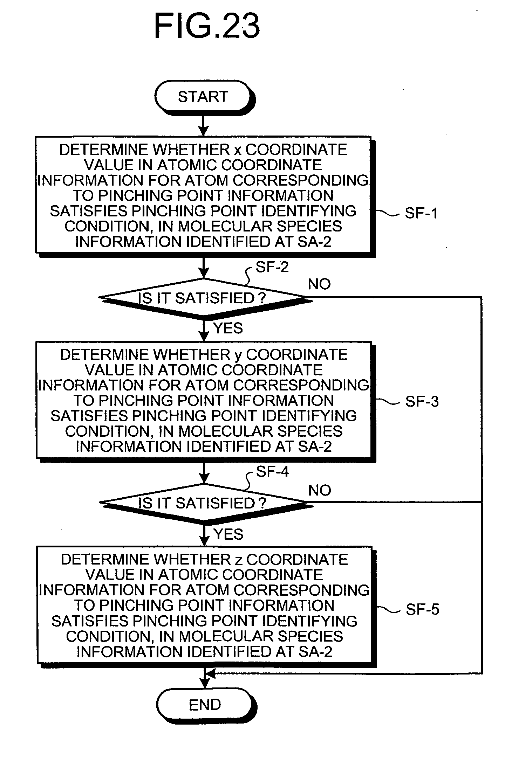

Details of the pinching-point-information identifying process are explained below with reference to Fig. 23. Fig. 23 is a flowchart of one example of the pinching-point-information identifying process in the system according to the embodiment.

At first, in the pinching-point-information identifying unit 102e, the coordinate-based pinching-point-information identifying unit 102x determines whether the x coordinate value in the atomic coordinate information for the atom corresponding to the pinching point information acquired at step SA-3 satisfies the pinching-point coordinate range (±x direction) defined by the pinching-point identifying condition stored in the pinching-point identifying condition file 106g, in the molecular species corresponding to the molecular species information identified at step SA-2 (step SF-1).

Then, in the pinching-point-information identifying unit 102e, if "it is satisfied" is determined at step SF-1 (step SF-2: Yes), the coordinate-based pinching-point-information identifying unit 102x determines whether the y coordinate value in the atomic coordinate information for the atom corresponding to the pinching point information satisfies the pinching-point coordinate range (±y direction) defined by the pinching-point identifying condition stored in the pinching-point identifying condition file 106g, in the molecular species corresponding to the molecular species information identified at step SA-2 (step SF-3).

Then, in the pinching-point-information identifying unit 102e, if "it is satisfied" is determined at step SF-3 (step SF-4: Yes), the coordinate-based pinching-point-information identifying unit 102x determines whether the z coordinate value in the atomic coordinate information for the atom corresponding to the pinching point information satisfies the pinching-point coordinate range (±z direction) defined by the pinching-point identifying condition stored in the pinching-point identifying condition file 106g, in the molecular species corresponding to the molecular species information identified at step SA-2 (step SF-5).

Then, in the pinching-point-information identifying unit 102e, if "it is satisfied" is determined at step SF-5, the coordinate-based pinching-point-information identifying unit 102x identifies the pinching point information acquired at step SA-3.

The pinching point identified may serve an atom which forms the binding site as a result.

Now, the pinching-point-information identifying process is completed.

In the drug-metabolizing enzyme prediction apparatus 100, if there exists an open space site being a site which is other than the reactive site corresponding to the reactive site information identified at step SA-4 and the binding site corresponding to the binding site information identified at step SA-2 and is bound to the pinching point corresponding to the pinching point information identified at step SA-5, the open-space-site-information acquiring unit 102f may acquire open-space site information being information on an atom forming the open space site from the binding site information, the reactive site information, the pinching point information, and the compound structure information, and store the open-space site information in a predetermined storage area of the open-space-site information file 106h; and the open-space-site-information identifying unit 102g may also identify the open-space site information acquired, from the molecular species information and the compound structure information, based on the open-space-site identifying condition stored in the open-space-site identifying condition file 106i for identifying the open space site. The open-space-site identifying condition may be a condition such that the open-space-site coordinate range, which is a range of the atomic coordinate information for an atom forming an open space site is defined for each of the predetermine molecular species. Alternatively, in the open-space-site-information identifying unit 102g, the coordinate-based open-space-site-information identifying unit 102y may identify open-space site information when the atomic coordinate information for the atom forming the open space site corresponding to the open-space site information acquired satisfies the open-space-site coordinate range defined by the open-space-site identifying condition stored in the open-space-site identifying condition file 106i, in the molecular species corresponding to the molecular species information identified (open-space-site-information identifying process).

Furthermore, as shown in Fig. 30, the open-space-site coordinate range may conceptually be a range such that the range of a value which each atom forming the open space site can take is defined by each of the width (±x direction), the depth (±y direction), and the thickness (±z direction).

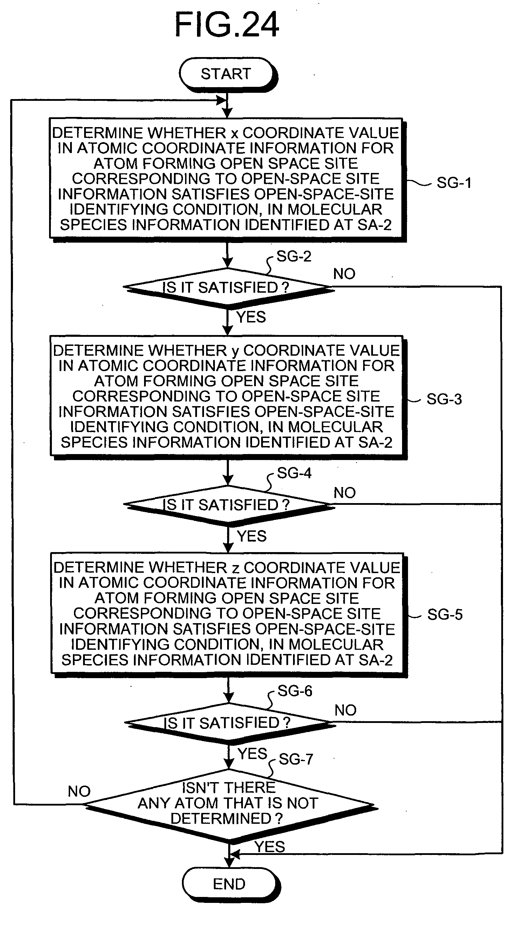

Details of the open-space-site-information identifying process are explained below with reference to Fig. 24. Fig. 24 is a flowchart of one example of the open-space-site-information identifying process in the system according to the embodiment.

At first, in the open-space-site-information identifying unit 102g, the coordinate-based open-space-site-information identifying unit 102y determines whether the x coordinate value in the atomic coordinate information for the atom forming the open space site corresponding to the open-space site information acquired by the open-space-site-information acquiring unit 102f satisfies the open-space-site coordinate range (±x direction) defined by the open-space-site identifying condition stored in the open-space-site identifying condition file 106i, in the molecular species corresponding to the molecular species information identified at step SA-2 (step SG-1).

Then, in the open-space-site-information identifying unit 102g, if "it is satisfied" is determined at step SG-1 (step SG-2: Yes), the coordinate-based open-space-site-information identifying unit 102y determines whether the y coordinate value in the atomic coordinate information satisfies the open-space-site coordinate range (±y direction) defined by the open-space-site identifying condition stored in the open-space-site identifying condition file 106i, in the molecular species corresponding to the molecular species information identified at step SA-2 (step SG-3).

Then, in the open-space-site-information identifying unit 102g, if "it is satisfied" is determined at step SG-3 (step SG-4: Yes), the coordinate-based open-space-site-information identifying unit 102y determines whether the z coordinate value in the atomic coordinate information satisfies the open-space-site coordinate range (±z direction) defined by the open-space-site identifying condition stored in the open-space-site identifying condition file 106i, in the molecular species corresponding to the molecular species information identified at step SA-2 (step SG-5).

Then, in the open-space-site-information identifying unit 102g, if "it is satisfied" is determined at step SG-5 (step SG-6: Yes), the coordinate-based open-space-site-information identifying unit 102y returns again to the process at step SG-1 if there is any atom that is not determined yet (step SG-7: Yes), and identifies the open-space site information acquired in the open-space-site-information acquiring unit 102f if there are no atoms that are not determined (step SB-7: No), in other words, if all the atoms are determined.

When "it is not satisfied" is determined at step SG-1, step SG-3, and step SG-5 (step SG-2: No, step SG-4: No, and step SG-6: No), the process is terminated even if there still remains an atom which is not determined.

Now, the open-space-site-information identifying process is completed.

In the drug-metabolizing enzyme prediction apparatus 100, if the reactive site information is identified at step SA-4, the inhibition determining unit 102h may determine whether an atom forming the reactive site corresponding to the reactive site information or a partial-structure atom group which is a group of part of atoms in the molecular structure at the reactive site inhibits a metabolic reaction in a compound, based on the inhibition determining condition (e.g., a list of predetermined atoms as inhibitors) for determining whether the metabolic reaction is inhibited in the compound of which the reactive site information is identified. More specifically, in the drug-metabolizing enzyme prediction apparatus 100, if the molecular species of the drug-metabolizing enzyme bound to the compound and the reactive site where a metabolic reaction occurs in the compound bound to the drug-metabolizing enzyme, are identified by the process, the inhibition determining unit 102h may determine whether any atom (e.g., amide (N)) inhibiting the metabolic reaction is contained in atoms forming the reactive site of the compound.

In the drug-metabolizing enzyme prediction apparatus 100, the prediction-result output unit 102i may store at least one of the binding site information, the molecular species information, the reactive site information, the pinching point information, and the open-space site information, for the compound, and the inhibition determination result, in a predetermined storage area of the prediction result file 106j, as a prediction result for the compound, and output the prediction result through the output device 114.

Now, the main process is completed.

As explained above, according to the embodiment, in the drug-metabolizing enzyme prediction apparatus 100, the compound-structure-information acquiring unit 102a acquires the compound structure information including at least one of the atomic coordinate information being information on coordinates of each of atoms forming a compound, the bond information being information on a bond between the atoms, and the ring structure information being information on a ring structure formed with a plurality of the atoms. The binding-site/molecular-species-information identifying unit 102b identifies the binding site information being information on the atom forming the binding site in the compound corresponding to the compound structure information and the molecular species information being information on the molecular species, from the compound structure information acquired, based on the binding-site identifying condition for identifying the binding site being a site where the compound and the drug-metabolizing enzyme are bound to each other and identifying the molecular species of the drug-metabolizing enzyme to be bound. The pinching-point/reactive-site-information acquiring unit 102c specifies the pinching point which is an atom binding at least the reactive site where a metabolic reaction occurs in the compound and the drug-metabolizing enzyme to the binding site, and acquires the pinching point information being the information on the atom as the pinching point specified and the reactive site information being information on the atom forming the reactive site bound to the pinching point corresponding to the pinching point information, from the binding site information and the compound structure information identified. The reactive-site-information identifying unit 102d identifies the reactive site information acquired, from the molecular species information and the compound structure information, based on the reactive-site identifying condition for identifying the reactive site. The pinching-point-information identifying unit 102e identifies the pinching point information acquired, from the molecular species information and the compound structure information, based on the pinching-point identifying condition for identifying the pinching point. Therefore, it is possible to accurately predict a molecular species of a drug-metabolizing enzyme and a reactive site of a compound with the drug-metabolizing enzyme, for all types of compounds without using training data for prediction and in consideration of each cubic form of the compounds.

That is, the drug-metabolizing enzyme prediction apparatus 100 allows accurate prediction of a molecular species of a drug-metabolizing enzyme and a reactive site of a compound with the drug-metabolizing enzyme, for all types of compounds without using training data for prediction and in consideration of each cubic form of the compounds. Therefore, a compound group can accurately be narrowed down, for example, in the early stage of drug development.

In the drug-metabolizing enzyme prediction apparatus 100, if the reactive site information is identified at step SA-4, the inhibition determining unit 102h may determine whether an atom forming a reactive site corresponding to the reactive site information or a partial-structure atom group which is a group of part of atoms in the molecular structure at the reactive site inhibits a metabolic reaction, based on the inhibition determining condition (e.g., a list of predetermined atoms as inhibitors) for determining whether the metabolic reaction is inhibited in the compound of which reactive site information is identified. Therefore, it is possible to determine (check) whether any atom inhibiting the metabolic reaction is contained in the atoms forming the reactive site predicted, and this allows the compound group to be more accurately narrowed.

### [Example]

An example is applied is explained below with reference to Fig. 31. Fig. 31 is a diagram for explaining one example of the main process in the drug-metabolizing enzyme prediction apparatus 100 according to an example of the present invention.

At first, in the drug-metabolizing enzyme prediction apparatus 100, the compound-structure-information acquiring unit 102a according to the embodiment acquires a compound library (which corresponds to the compound structure information according to the embodiment) stored in the external system 200 (step SH-1: compound-library acquiring process).

Then, in the drug-metabolizing enzyme prediction apparatus 100, if the compound library cannot be acquired at step SH-1 (step SH-2: No), the compound-structure-information acquiring unit 102a according to the embodiment acquires a virtual compound library input by the user (step SH-3: virtual-compound-library acquiring process).

Then, in the drug-metabolizing enzyme prediction apparatus 100, the binding-site/molecular-species-information identifying unit 102b according to the embodiment identifies the binding site information from the compound library acquired at step SH-1 or the virtual compound library acquired at step SH-3 (step SH-4: binding-site identifying process).

Then, in the drug-metabolizing enzyme prediction apparatus 100, the pinching-point-information identifying unit 102e according to the embodiment identifies the pinching point information from the compound library acquired at step SH-1 or the virtual compound library acquired at step SH-3 (step SH-5: pinching-point identifying process).

Then, in the drug-metabolizing enzyme prediction apparatus 100, the open-space-site-information identifying unit 102g according to the embodiment identifies the open-space site information from the compound library acquired at step SH-1 or the virtual compound library acquired at step SH-3 (step SH-6: open-space-site identifying process).

Then, in the drug-metabolizing enzyme prediction apparatus 100, the reactive-site-information identifying unit 102d according to the embodiment identifies the reactive site information from the compound library acquired at step SH-1 or the virtual compound library acquired at step SH-3 (step SH-7: reactive site identifying process).

Then, in the drug-metabolizing enzyme prediction apparatus 100, the control unit 102 according to the embodiment in the drug-metabolizing enzyme prediction apparatus 100 confirms the molecular species of a drug-metabolizing enzyme (CYP) based on the identification results at step SH-4 to step SH-7, and the inhibition determining unit 102h according to the embodiment further determines whether any atom inhibiting the metabolic reaction is contained in the atoms forming the reactive site of the compound in which the binding site, the pinching point, the open space site, and the reactive site are identified at step SH-4 to step SH-7 (step SH-8: CYP confirming process).

Then, in the drug-metabolizing enzyme prediction apparatus 100, the prediction-result output unit 102i according to the embodiment stores the binding site, the pinching point, the open space site, and the reactive site identified at step SH-4 to step SH-7, and the metabolic prediction result including the molecular species and the metabolic inhibition result confirmed at step SH-8, in the prediction result file 106j according to the embodiment (metabolic-prediction-result database), and outputs (displays) them to (on) the output device 114 (e.g., monitor) according to the embodiment (step SH-9).

According to the example, by respectively evaluating the binding site, the pinching point, the reactive site, and the open space site, it is possible to accurately predict a molecular species and a reactive site of a drug-metabolizing enzyme for all types of compounds, and also efficiently verify whether a metabolic reaction actually occurs at the reactive site predicted. This allows a useful compound group to be accurately narrowed, for example, in the early stage of drug development.

### INDUSTRIAL APPLICABILITY

The drug-metabolizing enzyme prediction apparatus, the drug-metabolizing enzyme prediction method, and the recording medium according to the present invention are useful for prediction of a molecular species and a reactive site of a drug-metabolizing enzyme, and particularly suitable for narrowing of a compound group in the field of drug discovery (e.g., a metabolic section and a synthesis section of drug discovery) in which drug design and the like are conducted.

## Claims

1. An apparatus comprising:
a first acquiring unit (102a) arranged to acquire, from a compound-structure information database, compound structure information including atomic coordinate information of each of a plurality of atoms forming a compound, bond information between the atoms, and ring structure information on a ring structure formed with the atoms;
a first identifying unit (102b) arranged to identify binding site information on an atom in the compound corresponding to the compound structure information and molecular species information, from the compound structure information acquired by the first acquiring unit, based on a binding-site identifying condition for identifying the molecular species of a drug-metabolizing enzyme belonging to the CYP families 1-4 of cytochromes P450 to be bound and for identifying a site in the compound where the compound and the drug-metabolizing enzyme are bound to each other, the binding-site identifying condition being a condition under which a binding-site coordinate range, which is a range of atomic coordinate information for an atom forming a binding site, is defined for each of the predetermined molecular species;
second acquiring unit (102c) arranged to specify a pinching point that is the atom binding the binding site to a reactive site in which a metabolic reaction of the compound and the drug-metabolizing enzyme occurs, the pinching point and the reactive site being in the same compound as the binding site, from the binding site information identified by the first identifying unit (102b) and the compound structure information, and to acquire pinching point information on the atom of the specified pinching point and the reactive site information on the atom forming the reactive site bound to the pinching point;
a second identifying unit (102e) arranged to identify the pinching point information acquired by the second acquiring unit (102c), from the molecular species information and the compound structure information, based on a pinching-point identifying condition, the pinching-point identifying condition being a condition under which a pinching-point coordinate range, which is a range of the atomic coordinate information for an atom as the pinching point, is defined for each of the predetermined molecular species;
a third identifying unit (102d) arranged to identify the reactive site information acquired by the second acquiring unit (102c), from the molecular species information and the compound structure information, based on a reactive-site identifying condition and the pinching point information identified, the reactive-site identifying condition being a condition under which a reactive-site coordinate range that is a range of the atomic coordinate information for the atoms forming the reactive site, a reactive-site angle range that is a range of an angle value between each of the atoms forming the reactive site and the pinching point, and a reactive-site distance range that is a range of a distance value between each of the atoms forming the reactive site and the pinching point are defined for each of the predetermined molecular species;
a molecular-species information identifying unit (102b) also being arranged to confirm the molecular species of drug-metabolizing enzyme based on the first, the second and the third identifying units; and
an outputting unit (102i) arranged to output to an output device the binding site, the pinching point, and the reactive site identified at the first identifying to the third identifying units, and the molecular species of the drug-metabolizing enzyme confirmed.

2. The apparatus according to claim 1, wherein
the first identifying unit (102b) includes
a first selecting unit (102j) arranged to select the binding site based on a predetermined criterion;
a third acquiring unit (102k) arranged to acquire the binding site information for the binding site selected by the binding-site selecting unit (102j), from the compound structure information; and
a fourth identifying unit (102m) arranged to identify the binding site information acquired by the third acquiring unit (102k), from the compound structure information, based on the binding-site identifying condition, and identifies the molecular species corresponding to identified binding-site information as the molecular species information.

3. The apparatus according to claim 2, wherein
the fourth identifying unit (102m) includes a fifth identifying unit (102n) arranged to identify the binding site information when the atomic coordinate information for the atom that forms the binding site corresponding to the binding site information acquired by the third acquiring unit (102k) satisfies the binding-site coordinate range defined by the binding-site identifying condition, and identifies the molecular species corresponding to the satisfied binding-site coordinate range as the molecular species information.

4. The apparatus according to any one of claims 1 to 3, wherein
the second acquiring unit (102c) includes
a first specifying unit (102p) arranged to specify an atom bonded to the atom forming the binding site corresponding to the binding site information identified by the first identifying unit (102b) and also specifies the atom bonded to the directly bonded atom, based on the bond information included in the compound structure information;
a first determining unit (102q) arranged to determine whether the atom specified by the first specifying unit (102p) is the atom forming the ring structure, based on the ring structure information included in the compound structure information;
a second specifying unit (102r) arranged to specify the atom specified and another atom forming the ring structure together with the atom, as the atoms forming the reactive site, when it is determined by the first determining unit (102q) that the atom specified by the first specifying unit (102p) is the atom forming the ring structure, and that specifies the directly bonded atom specified by the first specifying unit (102p), as the pinching point; and
a fourth acquiring unit (102s) arranged to acquire the pinching point information for the pinching point specified by the second specifying unit (102r) and the reactive site information for the reactive site specified, from the binding site information and the compound structure information.

5. The apparatus according to claim 4, wherein
the third identifying unit (102d) includes:
a second determining unit (102t) arranged to determine whether the atomic coordinate information for the atoms, which form the reactive site corresponding to the reactive site information acquired by the second acquiring unit (102c), satisfies the reactive-site coordinate range defined by the reactive-site identifying condition, in the molecular species corresponding to the molecular species information identified;
a third determining unit (102u) arranged to calculate the angle value between the atom forming the reactive site corresponding to the reactive site information and the pinching point corresponding to the pinching point information, and to determine whether the angle value calculated satisfies the reactive-site angle range defined by the reactive-site identifying condition, in the molecular species corresponding to the molecular species information identified;
a fourth determining unit (102v) arranged to calculate the distance value between the atom forming the reactive site corresponding to the reactive site information and the pinching point corresponding to the pinching point information, and to determine whether the distance value calculated satisfies the reactive-site distance range defined by the reactive-site identifying condition, in the molecular species corresponding to the molecular species information identified; and
a sixth identifying unit (102w) arranged to identify the reactive site information determined, as being satisfied, by the second determining unit (102t), the third determining unit (102u), and the fourth determining unit (102v), as the reactive site information that satisfies the reactive-site identifying condition.

6. The apparatus according to any one of claims 1 to 5, wherein
the second identifying unit (102e) includes a seventh identifying unit (102x) arranged to identify the pinching point information when the atomic coordinate information for the atom, which is the pinching point corresponding to the pinching point information acquired by the second acquiring unit (102c), satisfies the pinching-point coordinate range defined by the pinching-point identifying condition, in the molecular species corresponding to the molecular species information identified.

7. The apparatus according to any one of claims 1 to 6, further comprising:
a fifth acquiring unit (102f) arranged to acquire open-space site information that is information on the atom forming an open space site, from the binding site information, the reactive site information, the pinching point information, and the compound structure information, if the open space site exists a site other than the reactive site corresponding to the reactive site information identified by the third identifying unit (102d) and the binding site corresponding to the binding site information identified by the first identifying unit (102b) and which is bound to the pinching point corresponding to the pinching point information identified by the pinching-point-information identifying unit (102e); and
an eighth identifying unit (102g) arranged to identify the open-space site information acquired by the fifth acquiring unit (102f), from the molecular species information and the compound structure information, based on an open-space-site identifying condition for identifying the open space site, the open-space-site identifying condition being a condition under which an open-space-site coordinate range, which is a range of the atomic coordinate information for an atom forming an open space site is defined for each of the predetermined molecular species.

8. The apparatus according to claim 7, wherein
the eighth identifying unit (102g) includes a ninth identifying unit (102y) arranged to identify the open-space site information when the atomic coordinate information for the atom, which forms the open space site corresponding to the open-space site information acquired by the fifth acquiring unit (102f), satisfies the open-space-site coordinate range defined by the open-space-site identifying condition, in the molecular species corresponding to the molecular species information identified.

9. The apparatus according to any one of claims 1 to 8, further comprising:
a fifth determining unit (102h) arranged to determine whether the atom forming the reactive site corresponding to the reactive site information or a partial-structure atom group which is a group of part of the atoms in a molecular structure at the reactive site inhibits the metabolic reaction, when the reactive site information is identified by the third identifying unit (102d), based on an inhibition determining condition for determining whether the metabolic reaction is inhibited in the compound of which the reactive site information is identified, wherein
the outputting unit (102i) arranged to further output to the output device inhibited metabolic reaction in the compound of the reactive site identified.

10. A drug-metabolizing enzyme prediction method comprising:
first acquiring, from a compound-structure information database, using a processor, compound structure information including atomic coordinate information of each of a plurality of atoms forming a compound, bond information between the atoms, and ring structure information on a ring structure formed with the atoms;
first identifying, using a processor, binding site information on an atom forming a binding site in the compound corresponding to the compound structure information and molecular species information, from the compound structure information acquired at the first acquiring, based on a binding-site identifying condition for identifying the molecular species of a drug-metabolizing enzyme belonging to the CYP families 1-4 of cytochromes P450 to be bound and for identifying a site in the compound where the compound and the drug-metabolizing enzyme are bound to each other, the binding-site identifying condition being a condition under which a binding-site coordinate range, which is a range of atomic coordinate information for an atom forming a binding site, is defined for each of the predetermined molecular species;
second acquiring, using a processor, a pinching point and the reactive site that is the atom binding the binding site to a reactive site in which a metabolic reaction of the compound and the drug-metabolizing enzyme occurs, the pinching point and the reactive site being in the same compound as the binding site, from the binding site information identified at the first identifying and the compound structure information, and acquiring pinching point information on the atom of the specified pinching point and the reactive site information on the atom forming the reactive site bound to the pinching point;
second identifying, using a processor, the pinching point information acquired at the second acquiring, from the molecular species information and the compound structure information, based on a pinching-point identifying condition, the pinching-point identifying condition being a condition under which a pinching-point coordinate range, which is a range of the atomic coordinate information for an atom as the pinching point, is defined for each of the predetermined molecular species;
third identifying, using a processor, the reactive site information acquired at the second acquiring, from the molecular species information and the compound structure information, based on a reactive-site identifying condition and the pinching point information identified, the reactive-site identifying condition being a condition under which a reactive-site coordinate range that is a range of the atomic coordinate information for the atoms forming the reactive site, a reactive-site angle range that is a range of an angle value between each of the atoms forming the reactive site and the pinching point, and a reactive-site distance range that is a range of a distance value between each of the atoms forming the reactive site and the pinching point are defined for each of the predetermined molecular species;
confirming, using a processor, the molecular species of drug-metabolizing enzyme based on the first, the second and the third identifying; and
outputting to an output device the binding site, the pinching point, and the reactive site identified at the first identifying to the third identifying, and the molecular species of the drug-metabolizing enzyme confirmed.

11. The drug-metabolizing enzyme prediction method according to claim 10, wherein
the first identifying includes
first selecting, using a processor, the binding site based on a predetermined criterion;
third acquiring, using a processor, the binding site information for the binding site selected at the first selecting, from the compound structure information; and
fourth identifying, using a processor, the binding site information acquired at the third acquiring, from the compound structure information, based on the binding-site identifying condition, and the molecular species corresponding to the identified binding site information as the molecular species information.

12. The drug-metabolizing enzyme prediction method according to claim 11, wherein
the fourth identifying includes fifth identifying, using a processor, the binding site information when the atomic coordinate information for the atom that forms the binding site corresponding to the binding site information acquired at the third acquiring satisfies the binding-site coordinate range defined by the binding-site identifying condition, and identifying the molecular species corresponding to the satisfied binding-site coordinate range as the molecular species information.

13. The drug-metabolizing enzyme prediction method according to any one of claims 10 to 12, wherein
the second acquiring includes
first specifying, using a processor, an atom bonded to the atom forming the binding site corresponding to the binding site information identified at the first identifying and also specifying the atom bonded to the directly bonded atom, based on the bond information included in the compound structure information;
first determining, using a processor, whether the first specifying is the atom forming the ring structure, based on the ring structure information included in the compound structure information;
second specifying, using a processor, the atom specified and another atom forming the ring structure together with the atom, as the atoms forming the reactive site, when it is determined at the first determining that the atom specified at the first specifying is the atom forming the ring structure, and specifying the directly bonded atom specified at the atom specifying step, as the pinching point; and
fourth acquiring, using a processor, the pinching point information for the pinching point specified at the second specifying and the reactive site information for the reactive site specified, from the binding site information and the compound structure information.

14. The drug-metabolizing enzyme prediction method according to claim 13, wherein
the third identifying includes
second determining, using a processor, whether the atomic coordinate information for the atoms, which form the reactive site corresponding to the reactive site information acquired at the second acquiring, satisfies the reactive-site coordinate range defined by the reactive-site identifying condition, in the molecular species corresponding to the molecular species information identified;
third determining, using a processor, the angle value between the atom forming the reactive site corresponding to the reactive site information and the pinching point corresponding to the pinching point information in calculation, and whether the angle value calculated satisfies the reactive-site angle range defined by the reactive-site identifying condition, in the molecular species corresponding to the molecular species information identified;
fourth determining, using a processor, the distance value between the atom forming the reactive site corresponding to the reactive site information and the pinching point corresponding to the pinching point information in calculation, and whether the distance value calculated satisfies the reactive-site distance range defined by the reactive-site identifying condition, in the molecular species corresponding to the molecular species information identified; and
sixth identifying, using a processor, the reactive site information determined, as being satisfied, at the second determining, the third determining, and the fourth determining, as the reactive site information that satisfies the reactive-site identifying condition.

15. The drug-metabolizing enzyme prediction method according to any one of claims 10 to 14, wherein
the second identifying includes seventh identifying, using a processor, the pinching point information when the atomic coordinate information for the atom, which is the pinching point corresponding to the pinching point information acquired at the second acquiring, satisfies the pinching-point coordinate range defined by the pinching-point identifying condition, in the molecular species corresponding to the molecular species information identified.

16. The drug-metabolizing enzyme prediction method according to any one of claims 10 to 15, further comprising:
fifth acquiring, using a processor, information on the atom forming an open space site, from the binding site information, the reactive site information, the pinching point information, and the compound structure information, if there exists the open space site that is a site other than the reactive site corresponding to the reactive site information identified at the third identifying and the binding site corresponding to the binding site information identified at the first identifying and which is bound to the pinching point corresponding to the pinching point information identified at the second identifying; and
eighth identifying, using a processor, the open-space site information acquired at the fifth acquiring, from the molecular species information and the compound structure information, based on an open-space-site identifying condition for identifying the open space site, the open-space-site identifying condition being a condition under which an open-space-site coordinate range, which is a range of the atomic coordinate information for an atom forming an open space site is defined for each of the predetermined molecular species.

17. The drug-metabolizing enzyme prediction method according to claim 16, wherein
the eighth identifying includes ninth identifying, using a processor, the open-space site information when the atomic coordinate information for the atom, which forms the open space site corresponding to the open-space site information acquired at the fifth acquiring, satisfies the open-space-site coordinate range defined by the open-space-site identifying condition, in the molecular species corresponding to the molecular species information identified.

18. The drug-metabolizing enzyme prediction method according to any one of claims 10 to 17, further comprising:
fifth determining, using a processor, whether the atom forming the reactive site corresponding to the reactive site information or a partial-structure atom group which is a group of part of the atoms in a molecular structure at the reactive site inhibits the metabolic reaction, when the reactive site information is identified at the third identifying, based on an inhibition determining condition for determining whether the metabolic reaction is inhibited in the compound of which the reactive site information is identified, wherein
the outputting further outputs to the output device inhibited metabolic reaction in the compound of the reactive site identified.

19. A computer-readable recording medium that stores therein a drug-metabolizing enzyme prediction program, wherein
the drug-metabolizing enzyme prediction program causes a computer to execute:
first acquiring, from a compound-structure information database, compound structure information including atomic coordinate information of each of a plurality of atoms forming a compound, bond information between the atoms, and ring structure information that is information on a ring structure formed with the atoms;
first identifying binding site information that is information on an atom in the compound corresponding to the compound structure information and molecular species information, from the compound structure information acquired at the first acquiring, based on a binding-site identifying condition for identifying the molecular species of a drug-metabolizing enzyme belonging to the CYP families 1-4 of cytochromes P450 to be bound and for identifying a site in the compound where the compound and the drug-metabolizing enzyme are bound to each other, the binding-site identifying condition being a condition under which a binding-site coordinate range, which is a range of atomic coordinate information for an atom forming a binding site, is defined for each of the predetermined molecular species;
second acquiring a pinching point that is the atom binding the binding site to a reactive site in which a metabolic reaction occurs of the compound and the drug-metabolizing enzyme, the pinching point and the reactive site being in the same compound as the binding site, from the binding site information identified at the first identifying and the compound structure information, and acquiring pinching point information on the atom of the specified pinching point and the reactive site information on the atom forming the reactive site bound to the pinching point;
second identifying the pinching point acquired at the second acquiring, from the molecular species information and the compound structure information, based on a pinching-point identifying condition, the pinching-point identifying condition being a condition under which a pinching-point coordinate range, which is a range of the atomic coordinate information for an atom as the pinching point, is defined for each of the predetermined molecular species;
third identifying the reactive site information acquired at the second acquiring, from the molecular species information and the compound structure information, based on a reactive-site identifying condition and the pinching point information identified, the reactive-site identifying condition being a condition under which a reactive-site coordinate range that is a range of the atomic coordinate information for the atoms forming the reactive site, a reactive-site angle range that is a range of an angle value between each of the atoms forming the reactive site and the pinching point, and a reactive-site distance range that is a range of a distance value between each of the atoms forming the reactive site and the pinching point are defined for each of the predetermined molecular species;
confirming the molecular species of drug-metabolizing enzyme based on the first, the second and the third identifying; and
outputting to an output device the binding site, the pinching point, and the reactive site identified at the first identifying to the third identifying, and the molecular species of the drug-metabolizing enzyme confirmed.

20. The computer-readable recording medium according to claim 19, wherein the first identifying includes
first selecting the binding site based on a predetermined criterion; third acquiring the binding site information for the binding site selected at the first selecting, from the compound structure information; and
fourth identifying the binding site information acquired at the third acquiring, from the compound structure information, based on the binding-site identifying condition, and the molecular species corresponding to the identified binding site information as the molecular species information.

21. The computer-readable recording medium according to claim 20, wherein
the fourth identifying includes fifth identifying the binding site information when the atomic coordinate information for the atom that forms the binding site corresponding to the binding site information acquired at the third acquiring satisfies the binding-site coordinate range defined by the binding-site identifying condition, and identifying the molecular species corresponding to the satisfied binding-site coordinate range as the molecular species information.

22. The computer-readable recording medium according to any one of claims 19 to 21, wherein
the second acquiring includes
first specifying an atom bonded to the atom forming the binding site corresponding to the binding site information identified at the first identifying and also specifying the atom bonded to the directly bonded atom, based on the bond information included in the compound structure information;
first determining whether the atom specified at the atom specifying is the atom forming the ring structure, based on the ring structure information included in the compound structure information;
second specifying the atom specified and another atom forming the ring structure together with the atom, as the atoms forming the reactive site, when it is determined at the first determining that the atom specified at the first specifying is the atom forming the ring structure, and specifying the directly bonded atom specified at the first specifying, as the pinching point; and
fourth acquiring the pinching point information for the pinching point specified at the second specifying and the reactive site information for the reactive site specified, from the binding site information and the compound structure information.

23. The computer-readable recording medium according to claim 22, wherein the third identifying includes
second determining whether the atomic coordinate information for the atoms, which form the reactive site corresponding to the reactive site information acquired at the second acquiring, satisfies the reactive-site coordinate range defined by the reactive-site identifying condition, in the molecular species corresponding to the molecular species information identified;
third determining the angle value between the atom forming the reactive site corresponding to the reactive site information and the pinching point corresponding to the pinching point information in calculation, and whether the angle value calculated satisfies the reactive-site angle range defined by the reactive-site identifying condition, in the molecular species corresponding to the molecular species information identified;
fourth determining the distance value between the atom forming the reactive site corresponding to the reactive site information and the pinching point corresponding to the pinching point information in calculation, and whether the distance value calculated satisfies the reactive-site distance range defined by the reactive-site identifying condition, in the molecular species corresponding to the molecular species information identified; and
sixth identifying the reactive site information determined, as being satisfied, at the second determining, the third determining, and the fourth determining, as the reactive site information that satisfies the reactive-site identifying condition.

24. The computer-readable recording medium according to any one of claims 19 to 23, wherein
the second identifying includes seventh identifying the pinching point information when the atomic coordinate information for the atom, which is the pinching point corresponding to the pinching point information acquired at the second acquiring, satisfies the pinching-point coordinate range defined by the pinching-point identifying condition, in the molecular species corresponding to the molecular species information identified.

25. The computer-readable recording medium according to any one of claims 19 to 24, wherein
the drug-metabolizing enzyme prediction program further causes the computer to execute:
fifth acquiring information on the atom forming an open space site, from the binding site information, the reactive site information, the pinching point information, and the compound structure information, if there exists the open space site that is a site other than the reactive site corresponding to the reactive site information identified at the third identifying and the binding site corresponding to the binding site information identified at the first identifying and which is bound to the pinching point corresponding to the pinching point information identified at the second identifying; and
eighth identifying the open-space site information acquired at the fifth acquiring, from the molecular species information and the compound structure information, based on an open-space-site identifying condition for identifying the open space site, the open-space-site identifying condition being a condition under which an open-space-site coordinate range, which is a range of the atomic coordinate information for an atom forming an open space site is defined for each of the predetermined molecular species.

26. The computer-readable recording medium according to claim 25, wherein
the eighth identifying includes a ninth identifying the open-space site information when the atomic coordinate information for the atom, which forms the open space site corresponding to the open-space site information acquired at the fifth acquiring, satisfies the open-space-site coordinate range defined by the open-space-site identifying condition, in the molecular species corresponding to the molecular species information identified.

27. The computer-readable recording medium according to any one of claims 19 to 26, wherein
the drug-metabolizing enzyme prediction program further causes the computer to execute:
fifth determining whether the atom forming the reactive site corresponding to the reactive site information or a partial-structure atom group which is a group of part of the atoms in a molecular structure at the reactive site inhibits the metabolic reaction, when the reactive site information is identified at the third identifying, based on an inhibition determining condition for determining whether the metabolic reaction is inhibited in the compound of which the reactive site information is identified, wherein
the outputting further outputs to the output device inhibited metabolic reaction in the compound of the reactive site identified.

## Patentansprüche

1. Vorrichtung, umfassend:
eine erste Erfassungseinheit (102a), die ausgelegt ist, aus einer Verbindungsstruktur-Informationsdatenbank Verbindungsstruktur-Information zu erfassen, die Atomkoordinaten-Information jedes einer Mehrzahl von Atomen, die eine Verbindung bilden, Bindungsinformation zwischen den Atomen und Ringstruktur-Informationen über eine mit den Atomen gebildete Ringstruktur beinhaltet;
eine erste Identifikationseinheit (102b), die ausgelegt ist, Bindungsstelleninformation über ein Atom in der Verbindung entsprechend der Verbindungsstruktur-Information und Molekularspezies-Information aus der durch die erste Erfassungseinheit erfassten Verbindungsstruktur-Information zu identifizieren, basierend auf einer Bindungsstellen-Identifikationsbedingung zum Identifizieren der Molekularspezies eines Arzneimittel-metabolisierenden Enzyms, das zu den CYP-Familien 1-4 von Cytochromen P450 gehört, die zu binden sind, und zum Identifizieren einer Stelle in der Verbindung, wo die Verbindung und das Arzneimittel-metabolisierende Enzym aneinander gebunden sind, wobei die Bindungsstellen-Identifikations-Bedingung eine Bedingung ist, unter welcher ein Bindungsstellen-Koordinatenbereich, der ein Bereich von Atom-Koordinateninformation für ein eine Bindungsstelle bildendes Atom ist, für jede der vorbestimmten Molekularspezies definiert ist;
eine zweite Erfassungseinheit (102c) die ausgelegt ist, einen Klemmpunkt zu spezifizieren, welcher das Atom ist, das die Bindungsstelle an eine Reaktivstelle bindet, in welcher eine metabolische Reaktion der Verbindung und des Arzneimittel-metabolisierenden Enzyms auftritt, wobei der Klemmpunkt und die Reaktivstelle in derselben Verbindung wie die Bindungsstelle sind, aus der durch die erste Identifikationseinheit (102b) und der Verbindungsstruktur-Information identifizierten Bindungsstellen-Information, und Klemmpunkt-Information zu dem Atom des spezifizierten Klemmpunkts und die Reaktivstellen-Information über das die am Klemmpunkt gebundene Reaktivstelle bildende Atom zu erfassen;
eine zweite Identifikationseinheit (102e), die ausgelegt ist, die durch die zweite Erfassungseinheit (102) erfasste Klemmpunkt-Information aus der Molekularspezies-Information und der Verbindungsstruktur-Information zu identifizieren, basierend auf einer Klemmpunkt-Identifizierbedingung, wobei die Klemmpunkt-Identifizierbedingung eine Bedingung ist, unter welcher ein Klemmpunkt-Koordinatenbereich, der ein Bereich von Atom-Koordinateninformation für ein Atom als der Klemmpunkt ist, für jede der vorbestimmten Molekularspezies definiert ist;
eine dritte Identifikationseinheit (102d), die ausgelegt ist, die durch die zweite Erfassungseinheit (102c) erfasste Reaktivstellen-information aus der Molekularspezies-Information und der Strukturinformation zu identifizieren, basierend auf einer Reaktivstellen-Identifikationsbedingung und der identifizierten Klemmpunkt-Information, wobei die Reaktivstellen-Identifikationsbedingung eine Bedingung ist, unter welcher ein Reaktivstellen-Koordinatenbereich, der ein Bereich der Atom-Koordinateninformation für die die Reaktivstelle bildenden Atome ist, ein Reaktivstellen-Winkelbereich, der ein Bereich eines Winkelwerts zwischen jedem der die Reaktivstelle bildenden Atome und dem Klemmpunkt ist, und ein Reaktivstellen-Distanzbereich, der ein Bereich eines Distanzwerts zwischen jedem der die Reaktivstelle bildenden Atome und dem Klemmpunkt ist, für jede der vorbestimmten Molekularspezies definiert sind;
eine Molekularspezies-Informationsidentifikationseinheit (102b), die auch ausgelegt ist, die Molekularspezies eines Arzneimittel-metabolisierenden Enzyms zu bestätigen, basierend auf den ersten, den zweiten und den dritten Identifikationseinheiten; und
eine Ausgabeeinheit (102i), die ausgelegt ist, an eine Ausgabevorrichtung die Bindungsstelle, den Klemmpunkt und die Reaktivstelle, welche durch die ersten bis dritten Identifikationseinheiten identifiziert sind, und die Molekularspezies des Arzneimittel-metabolisierenden Enzyms bestätigt auszugeben.

2. Vorrichtung gemäß Anspruch 1, wobei die erste Identifikationseinheit (102b) beinhaltet eine erste Auswahleinheit (102j), die ausgelegt ist, die Bindungsstelle basierend auf einem vorbestimmten Kriterium auszuwählen;
eine dritte Erfassungseinheit (102k), die ausgelegt ist, die Bindungsstellen-Information für die Bindungsstelle, die durch die Bindungsstellen-Auswahleinheit (102j) ausgewählt ist, aus der Verbindungsstruktur-Information zu erfassen; und
eine vierte Identifikationseinheit (102m), die ausgelegt ist, die durch die dritte Erfassungseinheit (102k) erfasste Bindungsstellen-Information aus der Verbindungsstruktur-Information zu identifizieren, basierend auf der Bindungsstellen-Identifikationsbedingung, und die Molekularspezies entsprechend der identifizierten Bindungsstellen-Information als die Molekularspezies-Information identifiziert.

3. Vorrichtung gemäß Anspruch 2, wobei
die vierte Identifikationseinheit (102m) eine fünfte Identifikationseinheit (102n) beinhaltet, die ausgelegt ist, die Bindungsstellen-Information zu identifizieren,
wenn die Atom-Koordinateninformation für das Atom, das die Bindungsstelle bildet, entsprechend der durch die dritte Erfassungseinheit (102k) erfassten Bindungsstellen-Information den Bindungsstelle-Koordinatenbereich erfüllt, der durch die Bindungsstellen-Identifikationsbedingung definiert ist, und die dem erfüllten Bindungsstellen-Koordinatenbereich entsprechenden Molekularspezies als die Molekularspezies-Information identifiziert.

4. Vorrichtung gemäß einem von Ansprüchen 1 bis 3, wobei
die zweite Erfassungseinheit (102c) beinhaltet
eine erste Spezifikationseinheit (102p), die ausgelegt ist, ein an dem, die Bindungsstelle entsprechend der Bindungsstellen-Information bildendes Atom gebundenes Atom, das identifiziert durch die erste Identifikationseinheit (102b) ist, zu spezifizieren und auch das an das direkt gebundene Atom gebundene Atom zu spezifizieren, basierend auf der in der Verbindungsstruktur-Information enthaltenen Bindungsinformation;
eine erste Bestimmungseinheit (102q), die ausgelegt ist, zu bestimmen, ob das durch die erste Spezifikationseinheit (102p) spezifizierte Atom das die Ringstruktur bildende Atom ist, basierend auf der in der Verbindungsstruktur-Information enthaltenen Ringstruktur-Information;
eine zweite Spezifikationseinheit (102r), die ausgelegt ist, das spezifizierte Atom, und ein anderes Atom, das zusammen mit dem Atom die Ringstruktur bildet, als die, die Reaktivstelle bildenden Atome zu spezifizieren, wenn durch die erste Bestimmungseinheit (102q) festgestellt wird, dass das durch die erste Spezifikationseinheit (102p) spezifizierte Atom das die Ringstruktur bildende Atom ist, und die das durch die erste Spezifikationseinheit (102p) spezifizierte direkt gebundene Atom als den Klemmpunkt spezifiziert; und
eine vierte Erfassungseinheit (102s), die ausgelegt ist, die Klemmpunkt-Information für den durch die zweite Spezifikationseinheit (102r) spezifizierten Klemmpunkt und die Reaktivstellen-Information für die spezifizierte Reaktivstelle aus der Bindungsstelle-Information und der Verbindungsstruktur-Information zu erfassen.

5. Vorrichtung gemäß Anspruch 4, wobei
die dritte Identifikationseinheit (102d) beinhaltet
eine zweite Bestimmungseinheit (102t), die ausgelegt ist, zu bestimmen, ob die Atomkoordinateninformation für die Atome, welche die der durch die zweite Erfassungseinheit (102c) erfassten Reaktivstellen-Information entsprechende Reaktivstelle bilden, den durch die Reaktivstellen-Identifikationsbedingung definierten Reaktivstellen-Koordinatenbereich in der, der identifizierten Molekularspezies-Information entsprechenden Molekularspezies erfüllt;
eine dritte Bestimmungseinheit (102u), die ausgelegt ist, den Winkelwert zwischen dem die, die Reaktivstellen-Information entsprechenden Reaktivstelle bildenden Atom und dem der Klemmpunkt-Information entsprechenden Klemmpunkt zu berechnen, und zu bestimmen, ob der berechnete Winkelwert den Reaktivstellen-Winkelwert erfüllt, der durch die Reaktivstellen-Identifikationsbedingung definiert ist, in der, der identifizierten Molekularspezies-Information entsprechenden Molekularspezies;
eine vierte Bestimmungseinheit (102v), die ausgelegt ist, den Distanzwert zwischen dem, der der Reaktivstellen-Information entsprechenden Reaktivstelle bildenden Atom und dem, der Klemmpunkt-Information entsprechenden Klemmpunkt zu berechnen, und zu bestimmen, ob der berechnete Distanzwert den durch die Reaktivstellen-Identifikationsbedingung definierten Reaktivstellen-Distanzbereich erfüllt, in der, der identifizierten Molekularspezies-Information entsprechenden Molekularspezies; und
eine sechste Identifikationseinheit (102w), die ausgelegt ist, die Reaktivstellen-Information, die als durch die zweite Bestimmungseinheit (102t), die dritte Bestimmungseinheit (102u) und die vierte Bestimmungseinheit (102v) erfüllt bestimmt ist, als die Reaktivstellen-Information zu identifizieren, die die Reaktivstellen-Identifikationsbedingung erfüllt.

6. Vorrichtung gemäß einem der Ansprüche 1 bis 5, wobei
die zweite Identifikationseinheit (102e) eine siebte Identifikationseinheit (102x) enthält, die ausgelegt ist, die Klemmpunkt-Information zu identifizieren, wenn die Atomkoordinaten-Information für das Atom, welches der, der durch die zweite Erfassungseinheit (102c) erfassten Klemmpunkt-Information entsprechende Klemmpunkt ist, den durch den Klemmpunkt-Identifikationsbedingung definierten Klemmpunkt-Koordinatenbereich erfüllt, in der, der identifizierten Molekularspezies-Information entsprechenden Molekularspezies.

7. Vorrichtung gemäß einem der Ansprüche 1 bis 6, weiter umfassend:
eine fünfte Erfassungseinheit (102f), die ausgelegt ist, Freiraum-Stelleninformation zu erfassen, die Information über das eine Freiraumstelle bildende Atom ist, aus der Bindungsstellen-Information, der Reaktivstellen-Information, der Klemmpunkt-Information und der Verbindungsstruktur-Information, falls die Freiraumstelle existiert, einem anderen Ort als der Reaktivstelle entsprechend der durch die dritte Identifikationseinheit (102d) identifizierten Reaktivstellen-Information und der, der durch die erste Identifikationseinheit (102b) identifizierten Bindungsstelle-Information entsprechenden Bindungsstelle, und der an den Klemmpunkt gebunden ist, welcher der durch die Klemmpunkt-Informationsidentifikationseinheit (102e) identifizierten Klemmpunkt-Information entspricht; und
eine achte Identifikationseinheit (102g), die ausgelegt ist, die durch die fünfte Erfassungseinheit (102f) erfasste Freiraumstelleninformation aus der Molekularspezies-Information und der Verbindungsstruktur-Information zu identifizieren, basierend auf einer Freiraumstellen-Identifikationsbedingung zum Identifizieren der Freiraumstelle, wobei die Freiraumstellen-Identifikationsbedingung eine Bedingung ist, unter welcher ein Freiraumstellen-Koordinatenbereich, der ein Bereich der Atom-Koordinateninformation für ein, eine Freiraumstelle bildendes Atom ist, für jede der vorbestimmten Molekularspezies definiert ist.

8. Vorrichtung gemäß Anspruch 7, wobei
die achte Identifikationseinheit (102g) eine neunte Identifikationseinheit (102y) beinhaltet, die ausgelegt ist, die Freiraumstelleninformation zu identifizieren,
wenn die Atomkoordinaten-Information für das Atom, welches die, der durch die fünfte Erfassungseinheit (102f) erfassten Freiraumstelleninformation entsprechende Freiraumstelle bildet, den durch Freiraumstellen-Identifikationsbedingung definierten Freiraumstellen-Koordinatenbereich erfüllt, in der, der identifizierten Molekularspezies-Information entsprechenden Molekularspezies.

9. Vorrichtung gemäß einem der Ansprüche 1 bis 8, weiter umfassend:
eine fünfte Bestimmungseinheit (102h), die ausgelegt ist, zu bestimmen, ob das die, der Reaktivstellen-Information entsprechende Reaktivstelle bildende Atom oder eine Partialstruktur-Atomgruppe, die eine Gruppe eines Teils des Atoms in einer Molekularstruktur an dem Reaktivstellen ist, die metabolische Reaktion hemmt, wenn die Reaktivstellen-Information durch die dritte Identifikationseinheit (102d) identifiziert ist, basierend auf einer Hemm-Bestimmungsbedingung zum Bestimmen, ob die metabolische Reaktion in der Verbindung, deren Reaktivstellen-Information identifiziert ist, gehemmt ist, wobei die Ausgabeeinheit (102i) ausgelegt ist, an die Ausgabevorrichtung weiterhin gehemmte metabolische Reaktion in der Verbindung der identifizierten Reaktivstelle auszugeben.

10. Arzneimittel-metabolisierendes Enzym-Vorhersageverfahren, umfassend
ein erstes Erfassen, aus einer Verbindungsstruktur-Informationsdatenbank, unter Verwendung eines Prozessors, von Verbindungsstruktur-Information, die Atomkoordinaten-Information jedes einer Mehrzahl von Atomen, die eine Verbindung bilden, Bindungsinformation zwischen den Atomen und Ringstruktur-Informationen über eine mit den Atomen gebildete Ringstruktur beinhaltet;
ein erstes Identifizieren, unter Verwendung eines Prozessors, von Bindungsstelleninformation über ein Atom in der Verbindung entsprechend der Verbindungsstruktur-Information und Molekularspezies-Information aus der durch die erste Erfassungseinheit erfassten Verbindungsstruktur-Information, basierend auf einer Bindungsstellen-Identifikationsbedingung zum Identifizieren der Molekularspezies eines Arzneimittel-metabolisierenden Enzyms, das zu den CYP-Familien 1-4 von Cytochromen P450 gehört, die zu binden sind, und zum Identifizieren einer Stelle in der Verbindung, wo die Verbindung und das Arzneimittel-metabolisierende Enzym aneinander gebunden sind, wobei die Bindungsstellen-Identifikations-Bedingung eine Bedingung ist, unter welcher ein Bindungsstellen-Koordinatenbereich, der ein Bereich von Atom-Koordinateninformation für ein eine Bindungsstelle bildendes Atom ist, für jede der vorbestimmten Molekularspezies definiert ist;
ein zweites Erfassen, unter Verwendung eines Prozessors, eines Klemmpunkts und der Reaktivstelle, welcher das Atom ist, das die Bindungsstelle an eine Reaktivstelle bindet, in welcher eine metabolische Reaktion der Verbindung und des Arzneimittel-metabolisierenden Enzyms auftritt, wobei der Klemmpunkt und die Reaktivstelle in derselben Verbindung wie die Bindungsstelle sind, aus der beim ersten Identifizieren und der
Verbindungsstruktur-Information identifizierten Bindungsstellen-Information, und Erfassen von Klemmpunkt-Information zu dem Atom des spezifizierten Klemmpunkts und die Reaktivstellen-Information über das die am Klemmpunkt gebundene Reaktivstelle bildende Atom;
ein zweites Identifizieren, unter Verwendung eines Prozessors, der beim zweiten Erfassen erfassten Klemmpunkt-Information aus der Molekularspezies-Information und der Verbindungsstruktur-Information zu, basierend auf einer Klemmpunkt-Identifizierbedingung, wobei die Klemmpunkt-Identifizierbedingung eine Bedingung ist, unter welcher ein Klemmpunkt-Koordinatenbereich, der ein Bereich von Atom-Koordinateninformation für ein Atom als der Klemmpunkt ist, für jede der vorbestimmten Molekularspezies definiert ist;
ein drittes Identifizieren, unter Verwendung eines Prozessors, der beim zweiten Erfassen erfassten Reaktivstellen-information aus der Molekularspezies-Information und der Strukturinformation zu, basierend auf einer Reaktivstellen-Identifikationsbedingung und der identifizierten Klemmpunkt-Information, wobei die Reaktivstellen-Identifikationsbedingung eine Bedingung ist, unter welcher ein Reaktivstellen-Koordinatenbereich, der ein Bereich der Atom-Koordinateninformation für die die Reaktivstelle bildenden Atome ist, ein Reaktivstellen-Winkelbereich, der ein Bereich eines Winkelwerts zwischen jedem der die Reaktivstelle bildenden Atome und dem Klemmpunkt ist, und ein Reaktivstellen-Distanzbereich, der ein Bereich eines Distanzwerts zwischen jedem der die Reaktivstelle bildenden Atome und dem Klemmpunkt ist, für jede der vorbestimmten Molekularspezies definiert sind;
Bestätigen, unter Verwendung eines Prozessors, der Molekularspezies eines Arzneimittel-metabolisierenden Enzyms zu, basierend auf den ersten, den zweiten und den dritten Identifizierungen; und
Ausgeben, an eine Ausgabevorrichtung, der Bindungsstelle, des Klemmpunkts und der Reaktivstelle, welche durch die ersten bis dritten Identifizierungen identifiziert sind, und der bestätigten Molekularspezies des Arzneimittel-metabolisierenden Enzyms.

11. Arzneimittel-metabolisierendes Enzym-Vorhersageverfahren gemäß Anspruch 10, wobei
das erste Identifizieren beinhaltet
ein erstes Auswählen, unter Verwendung eines Prozessors, der Bindungsstelle basierend auf einem vorbestimmten Kriterium;
ein drittes Erfassen, unter Verwendung eines Prozessors, der Bindungsstellen-Information für die Bindungsstelle, die durch das erste Auswählen ausgewählt ist, aus der Verbindungsstruktur-Information; und
ein viertes Identifizieren, unter Verwendung eines Prozessors, der beim dritten Erfassen erfassten Bindungsstellen-Information aus der Verbindungsstruktur-Information, basierend auf der Bindungsstellen-Identifikationsbedingung, und der Molekularspezies entsprechend der identifizierten Bindungsstellen-Information, als die Molekularspezies-Information.

12. Arzneimittel-metabolisierendes Enzym-Vorhersageverfahren gemäß Anspruch 11, wobei
das vierte Identifizieren beinhaltet: ein fünftes Identifizieren, unter Verwendung eines Prozessors, der Bindungsstellen-Information, wenn die Atom-Koordinateninformation für das Atom, das die Bindungsstelle bildet, entsprechend der beim dritten Erfassen erfassten Bindungsstellen-Information den Bindungsstelle-Koordinatenbereich erfüllt, der durch die Bindungsstellen-Identifikationsbedingung definiert ist, und Identifizieren der dem erfüllten Bindungsstellen-Koordinatenbereich entsprechenden Molekularspezies als die Molekularspezies-Information.

13. Arzneimittel-metabolisierendes Enzym-Vorhersageverfahren gemäß einem von Ansprüchen 10 bis 12, wobei
das zweite Erfassen beinhaltet
ein erstes Spezifizieren, unter Verwendung eines Prozessors, eines an dem, die Bindungsstelle entsprechend der Bindungsstellen-Information bildendes Atom gebundenen Atoms, das beim ersten Identifizieren identifiziert ist, und auch Spezifizieren des an das direkt gebundene Atom gebundenem Atom, basierend auf der in der Verbindungsstruktur-Information enthaltenen Bindungsinformation;
ein erstes Bestimmen, unter Verwendung eines Prozessors, ob das erste Spezifizieren das die Ringstruktur bildende Atom ist, basierend auf der in der Verbindungsstruktur-Information enthaltenen Ringstruktur-Information;
ein zweites Spezifizieren, unter Verwendung eines Prozessors, des spezifizierten Atoms, und eines anderen Atoms, das zusammen mit dem Atom die Ringstruktur bildet, als die, die Reaktivstelle bildenden Atome, wenn beim erste Bestimmen festgestellt wird, dass das beim ersten Spezifizieren spezifizierte Atom das die Ringstruktur bildende Atom ist, und Spezifizieren des im Atom-Spezifikationsschritt spezifizierten, direkt gebundenen Atoms als den Klemmpunkt; und
ein viertes Erfassen, unter Verwendung eines Prozessors, der Klemmpunkt-Information für den beim zweiten Spezifizieren spezifizierten Klemmpunkt und der Reaktivstellen-Information für die spezifizierte Reaktivstelle aus der Bindungsstelle-Information und der Verbindungsstruktur-Information.

14. Arzneimittel-metabolisierendes Enzym-Vorhersageverfahren gemäß Anspruch 13, wobei
das dritte Identifizieren beinhaltet
ein zweites Bestimmen, unter Verwendung eines Prozessors, ob die Atomkoordinateninformation für die Atome, welche die der beim zweiten Erfassen erfassten Reaktivstellen-Information entsprechende Reaktivstelle bilden, den durch die Reaktivstellen-Identifikationsbedingung definierten Reaktivstellen-Koordinatenbereich in der, der identifizierten Molekularspezies-Information entsprechenden Molekularspezies erfüllt;
ein drittes Bestimmen, unter Verwendung eines Prozessors, des Winkelwerts zwischen dem die, der Reaktivstellen-Information entsprechenden Reaktivstelle bildenden Atom und dem der Klemmpunkt-Information entsprechenden Klemmpunkt in Rechnung, und, ob der berechnete Winkelwert den Reaktivstellen-Winkelwert erfüllt, der durch die Reaktivstellen-Identifikationsbedingung definiert ist, in der, der identifizierten Molekularspezies-Information entsprechenden Molekularspezies;
ein viertes Bestimmen, unter Verwendung eines Prozessors, des Distanzwerts zwischen dem, die der Reaktivstellen-Information entsprechenden Reaktivstelle bildenden Atom und dem, der Klemmpunkt-Information entsprechenden Klemmpunkt in Rechnung, und ob der berechnete Distanzwert den durch die Reaktivstellen-Identifikationsbedingung definierten Reaktivstellen-Distanzbereich erfüllt, in der, der identifizierten Molekularspezies-Information entsprechenden Molekularspezies; und
ein sechstes Identifizieren, unter Verwendung eines Prozessors, der Reaktivstellen-Information, die als beim zweiten Bestimmen, beim dritten Bestimmen und beim vierten Bestimmen erfüllt bestimmt ist, als die Reaktivstellen-Information, die die Reaktivstellen-Identifikationsbedingung erfüllt.

15. Arzneimittel-metabolisierendes Enzym-Vorhersageverfahren gemäß einem der Ansprüche 10 bis 14, wobei
das zweite Identifizieren ein siebtes Identifizieren, unter Verwendung eines Prozessors, der Klemmpunkt-Information enthält, wenn die Atomkoordinaten-Information für das Atom, welches der, der beim zweiten Erfassen erfassten Klemmpunkt-Information entsprechende Klemmpunkt ist, den durch den Klemmpunkt-Identifikationsbedingung definierten Klemmpunkt-Koordinatenbereich erfüllt, in der, der identifizierten Molekularspezies-Information entsprechenden Molekularspezies.

16. Arzneimittel-metabolisierendes Enzym-Vorhersageverfahren gemäß einem der Ansprüche 10 bis 15, weiter umfassend:
ein fünftes Erfassen, unter Verwendung eines Prozessors, von Information, die Information über das eine Freiraumstelle bildende Atom ist, aus der Bindungsstellen-Information, der Reaktivstellen-Information, der Klemmpunkt-Information und der Verbindungsstruktur-Information, falls die Freiraumstelle existiert, die eine andere Stelle als die Reaktivstelle entsprechend der beim dritten Identifizieren identifizierten Reaktivstellen-Information und die, der beim ersten Identifizieren identifizierten Bindungsstelle-Information entsprechenden Bindungsstelle, und der an den Klemmpunkt gebunden ist, welcher der beim zweiten Identifizieren identifizierten Klemmpunkt-Information entspricht; und
ein achtes Identifizieren, unter Verwendung eines Prozessors, der beim fünften Erfassen erfassten Freiraumstelleninformation aus der Molekularspezies-Information und der Verbindungsstruktur-Information, basierend auf einer Freiraumstellen-Identifikationsbedingung zum Identifizieren der Freiraumstelle, wobei die Freiraumstellen-Identifikationsbedingung eine Bedingung ist, unter welcher ein Freiraumstellen-Koordinatenbereich, der ein Bereich der Atom-Koordinateninformation für ein, eine Freiraumstelle bildendes Atom ist, für jede der vorbestimmten Molekularspezies definiert ist.

17. Arzneimittel-metabolisierendes Enzym-Vorhersageverfahren gemäß Anspruch 16, wobei
das achte Identifizieren beinhaltet: ein neuntes Identifizieren, unter Verwendung eines Prozessors, der Freiraumstelleninformation, wenn die Atomkoordinaten-Information für das Atom, welches die, der beim fünfte Erfassen erfassten Freiraumstelleninformation entsprechende Freiraumstelle bildet, den durch Freiraumstellen-Identifikationsbedingung definierten Freiraumstellen-Koordinatenbereich erfüllt, in der, der identifizierten Molekularspezies-Information entsprechenden Molekularspezies.

18. Arzneimittel-metabolisierendes Enzym-Vorhersageverfahren gemäß einem der Ansprüche 10 bis 17, weiter umfassend:
ein fünftes Bestimmen, unter Verwendung eines Prozessors,ob das die, der Reaktivstellen-Information entsprechende Reaktivstelle bildende Atom oder eine Partialstruktur-Atomgruppe, die eine Gruppe eines Teils der Atome in einer Molekularstruktur an der Reaktivstelle ist, die metabolische Reaktion hemmt, wenn die Reaktivstellen-Information beim dritten Identifizieren identifiziert ist, basierend auf einer Hemm-Bestimmungsbedingung zum Bestimmen, ob die metabolische Reaktion in der Verbindung, deren Reaktivstellen-Information identifiziert ist, gehemmt ist, wobei
das Ausgeben weiterhin an die Ausgabevorrichtung gehemmte metabolische Reaktion in der Verbindung der identifizierten Reaktivstelle ausgibt.

19. Computerlesbares Aufzeichnungsmedium, das darauf ein Arzneimittel-Metabolisierungsenzym-Vorhersageprogramm speichert, wobei
das Arzneimittel-metabolisierende Enzym-Vorhersageprogramm einen Computer veranlasst, auszuführen:
ein erstes Erfassen, aus einer Verbindungsstruktur-Informationsdatenbank, von Verbindungsstruktur-Information, die Atomkoordinaten-Information jedes einer Mehrzahl von Atomen, die eine Verbindung bilden, Bindungsinformation zwischen den Atomen und Ringstruktur-Informationen über eine mit den Atomen gebildete Ringstruktur beinhaltet;
ein erstes Identifizieren von Bindungsstelleninformation über ein Atom in der Verbindung entsprechend der Verbindungsstruktur-Information und Molekularspezies-Information aus der durch die erste Erfassungseinheit erfassten Verbindungsstruktur-Information, basierend auf einer Bindungsstellen-Identifikationsbedingung zum Identifizieren der Molekularspezies eines Arzneimittel-metabolisierenden Enzyms, das zu den CYP-Familien 1-4 von Cytochromen P450 gehört, die zu binden sind, und zum Identifizieren einer Stelle in der Verbindung, wo die Verbindung und das Arzneimittel-metabolisierende Enzym aneinander gebunden sind, wobei die Bindungsstellen-Identifikations-Bedingung eine Bedingung ist, unter welcher ein Bindungsstellen-Koordinatenbereich, der ein Bereich von Atom-Koordinateninformation für ein eine Bindungsstelle bildendes Atom ist, für jede der vorbestimmten Molekularspezies definiert ist;
ein zweites Erfassen eines Klemmpunkts und der Reaktivstelle, welcher das Atom ist, das die Bindungsstelle an eine Reaktivstelle bindet, in welcher eine metabolische Reaktion der Verbindung und des Arzneimittel-metabolisierenden Enzyms auftritt, wobei der Klemmpunkt und die Reaktivstelle in derselben Verbindung wie die Bindungsstelle sind, aus der beim ersten Identifizieren und der Verbindungsstruktur-Information identifizierten Bindungsstellen-Information, und Erfassen von Klemmpunkt-Information zu dem Atom des spezifizierten Klemmpunkts und die Reaktivstellen-Information über das die am Klemmpunkt gebundene Reaktivstelle bildende Atom;
ein zweites Identifizieren der beim zweiten Erfassen erfassten Klemmpunkt-Information aus der Molekularspezies-Information und der Verbindungsstruktur-Information zu, basierend auf einer Klemmpunkt-Identifizierbedingung, wobei die Klemmpunkt-Identifizierbedingung eine Bedingung ist, unter welcher ein Klemmpunkt-Koordinatenbereich, der ein Bereich von Atom-Koordinateninformation für ein Atom als der Klemmpunkt ist, für jede der vorbestimmten Molekularspezies definiert ist;
ein drittes Identifizieren der beim zweiten Erfassen erfassten Reaktivstellen-information aus der Molekularspezies-Information und der Strukturinformation zu, basierend auf einer Reaktivstellen-Identifikationsbedingung und der identifizierten Klemmpunkt-Information, wobei die Reaktivstellen-Identifikationsbedingung eine Bedingung ist, unter welcher ein Reaktivstellen-Koordinatenbereich, der ein Bereich der Atom-Koordinateninformation für die die Reaktivstelle bildenden Atome ist, ein Reaktivstellen-Winkelbereich, der ein Bereich eines Winkelwerts zwischen jedem der die Reaktivstelle bildenden Atome und dem Klemmpunkt ist, und ein Reaktivstellen-Distanzbereich, der ein Bereich eines Distanzwerts zwischen jedem der die Reaktivstelle bildenden Atome und dem Klemmpunkt ist, für jede der vorbestimmten Molekularspezies definiert sind;
Bestätigen der Molekularspezies eines Arzneimittel-metabolisierenden Enzyms zu, basierend auf den ersten, den zweiten und den dritten Identifizierungen; und Ausgeben, an eine Ausgabevorrichtung, der Bindungsstelle, des Klemmpunkts und der Reaktivstelle,
welche durch die ersten bis dritten Identifizierungen identifiziert sind, und der bestätigten Molekularspezies des Arzneimittel-metabolisierenden Enzyms.

20. Computerlesbares Aufzeichnungsmedium gemäß Anspruch 19, wobei
das erste Identifizieren beinhaltet
ein erstes Auswählen der Bindungsstelle basierend auf einem vorbestimmten Kriterium;
ein drittes Erfassen der Bindungsstellen-Information für die Bindungsstelle, die durch das erste Auswählen ausgewählt ist, aus der Verbindungsstruktur-Information; und
ein viertes Identifizieren der beim dritten Erfassen erfassten Bindungsstellen-Information aus der Verbindungsstruktur-Information, basierend auf der Bindungsstellen-Identifikationsbedingung, und der Molekularspezies entsprechend der identifizierten Bindungsstellen-Information, als die Molekularspezies-Information.

21. Computerlesbares Aufzeichnungsmedium gemäß Anspruch 20, wobei
das vierte Identifizieren beinhaltet: ein fünftes Identifizieren der Bindungsstellen-Information, wenn die Atom-Koordinateninformation für das Atom, das die Bindungsstelle bildet, entsprechend der beim dritten Erfassen erfassten Bindungsstellen-Information den Bindungsstelle-Koordinatenbereich erfüllt, der durch die Bindungsstellen-Identifikationsbedingung definiert ist, und Identifizieren der dem erfüllten Bindungsstellen-Koordinatenbereich entsprechenden Molekularspezies als die Molekularspezies-Information.

22. Computerlesbares Aufzeichnungsmedium gemäß einem von Ansprüchen 19 bis 21, wobei
das zweite Erfassen beinhaltet
ein erstes Spezifizieren eines an dem, die Bindungsstelle entsprechend der Bindungsstellen-Information bildendes Atom gebundenen Atoms, das beim ersten Identifizieren identifiziert ist, und auch Spezifizieren des an das direkt gebundene Atom gebundenem Atom, basierend auf der in der Verbindungsstruktur-Information enthaltenen Bindungsinformation;
ein erstes Bestimmen, ob das erste Spezifizieren das die Ringstruktur bildende Atom ist, basierend auf der in der Verbindungsstruktur-Information enthaltenen Ringstruktur-Information;
ein zweites Spezifizieren des spezifizierten Atoms, und
eines anderen Atoms, das zusammen mit dem Atom die Ringstruktur bildet, als die, die Reaktivstelle bildenden Atome, wenn beim erste Bestimmen festgestellt wird, dass das beim ersten Spezifizieren spezifizierte Atom das die Ringstruktur bildende Atom ist, und Spezifizieren des im Atom-Spezifikationsschritt spezifizierten, direkt gebundenen Atoms als den Klemmpunkt; und
ein viertes Erfassen der Klemmpunkt-Information für den beim zweiten Spezifizieren spezifizierten Klemmpunkt und der Reaktivstellen-Information für die spezifizierte Reaktivstelle aus der Bindungsstelle-Information und der Verbindungsstruktur-Information.

23. Computerlesbares Aufzeichnungsmedium gemäß Anspruch 22, wobei
das dritte Identifizieren beinhaltet
ein zweites Bestimmen, ob die Atomkoordinateninformation für die Atome, welche die der beim zweiten Erfassen erfassten Reaktivstellen-Information entsprechende Reaktivstelle bilden, den durch die Reaktivstellen-Identifikationsbedingung definierten Reaktivstellen-Koordinatenbereich in der, der identifizierten Molekularspezies-Information entsprechenden Molekularspezies erfüllt;
ein drittes Bestimmen des Winkelwerts zwischen dem die, der Reaktivstellen-Information entsprechenden Reaktivstelle bildenden Atom und dem der Klemmpunkt-Information entsprechenden Klemmpunkt in Rechnung, und, ob der berechnete Winkelwert den Reaktivstellen-Winkelwert erfüllt, der durch die Reaktivstellen-Identifikationsbedingung definiert ist, in der, der identifizierten Molekularspezies-Information entsprechenden Molekularspezies;
ein viertes Bestimmen des Distanzwerts zwischen dem, die der Reaktivstellen-Information entsprechenden Reaktivstelle bildenden Atom und dem, der Klemmpunkt-Information entsprechenden Klemmpunkt in Rechnung, und ob der berechnete Distanzwert den durch die Reaktivstellen-Identifikationsbedingung definierten Reaktivstellen-Distanzbereich erfüllt, in der, der identifizierten Molekularspezies-Information entsprechenden Molekularspezies; und
ein sechstes Identifizieren der Reaktivstellen-Information, die als beim zweiten Bestimmen, beim dritten Bestimmen und beim vierten Bestimmen erfüllt bestimmt ist, als die Reaktivstellen-Information, die die Reaktivstellen-Identifikationsbedingung erfüllt.

24. Computerlesbares Aufzeichnungsmedium gemäß einem der Ansprüche 19 bis 23, wobei
das zweite Identifizieren ein siebtes Identifizieren der Klemmpunkt-Information enthält, wenn die Atomkoordinaten-Information für das Atom, welches der, der beim zweiten Erfassen erfassten Klemmpunkt-Information entsprechende Klemmpunkt ist, den durch den Klemmpunkt-Identifikationsbedingung definierten Klemmpunkt-Koordinatenbereich erfüllt, in der, der identifizierten Molekularspezies-Information entsprechenden Molekularspezies.

25. Computerlesbares Aufzeichnungsmedium gemäß einem der Ansprüche 19 bis 24, wobei
das Arzneimittel-metabolisierendes Enzym-Vorhersageprogramm weiter einen Computer veranlasst, auszuführen:
ein fünftes Erfassen von Information, die Information über das eine Freiraumstelle bildende Atom ist, aus der Bindungsstellen-Information, der Reaktivstellen-Information, der Klemmpunkt-Information und der Verbindungsstruktur-Information, falls die Freiraumstelle existiert, die eine andere Stelle als die Reaktivstelle entsprechend der beim dritten Identifizieren identifizierten Reaktivstellen-Information und die, der beim ersten Identifizieren identifizierten Bindungsstelle-Information entsprechenden Bindungsstelle, und der an den Klemmpunkt gebunden ist, welcher der beim zweiten Identifizieren identifizierten Klemmpunkt-Information entspricht; und
ein achtes Identifizieren der beim fünften Erfassen erfassten Freiraumstelleninformation aus der Molekularspezies-Information und der
Verbindungsstruktur-Information, basierend auf einer Freiraumstellen-Identifikationsbedingung zum Identifizieren der Freiraumstelle, wobei die Freiraumstellen-Identifikationsbedingung eine Bedingung ist, unter welcher ein Freiraumstellen-Koordinatenbereich, der ein Bereich der Atom-Koordinateninformation für ein, eine Freiraumstelle bildendes Atom ist, für jede der vorbestimmten Molekularspezies definiert ist.

26. Computerlesbares Aufzeichnungsmedium gemäß Anspruch 25, wobei
das achte Identifizieren beinhaltet: ein neuntes Identifizieren der Freiraumstelleninformation, wenn die Atomkoordinaten-Information für das Atom, welches die, der beim fünfte Erfassen erfassten Freiraumstelleninformation entsprechende Freiraumstelle bildet, den durch Freiraumstellen-Identifikationsbedingung definierten Freiraumstellen-Koordinatenbereich erfüllt, in der, der identifizierten Molekularspezies-Information entsprechenden Molekularspezies.

27. Computerlesbares Aufzeichnungsmedium gemäß gemäß einem der Ansprüche 19 bis 26, wobei
das Arzneimittel-metabolisierendes Enzym-Vorhersageprogramm weiter einen Computer veranlasst, auszuführen:
ein fünftes Bestimmen, ob das die, der Reaktivstellen-Information entsprechende Reaktivstelle bildende Atom oder eine Partialstruktur-Atomgruppe, die eine Gruppe eines Teils der Atome in einer Molekularstruktur an der Reaktivstelle ist, die metabolische Reaktion hemmt, wenn die Reaktivstellen-Information beim dritten Identifizieren identifiziert ist, basierend auf einer Hemm-Bestimmungsbedingung zum Bestimmen, ob die metabolische Reaktion in der Verbindung, deren Reaktivstellen-Information identifiziert ist, gehemmt ist, wobei
das Ausgeben weiterhin an die Ausgabevorrichtung gehemmte metabolische Reaktion in der Verbindung der identifizierten Reaktivstelle ausgibt.

## Revendications

1. Appareil comprenant :
une première unité d'acquisition (102a) agencée pour acquérir, à partir d'une base de données d'informations sur la structure de composés, des informations sur la structure d'un composé incluant des informations sur les coordonnées atomiques de chacun d'une pluralité d'atomes formant un composé, des informations sur les liaisons entre les atomes, et des informations sur la structure cyclique relatives à une structure cyclique formée par les atomes ;
une première unité d'identification (102b) agencée pour identifier les informations sur le site de liaison relatives à un atome du composé correspondant aux informations sur la structure du composé et aux informations sur l'espèce moléculaire, d'après les informations sur la structure du composé acquises par la première unité d'acquisition, sur la base d'une condition d'identification du site de liaison permettant d'identifier l'espèce moléculaire d'une enzyme de métabolisation de médicaments appartenant aux familles CYP 1 à 4 des cytochromes P450 à relier et permettant d'identifier un site du composé où le composé et l'enzyme de métabolisation de médicaments sont liés l'un à l'autre, la condition d'identification du site de liaison étant une condition dans laquelle une plage de coordonnées du site de liaison, qui est une plage d'informations sur les coordonnées atomiques pour un atome formant un site de liaison, est définie pour chacune des espèces moléculaires prédéterminées ;
une deuxième unité d'acquisition (102c) agencée pour spécifier un point de pincement qui est l'atome reliant le site de liaison à un site réactif dans lequel une réaction métabolique du composé et de l'enzyme de métabolisation de médicaments a lieu, le point de pincement et le site réactif étant dans le même composé que le site de liaison, d'après les informations sur le site de liaison identifiées par la première unité d'identification (102b) et les informations sur la structure du composé, et pour acquérir des informations sur le point de pincement relatives à l'atome du point de pincement spécifié et des informations sur le site réactif relatives à l'atome formant le site réactif lié au point de pincement ;
une deuxième unité d'identification (102e) agencée pour identifier les informations sur le point de pincement acquises par la deuxième unité d'acquisition (102c), d'après les informations sur l'espèce moléculaire et les informations sur la structure du composé, sur la base d'une condition d'identification du point de pincement, la condition d'identification du point de pincement étant une condition dans laquelle une plage de coordonnées du point de pincement, qui est une plage des informations sur les coordonnées atomiques pour un atome en tant que point de pincement, est définie pour chacune des espèces moléculaires prédéterminées ;
une troisième unité d'identification (102d) agencée pour identifier les informations sur le site réactif acquises par la deuxième unité d'acquisition (102c), d'après les informations sur l'espèce moléculaire et les informations sur la structure du composé, sur la base d'une condition d'identification du site réactif et des informations sur le point de pincement identifiées, la condition d'identification du site réactif étant une condition dans laquelle une plage de coordonnées du site réactif qui est une plage des informations sur les coordonnées atomiques pour les atomes formant le site réactif, une plage d'angle du site réactif qui est une plage d'une valeur d'angle entre chacun des atomes formant le site réactif et le point de pincement, et une plage de distance du site réactif qui est une plage d'une valeur de distance entre chacun des atomes formant le site réactif et le point de pincement sont définies pour chacune des espèces moléculaires prédéterminées ;
une unité d'identification des informations sur les espèces moléculaires (102b) étant également agencée pour confirmer l'espèce moléculaire d'une enzyme de métabolisation de médicaments sur la base de la première, de la deuxième et de la troisième unité d'identification ; et
une unité de sortie (102i) agencée pour indiquer à un dispositif de sortie le site de liaison, le point de pincement, et le site réactif identifiés au niveau de la première à la troisième unité d'identification, et l'espèce moléculaire de l'enzyme de métabolisation de médicaments confirmée.

2. Appareil selon la revendication 1, dans lequel
la première unité d'identification (102b) inclut
une première unité de sélection (102j) agencée pour sélectionner le site de liaison sur la base d'un critère prédéterminé ;
une troisième unité d'acquisition (102k) agencée pour acquérir les informations sur le site de liaison pour le site de liaison sélectionné par l'unité de sélection du site de liaison (102j), d'après les informations sur la structure du composé ; et
une quatrième unité d identification (102m) agencée pour identifier les informations sur le site de liaison acquises par la troisième unité d'acquisition (102k), d'après les informations sur la structure du composé, sur la base de la condition d'identifcation du site de liaison, et qui identifie l'espèce moléculaire correspondant aux informations sur le site de liaison identifié comme étant les informations sur l'espèce moléculaire.

3. Appareil selon la revendication 2, dans lequel
la quatrième unité d'identifcation (102m) inclut une cinquième unité d'identification (102n) agencée pour identifier les informations sur le site de liaison lorsque les informations sur les coordonnées atomiques pour l'atome qui forme le site de liaison correspondant aux informations sur le site de liaison acquises par la troisième unité d'acquisition (102k) satisfont la plage de coordonnées du site de liaison définie par la condition d'identification du site de liaison, et qui identifie l'espèce moléculaire correspondant à la plage de coordonnées du site de liaison satisfaite comme étant les informations sur l'espèce moléculaire.

4. Appareil selon l'une quelconque des revendications 1 à 3, dans lequel
la deuxième unité d'acquisition (102c) inclut
une première unité de spécification (102p) agencée pour spécifier un atome lié à l'atome formant le site de liaison correspondant aux informations sur le site de liaison identifiées par la première unité d'identification (102b) et qui spécifie également l'atome lié à l'atome directement lié, sur la base des informations sur les liaisons incluses dans les informations sur la structure du composé ;
une première unité de détermination (102q) agencée pour déterminer si l'atome spécifié par la première unité de spécification (102p) est l'atome formant la structure cyclique, sur la base des informations sur la structure cyclique incluses dans les informations sur la structure du composé ;
une deuxième unité de spécification (102r) agencée pour spécifier l'atome spécifié et un autre atome formant la structure cyclique conjointement avec l'atome, en tant qu'atomes formant le site réactif, lorsqu'il est déterminé par la première unité de détermination (102q) que l'atome spécifié par la première unité de spécification (102p) est l'atome formant la structure cyclique, et qui spécifie l'atome directement lié spécifié par la première unité de spécification (102p), comme étant le point de pincement ; et
une quatrième unité d'acquisition (102s) agencée pour acquérir les informations sur le point de pincement pour le point de pincement spécifié par la deuxième unité de spécification (102r) et les informations sur le site réactif pour le site réactif spécifié, d'après les informations sur le site de liaison et les informations sur la structure du composé.

5. Appareil selon la revendication 4, dans lequel
la troisième unité d'identification (102d) inclut :
une deuxième unité de détermination (102t) agencée pour déterminer si les informations sur les coordonnées atomiques pour les atomes, qui forment le site réactif correspondant aux informations sur le site réactif acquises par la deuxième unité d'acquisition (102c), satisfont la plage de coordonnées du site réactif définie par la condition identification du site réactif, dans l'espèce moléculaire correspondant aux informations sur l'espèce moléculaire identifiées ;
une troisième unité de détermination (102u) agencée pour calculer la valeur d'angle entre l'atome formant le site réactif correspondant aux informations sur le site réactif et le point de pincement correspondant aux informations sur le point de pincement, et pour déterminer si la valeur d'angle calculée satisfait la plage d'angle du site réactif définie par la condition d'identification du site réactif, dans l'espèce moléculaire correspondant aux informations sur l'espèce moléculaire identifiées ;
une quatrième unité de détermination (102v) agencée pour calculer la valeur de distance entre l'atome formant le site réactif correspondant aux informations sur le site réactif et le point de pincement correspondant aux informations sur le point de pincement, et pour déterminer si la valeur de distance calculée satisfait la plage de distance du site réactif définie par la condition d'identification du site réactif, dans l'espèce moléculaire correspondant aux informations sur l'espèce moléculaire identifiées ; et
une sixième unité d'identification (102w) agencée pour identifier les informations sur le site réactif déterminées, telles que satisfaites, par la deuxième unité de détermination (102t), la troisième unité de détermination (102u) et la quatrième unité de détermination (102v), comme étant les informations sur le site réactif qui satisfont la condition d'identification du site réactif.

6. Appareil selon l'une quelconque des revendications 1 à 5, dans lequel
la deuxième unité d'identification (102e) inclut une septième unité d'identification (102x) agencée pour identifier les informations sur le point de pincement lorsque les informations sur les coordonnées atomiques pour l'atome, qui est le point de pincement correspondant aux informations sur le point de pincement acquises par la deuxième unité d'acquisition (102c), satisfont la plage de coordonnées du point de pincement définie par la condition d'identification du point de pincement, dans l'espèce moléculaire correspondant aux informations sur l'espèce moléculaire identifiées.

7. Appareil selon l'une quelconque des revendications 1 à 6, comprenant en outre :
une cinquième unité d'acquisition (102f) agencée pour acquérir des informations sur le site à espace libre qui sont des informations sur l'atome formant un site à espace libre, d'après les informations sur le site de liaison, les informations sur le site réactif, les informations sur le point de pincement et les informations sur la structure du composé, si le site à espace libre existe un site autre que le site réactif correspondant aux informations sur le site réactif identifiées par la troisième unité d'identification (102d) et le site de liaison correspondant aux informations sur le site de liaison identifiées par la première unité d'identification (102b) et qui est lié au point de pincement correspondant aux informations sur le point de pincement identifiées par l'unité d'identification des informations sur le point de pincement (102e) ; et
une huitième unité d'identification (102g) agencée pour identifier les informations sur le site à espace libre acquises par la cinquième unité d'acquisition (102f), d'après les informations sur l'espèce moléculaire et les informations sur la structure du composé, sur la base d'une condition d'identification du site à espace libre permettant d'identifier le site à espace libre, la condition d'identification du site à espace libre étant une condition dans laquelle une plage de coordonnées du site à espace libre, qui est une plage des informations sur les coordonnées atomiques pour un atome formant un site à espace libre est définie pour chacune des espèces moléculaires prédéterminées.

8. Appareil selon la revendication 7, dans lequel
la huitième unité d'identification (102g) inclut une neuvième unité d'identification (102y) agencée pour identifier les informations sur le site à espace libre lorsque les informations sur les coordonnées atomiques pour l'atome, qui forme le site à espace libre correspondant aux informations sur le site à espace libre acquises par la cinquième unité d'acquisition (102f), satisfont la plage de coordonnées du site à espace libre définie par la condition d'identification du site à espace libre, dans l'espèce moléculaire correspondant aux informations sur l'espèce moléculaire identifiées.

9. Appareil selon l'une quelconque des revendications 1 à 8, comprenant en outre :
une cinquième unité de détermination (102h) agencée pour déterminer si l'atome formant le site réactif correspondant aux informations sur le site réactif ou un groupe atomique à structure partielle qui est un groupe d'une partie des atomes d'une structure moléculaire au niveau du site réactif inhibe la réaction métabolique, lorsque les informations sur le site réactif sont identifiées par la troisième unité d'identification (102d), sur la base d'une condition de détermination d'une inhibition permettant de déterminer si la réaction métabolique est inhibée dans le composé dont les informations sur le site réactif sont identifiées, dans lequel
l'unité de sortie (102i) est agencée pour en outre indiquer au dispositif de sortie une réaction métabolique inhibée dans le composé du site réactif identifié.

10. Procédé de prévision des enzymes de métabolisation de médicaments comprenant :
une première acquisition, à partir d'une base de données d'informations sur la structure de composés, à l'aide d'un processeur, des informations sur la structure d'un composé incluant des informations sur les coordonnées atomiques de chacun d'une pluralité d'atomes formant un composé, des informations sur les liaisons entre les atomes, et des informations sur la structure cyclique relatives à une structure cyclique formée par les atomes ;
une première identification, à l'aide d'un processeur, des informations sur le site de liaison relatives à un atome formant un site de liaison dans le composé correspondant aux informations sur la structure du composé et des informations sur l'espèce moléculaire, d'après les informations sur la structure du composé acquises au moment de la première acquisition, sur la base d'une condition d'identification du site de liaison permettant d'identifier l'espèce moléculaire d'une enzyme de métabolisation de médicaments appartenant aux familles CYP 1 à 4 des cytochromes P450 à relier et permettant d'identifier un site du composé où le composé et l'enzyme de métabolisation de médicaments sont liés l'un à l'autre, la condition d'identification du site de liaison étant une condition dans laquelle une plage de coordonnées du site de liaison, qui est une plage d'informations sur les coordonnées atomiques pour un atome formant un site de liaison, est définie pour chacune des espèces moléculaires prédéterminées ;
une deuxième acquisition, à l'aide d'un processeur, d'un point de pincement et du site réactif qui est l'atome reliant le site de liaison à un site réactif dans lequel une réaction métabolique du composé et de l'enzyme de métabolisation de médicaments a lieu, le point de pincement et le site réactif étant dans le même composé que le site de liaison, d'après les informations sur le site de liaison identifiées au moment de la première identification et les informations sur la structure du composé, et l'acquisition des informations sur le point de pincement relatives à l'atome du point de pincement spécifié et des informations sur le site réactif relatives à l'atome formant le site réactif lié au point de pincement ;
une deuxième identification, à l'aide d'un processeur, des informations sur le point de pincement acquises au moment de la deuxième acquisition, d'après les informations sur l'espèce moléculaire et les informations sur la structure du composé, sur la base d'une condition d'identifcation du point de pincement, la condition d'identification du point de pincement étant une condition dans laquelle une plage de coordonnées du point de pincement, qui est une plage des informations sur les coordonnées atomiques pour un atome en tant que point de pincement, est définie pour chacune des espèces moléculaires prédéterminées ;
une troisième identification, à l'aide d'un processeur, des informations sur le site réactif acquises au moment de la deuxième acquisition, d'après les informations sur l'espèce moléculaire et les informations sur la structure du composé, sur la base d'une condition d'identification du site réactif et des informations sur le point de pincement identifiées, la condition d identification du site réactif étant une condition dans laquelle une plage de coordonnées du site réactif qui est une plage des informations sur les coordonnées atomiques pour les atomes formant le site réactif, une plage d'angle du site réactif qui est une plage d'une valeur d'angle entre chacun des atomes formant le site réactif et le point de pincement, et une plage de distance du site réactif qui est une plage d'une valeur de distance entre chacun des atomes formant le site réactif et le point de pincement sont définies pour chacune des espèces moléculaires prédéterminées ;
la confirmation, à l'aide d'un processeur, de l'espèce moléculaire d'une enzyme de métabolisation de médicaments sur la base de la première, de la deuxième et de la troisième identification ; et
l'indication à un dispositif de sortie du site de liaison, du point de pincement et du site réactif identifiés au moment de la première identification à la troisième identification, et de l'espèce moléculaire de l'enzyme de métabolisation de médicaments confirmée.

11. Procédé de prévision des enzymes de métabolisation de médicaments selon la revendication 10, dans lequel
la première identification inclut
une première sélection, à l'aide d'un processeur, du site de liaison sur la base d'un critère prédéterminé ;
une troisième acquisition, à l'aide d'un processeur, des informations sur le site de liaison pour le site de liaison sélectionné au moment de la première sélection, d'après les informations sur la structure du composé ; et
une quatrième identification, à l'aide d'un processeur, des informations sur le site de liaison acquises au moment de la troisième acquisition, d'après les informations sur la structure du composé, sur la base de la condition d identification du site de liaison, et de l'espèce moléculaire correspondant aux informations sur le site de liaison identifiées comme étant les informations sur l'espèce moléculaire.

12. Procédé de prévision des enzymes de métabolisation de médicaments selon la revendication 11, dans lequel
la quatrième identification inclut une cinquième identification, à l'aide d'un processeur, des informations sur le site de liaison lorsque les informations sur les coordonnées atomiques pour l'atome qui forme le site de liaison correspondant aux informations sur le site de liaison acquises au moment de la troisième acquisition satisfont la plage de coordonnées du site de liaison définie par la condition d'identification du site de liaison, et l'identification de l'espèce moléculaire correspondant à la plage de coordonnées du site de liaison satisfaite comme étant les informations sur l'espèce moléculaire.

13. Procédé de prévision des enzymes de métabolisation de médicaments selon l'une quelconque des revendications 10 à 12, dans lequel
la deuxième acquisition inclut
une première spécification, à l'aide d'un processeur, d'un atome lié à l'atome formant le site de liaison correspondant aux informations sur le site de liaison identifiées au moment de la première identification et également la spécification de l'atome lié à l'atome directement lié, sur la base des informations sur les liaisons incluses dans les informations sur la structure du composé ;
une première détermination, à l'aide d'un processeur, indiquant si la première spécification est l'atome formant la structure cyclique, sur la base des informations sur la structure cyclique incluses dans les informations sur la structure du composé ;
une deuxième spécification, à l'aide d'un processeur, de l'atome spécifié et d'un autre atome formant la structure cyclique conjointement avec l'atome, en tant qu'atomes formant le site réactif, lorsqu'il est déterminé au moment de la première détermination que l'atome spécifié au moment de la première spécification est l'atome formant la structure cyclique, et la spécification de l'atome directement lié spécifié au moment de la spécification de l'atome, en tant que point de pincement ; et
une quatrième acquisition, à l'aide d'un processeur, des informations sur le point de pincement pour le point de pincement spécifié au moment de la deuxième spécification et des informations sur le site réactif pour le site réactif spécifié, d'après les informations sur le site de liaison et les informations sur la structure du composé.

14. Procédé de prévision des enzymes de métabolisation de médicaments selon la revendication 13, dans lequel
la troisième identification inclut
une deuxième détermination, à l'aide d'un processeur, indiquant si les informations sur les coordonnées atomiques pour les atomes, qui forment le site réactif correspondant aux informations sur le site réactif acquises au moment de la deuxième acquisition, satisfont la plage de coordonnées du site réactif définie par la condition d'identification du site réactif, dans l'espèce moléculaire correspondant aux informations sur l'espèce moléculaire identifiées ;
une troisième détermination, à l'aide d'un processeur, de la valeur d'angle entre l'atome formant le site réactif correspondant aux informations sur le site réactif et le point de pincement correspondant aux informations sur le point de pincement qui est calculée, et indiquant si la valeur d'angle calculée satisfait la plage d'angle du site réactif définie par la condition d'identification du site réactif, dans l'espèce moléculaire correspondant aux informations sur l'espèce moléculaire identifiées ;
une quatrième détermination, à l'aide d'un processeur, de la valeur de distance entre l'atome formant le site réactif correspondant aux informations sur le site réactif et le point de pincement correspondant aux informations sur le point de pincement qui est calculée, et indiquant si la valeur de distance calculée satisfait la plage de distance du site réactif définie par la condition d'identification du site réactif, dans l'espèce moléculaire correspondant aux informations sur l'espèce moléculaire identifiées ; et
une sixième identification, à l'aide d'un processeur, des informations sur le site réactif déterminées, telles que satisfaites, au moment de la deuxième détermination, de la troisième détermination et de la quatrième détermination, en tant qu'informations sur le site réactif qui satisfont la condition d'identification du site réactif.

15. Procédé de prévision des enzymes de métabolisation de médicaments selon l'une quelconque des revendications 10 à 14, dans lequel
la deuxième identification inclut une septième identification, à l'aide d'un processeur, des informations sur le point de pincement lorsque les informations sur les coordonnées atomiques pour l'atome, qui est le point de pincement correspondant aux informations sur le point de pincement acquises au moment de la deuxième acquisition, satisfont la plage de coordonnées du point de pincement définie par la condition d'identification du point de pincement, dans l'espèce moléculaire correspondant aux informations sur l'espèce moléculaire identifiées.

16. Procédé de prévision des enzymes de métabolisation de médicaments selon l'une quelconque des revendications 10 à 15, comprenant en outre :
une cinquième acquisition, à l'aide d'un processeur, des informations sur l'atome formant un site à espace libre, d'après les informations sur le site de liaison, des informations sur le site réactif, des informations sur le point de pincement et des informations sur la structure du composé, s'il existe le site à espace libre qui est un site autre que le site réactif correspondant aux informations sur le site réactif identifiées au moment de la troisième identification et le site de liaison correspondant aux informations sur le site de liaison identifiées au moment de la première identification et qui est lié au point de pincement correspondant aux informations sur le point de pincement identifiées au moment de la deuxième identification ; et
une huitième identification, à l'aide d'un processeur, des informations sur le site à espace libre acquises au moment de la cinquième acquisition, d'après les informations sur l'espèce moléculaire et les informations sur la structure du composé, sur la base d'une condition d identification du site à espace libre permettant d'identifier le site à espace libre, la condition d'identifcation du site à espace libre étant une condition dans laquelle une plage de coordonnées du site à espace libre, qui est une plage des informations sur les coordonnées atomiques pour un atome formant un site à espace libre est définie pour chacune des espèces moléculaires prédéterminées.

17. Procédé de prévision des enzymes de métabolisation de médicaments selon la revendication 16, dans lequel
la huitième identification inclut une neuvième identification, à l'aide d'un processeur, des informations sur le site à espace libre lorsque les informations sur les coordonnées atomiques pour l'atome, qui forme le site à espace libre correspondant aux informations sur le site à espace libre acquises au moment de la cinquième acquisition, satisfont la plage de coordonnées du site à espace libre définie par la condition d'identification du site à espace libre, dans l'espèce moléculaire correspondant aux informations sur l'espèce moléculaire identifiées.

18. Procédé de prévision des enzymes de métabolisation de médicaments selon l'une quelconque des revendications 10 à 17, comprenant en outre :
une cinquième détermination, à l'aide d'un processeur, indiquant si l'atome formant le site réactif correspondant aux informations sur le site réactif ou un groupe atomique à structure partielle qui est un groupe d'une partie des atomes d'une structure moléculaire au niveau du site réactif inhibe la réaction métabolique, lorsque les informations sur le site réactif sont identifiées au moment de la troisième identification, sur la base d'une condition de détermination d'une inhibition permettant de déterminer si la réaction métabolique est inhibée dans le composé dont les informations sur le site réactif sont identifiées, dans lequel
la sortie indique en outre au dispositif de sortie une réaction métabolique inhibée dans le composé du site réactif identifié.

19. Support d'enregistrement lisible par ordinateur qui y stocke un programme de prévision des enzymes de métabolisation de médicaments, dans lequel
le programme de prévision des enzymes de métabolisation de médicaments demande à un ordinateur d'exécuter :
une première acquisition, à partir d'une base de données d informations sur la structure de composés, des informations sur la structure d'un composé incluant des informations sur les coordonnées atomiques de chacun d'une pluralité d'atomes formant un composé, des informations sur les liaisons entre les atomes, et des informations sur la structure cyclique qui sont des informations sur une structure cyclique formée par les atomes ;
une première identification des informations sur le site de liaison qui sont des informations sur un atome du composé correspondant aux informations sur la structure du composé et aux informations sur l'espèce moléculaire, d'après les informations sur la structure du composé acquises au moment de la première acquisition, sur la base d'une condition d'identification du site de liaison permettant d'identifier l'espèce moléculaire d'une enzyme de métabolisation de médicaments appartenant aux familles CYP 1 à 4 des cytochromes P450 à relier et permettant d'identifer un site du composé où le composé et l'enzyme de métabolisation de médicaments sont liés l'un à l'autre, la condition d'identification du site de liaison étant une condition dans laquelle une plage de coordonnées du site de liaison, qui est une plage d'informations sur les coordonnées atomiques pour un atome formant un site de liaison, est définie pour chacune des espèces moléculaires prédéterminées ;
une deuxième acquisition d'un point de pincement qui est l'atome reliant le site de liaison à un site réactif dans lequel une réaction métabolique a lieu entre le composé et l'enzyme de métabolisation de médicaments, le point de pincement et le site réactif étant dans le même composé que le site de liaison, d'après les informations sur le site de liaison identifiées au moment de la première identification et les informations sur la structure du composé, et l'acquisition des informations sur le point de pincement relatives à l'atome du point de pincement spécifié et des informations sur le site réactif relatives à l'atome formant le site réactif lié au point de pincement ;
une deuxième identification du point de pincement acquis au moment de la deuxième acquisition, d'après les informations sur l'espèce moléculaire et les informations sur la structure du composé, sur la base d'une condition d'identification du point de pincement, la condition d'identification du point de pincement étant une condition dans laquelle une plage de coordonnées du point de pincement, qui est une plage des informations sur les coordonnées atomiques pour un atome en tant que point de pincement, est définie pour chacune des espèces moléculaires prédéterminées ;
une troisième identification des informations sur le site réactif acquises au moment de la deuxième acquisition, d'après les informations sur l'espèce moléculaire et les informations sur la structure du composé, sur la base d'une condition d'identification du site réactif et des informations sur le point de pincement identifiées, la condition d'identification du site réactif étant une condition dans laquelle une plage de coordonnées du site réactif qui est une plage des informations sur les coordonnées atomiques pour les atomes formant le site réactif, une plage d'angle du site réactif qui est une plage d'une valeur d'angle entre chacun des atomes formant le site réactif et le point de pincement, et une plage de distance du site réactif qui est une plage d'une valeur de distance entre chacun des atomes formant le site réactif et le point de pincement sont définies pour chacune des espèces moléculaires prédéterminées ;
la confirmation de l'espèce moléculaire d'une enzyme de métabolisation de médicaments sur la base de la première, de la deuxième et de la troisième identification ; et
l'indication à un dispositif de sortie du site de liaison, du point de pincement et du site réactif identifiés au moment de la première identification à la troisième identification, et de l'espèce moléculaire de l'enzyme de métabolisation de médicaments confirmée.

20. Support d'enregistrement lisible par ordinateur selon la revendication 19, dans lequel
la première identification inclut
une première sélection du site de liaison sur la base d'un critère prédéterminé ;
une troisième acquisition des informations sur le site de liaison pour le site de liaison sélectionné au moment de la première sélection, d'après les informations sur la structure du composé ; et
une quatrième identification des informations sur le site de liaison acquises au moment de la troisième acquisition, d'après les informations sur la structure du composé, sur la base de la condition d'identification du site de liaison, et de l'espèce moléculaire correspondant aux informations sur le site de liaison identifiées comme étant les informations sur l'espèce moléculaire.

21. Support d'enregistrement lisible par ordinateur selon la revendication 20, dans lequel
la quatrième identification inclut une cinquième identification des informations sur le site de liaison lorsque les informations sur les coordonnées atomiques pour l'atome qui forme le site de liaison correspondant aux informations sur le site de liaison acquises au moment de la troisième acquisition satisfont la plage de coordonnées du site de liaison définie par la condition d'identification du site de liaison, et l'identification de l'espèce moléculaire correspondant à la plage de coordonnées du site de liaison satisfaite comme étant les informations sur l'espèce moléculaire.

22. Support d'enregistrement lisible par ordinateur selon l'une quelconque des revendications 19 à 21, dans lequel
la deuxième acquisition inclut
une première spécification d'un atome lié à l'atome formant le site de liaison correspondant aux informations sur le site de liaison identifiées au moment de la première identification et également la spécification de l'atome lié à l'atome directement lié, sur la base des informations sur les liaisons incluses dans les informations sur la structure du composé ;
une première détermination indiquant si l'atome spécifié au moment de la spécification de l'atome est l'atome formant la structure cyclique, sur la base des informations sur la structure cyclique incluses dans les informations sur la structure du composé ;
une deuxième spécification de l'atome spécifié et d'un autre atome formant la structure cyclique conjointement avec l'atome, en tant qu'atomes formant le site réactif, lorsqu'il est déterminé au moment de la première détermination que l'atome spécifié au moment de la première spécification est l'atome formant la structure cyclique, et la spécification de l'atome directement lié spécifié au moment de la première spécification, en tant que point de pincement ; et
une quatrième acquisition des informations sur le point de pincement pour le point de pincement spécifié au moment de la deuxième spécification et des informations sur le site réactif pour le site réactif spécifié, d'après les informations sur le site de liaison et les informations sur la structure du composé.

23. Support d'enregistrement lisible par ordinateur selon la revendication 22, dans lequel
la troisième identification inclut
une deuxième détermination indiquant si les informations sur les coordonnées atomiques pour les atomes, qui forment le site réactif correspondant aux informations sur le site réactif acquises au moment de la deuxième acquisition, satisfont la plage de coordonnées du site réactif définie par la condition d'identification du site réactif, dans l'espèce moléculaire correspondant aux informations sur l'espèce moléculaire identifiées ;
une troisième détermination de la valeur d'angle entre l'atome formant le site réactif correspondant aux informations sur le site réactif et le point de pincement correspondant aux informations sur le point de pincement qui est calculée, et indiquant si la valeur d'angle calculée satisfait la plage d'angle du site réactif définie par la condition d identification du site réactif, dans l'espèce moléculaire correspondant aux informations sur l'espèce moléculaire identifiées ;
une quatrième détermination de la valeur de distance entre l'atome formant le site réactif correspondant aux informations sur le site réactif et le point de pincement correspondant aux informations sur le point de pincement qui est calculée, et indiquant si la valeur de distance calculée satisfait la plage de distance du site réactif définie par la condition d'identification du site réactif, dans l'espèce moléculaire correspondant aux informations sur l'espèce moléculaire identifiées ; et
une sixième identification des informations sur le site réactif déterminées, telles que satisfaites, au moment de la deuxième détermination, de la troisième détermination et de la quatrième détermination, en tant qu'informations sur le site réactif qui satisfont la condition d'identification du site réactif.

24. Support d'enregistrement lisible par ordinateur selon l'une quelconque des revendications 19 à 23, dans lequel
la deuxième identification inclut une septième identification des informations sur le point de pincement lorsque les informations sur les coordonnées atomiques pour l'atome, qui est le point de pincement correspondant aux informations sur le point de pincement acquises au moment de la deuxième acquisition, satisfont la plage de coordonnées du point de pincement définie par la condition d identification du point de pincement, dans l'espèce moléculaire correspondant aux informations sur l'espèce moléculaire identifiées.

25. Support d'enregistrement lisible par ordinateur selon l'une quelconque des revendications 19 à 24, dans lequel
le programme de prévision des enzymes de métabolisation de médicaments demande également à l'ordinateur d'exécuter :
une cinquième acquisition des informations sur l'atome formant un site à espace libre, d'après les informations sur le site de liaison, les informations sur le site réactif, les informations sur le point de pincement et les informations sur la structure du composé, s'il existe le site à espace libre qui est un site autre que le site réactif correspondant aux informations sur le site réactif identifiées au moment de la troisième identification et le site de liaison correspondant aux informations sur le site de liaison identifiées au moment de la première identification et qui est lié au point de pincement correspondant aux informations sur le point de pincement identifiées au moment de la deuxième identification ; et
une huitième identification des informations sur le site à espace libre acquises au moment de la cinquième acquisition, d'après les informations sur l'espèce moléculaire et les informations sur la structure du composé, sur la base d'une condition d'identification du site à espace libre permettant d'identifier le site à espace libre, la condition d'identification du site à espace libre étant une condition dans laquelle une plage de coordonnées du site à espace libre, qui est une plage des informations sur les coordonnées atomiques pour un atome formant un site à espace libre est définie pour chacune des espèces moléculaires prédéterminées.

26. Support d'enregistrement lisible par ordinateur selon la revendication 25, dans lequel
la huitième identification inclut une neuvième identification des informations sur le site à espace libre lorsque les informations sur les coordonnées atomiques pour l'atome, qui forme le site à espace libre correspondant aux informations sur le site à espace libre acquises au moment de la cinquième acquisition, satisfont la plage de coordonnées du site à espace libre définie par la condition d'identification du site à espace libre, dans l'espèce moléculaire correspondant aux informations sur l'espèce moléculaire identifiées.

27. Support d'enregistrement lisible par ordinateur selon l'une quelconque des revendications 19 à 26, dans lequel
le programme de prévision des enzymes de métabolisation de médicaments demande également à un ordinateur d'exécuter :
une cinquième détermination indiquant si l'atome formant le site réactif correspondant aux informations sur le site réactif ou un groupe atomique à structure partielle qui est un groupe d'une partie des atomes d'une structure moléculaire au niveau du site réactif inhibe la réaction métabolique, lorsque les informations sur le site réactif sont identifiées au moment de la troisième identification, sur la base d'une condition de détermination d'une inhibition permettant de déterminer si la réaction métabolique est inhibée dans le composé dont les informations sur le site réactif sont identifiées, dans lequel
la sortie indique en outre au dispositif de sortie une réaction métabolique inhibée dans le composé du site réactif identifié.
